# EUROPEAN PATENT APPLICATION

(11) **EP 1 179 347 A1**
(43) Date of publication of application: **13.02.2002**
(21) Application number: 00929806.8
(22) Date of filing: 19.05.2000
(51) Int. Cl.: A61K 38/17, A61K 31/711, A61K 48/00, A61P 1/16, C12N 15/12, C12Q 1/68, C07K 14/47, C12N 1/19, C12N 1/15, C12N 1/21, C12N 5/10, C07K 16/18

(54) **LIVER FUNCTION CONTROLLING AGENTS**

(30) Priority: 21.05.1999 JP 14110699; 19.01.2000 JP 2000014044
(71) Applicant: Takeda Chemical Industries, Ltd., Osaka-shi, Osaka 541-0045 (JP)
(72) Inventor: MATSUI, Hideki, Tsukuba-shi, Ibaraki 305-0044 (JP); SHINTANI, Ysushi, Tsukuba-shi, Ibaraki 305-0821 (JP); HIKICHI, Yukiko, Shareru Tsukuba Matsuhiro 1-504, Tsukuba-shi, Ibaraki 305-0035 (JP)
(74) Representative: Keller, Günter, Dr.
(86) International application number: JP0003222
(87) International publication number: WO0071151

(57) **Abstract**

The invention provides novel liver function controlling agents and others.

The liver function controlling agents of the present invention are useful as preventive/therapeutic agents for diseases such as hepatic dysfunction, hepatitis, liver cancer, liver cirrhosis, etc.

## Description

### FIELD OF THE INVENTION

This invention relates to a novel liver function controlling agent, etc.

### BACKGROUND ART

Liver is an essential organ to the maintenance of life that plays vital roles in regulating our total energy metabolism and hence, has widely diverse functions. Major functions of liver in vivo include the function of storage and circulation, the function of degradation and excretion, the function of metabolism (metabolism of various nutrients like sugars, proteins, lipids, vitamins, hormones, etc.), the function of protection and detoxication, the hematological function, and so on. Injury of some of these functions results in various conditions inherent to the liver dysfunction, including feeling of fatigue, feeling of weariness, anorexia, jaundice and a slight fever. Moreover, serious hepatic diseases such as liver cirrhosis, viral hepatitis, fulminant hepatitis, hepatic cancer, etc. have been left unattended to date, without any established effective therapy.

On the other hand, the liver is the most highly differentiated and most reproducible organ in a living body (Cell, 96, 235-244 (1999)). Liver parenchymal cells that control metabolism or synthesis and various non-parenchymal cells such as Kupffer cells, endothelial cells, Ito cells, etc. constitute the liver. Among them, liver parenchymal cells that play a central role are known to be controlled by a variety of hormones in vivo and to be very active in proliferation under certain conditions (*Science*, 276, 60-65 (1997)). For example, it is known that when rat liver is removed by 70%, the liver is restored to the almost original weight in about 10 days. Also in human, patients with liver cancer are treated by partial hepatectomy and regeneration of the liver after hepatectomy. For this reason, regeneration of the liver has been of interest from old in terms of its regeneration mechanism. The mechanism of liver regeneration is not only an important issue in cell biology but also has attracted a keen attention for establishing therapy for various hepatic failures. In fact, extensive studies have been hitherto made on the mechanism of liver regeneration by proliferation of liver parenchymal cells, and liver parenchymal cell growth factor candidates have been reported (Molecular Medicine, 34, 544-553 (1997)).

At present, HGF, TGFα, EGF, HB-EGF, TNFα, IL-6 and so on are known as factors for promoting liver regeneration. HGF is produced in various cells of the whole body, other than liver parenchymal cells, and is considered to take part in the proliferation of liver parenchymal cells, since hepatectomy results in increased production of HGF from many organs including liver to enhance HGF level in blood (FASEB J., 9, 1527-1536 (1995)). TGFα is a factor belonging to the EGF family, and produced in liver parenchymal cells, which is considered to act as an autocrine factor (Proc. Natl. Acad. Sci. USA, 86, 1558-1562 (1989)). On the other hand, EGF was known from old to be a factor that promotes growth of liver cells in vitro extremely strongly. However, since it is not considered that EGF would be expressed in liver, it is controversial to what extent EGF participates in the actual liver regeneration (FASEB J., 9, 1527-1536 (1995)). Also, HB-EGF exhibits a very strong growth promoting activity on hepatocytes and thus, is a molecule that has lately drawn attention (Biochem. Biophys. Res. Commun., 198, 25-31 (1994)).

However, the results of recent researches reveal that the very first trigger on liver regeneration is , not a growth factor such as HGF, etc. but a factor for promoting transfer from the G0 phase to the G1 phase of the cell cycle, though the factor does not take part in cell division (Progress in Cell Cycle Research, 2, 37-47 (1996)). For convenience, the factor is called a priming factor. Since separation of liver parenchymal cells from the liver followed by incubation result in transfer to the G1 phase, the growth factor mentioned above can strongly promote the proliferation of liver parenchymal cells in vitro. In normal animal, however, even though a growth factor is given, the proliferation of liver parenchymal cells cannot be induced unless the factor for promoting the transfer is expressed or activated. Identification of the factor that would participate in the transfer from the G0 phase to the G1 phase is now under way. It is reported that activation of transcription factors including posthepatectomy factor (PHF)/NF-κB, ApP-1 or STAT3 occurs prior to DNA synthesis, which is considered to play an important role in liver regeneration. Factors that induce the activation of these transcription factors are considered to be a priming factor. TNFα and IL-6 are candidates for the priming factor. It is reported that these cytokines take part in activation of transcription factors such as NF-κB, STAT3, etc.; liver regeneration after hepatectomy is seriously poor in TNFα receptor- or IL-6-deficient knockout mice in which activation of NF-κB, STAT3, etc. is inhibited; and further that administration of IL-6 to these animals makes liver regeneration possible (Proc. Natl. Acad. Sci. USA, 94, 1441-1446 (1997), Science, 274, 1379-1383 (1996)). These reports are strong bases to suggest that these candidates would be priming factors. However, it is controversial how much these factors actually participate in clinical liver regeneration. No study clearly showing the extent of participation has been made yet. In particular, it is reported on TNFα to participate in liver regeneration on one hand, and on the other hand, it is considered that TNFα would be deeply associated also with the mechanism of liver injury (Hepatology, 29, 1-4, (1999) ; J. Immunol., 153, 1778-1788 (1994)). Like this, the principal physiological significance of TNFα is unknown. Availability of these factors as therapeutic agents is yet unclear.

Under the foregoing situation, it has been sought to find a novel factor having an activity effective for liver regeneration and if such a factor has no side effect against liver cells (e.g., any serious action such as induction of apoptosis, etc.), it is expected that the factor will be able to regulate the liver function of mammal including human to a normal condition.

### DISCLOSURE OF THE INVENTION

The present inventors have found that a ligand molecule. TL4 (WO 98/03648) belonging to the TNF family can unexpectedly promote the DNA synthesis capability of normal human liver parenchymal cells, and also found that TL4 does not have any activity to induce cell death markedly noted with other ligand molecules of the TNF family when used in combination with actinomycin D. Further investigations have come to accomplish the present invention.

That is, the present invention relates to the following features.
(1) A liver function controlling agent comprising a protein containing an amino acid sequence, which is the same or substantially the same as the amino acid sequence represented by SEQ ID NO:1, SEQ ID NO:2, SEQ ID NO:3 or SEQ ID NO:31, or a salt thereof.
(2) The agent according to (1), wherein substantially the same amino acid sequence as the amino acid sequence represented by SEQ ID NO:1, SEQ ID NO:2 or SEQ ID NO:3 is an amino acid sequence of 8-21, 54-59, 93-102, 109-116, 118-126, 128-134, 144-149, 162-170, 176-182, 184-189, 193-213, 215-219 and 228-240 in the amino acid sequence represented by SEQ ID NO:1.
(3) The liver function controlling agent comprising a partial peptide of the protein according to (1), or a salt thereof.
(4) The agent according to (3), wherein a partial peptide of the protein according to (1) is a peptide comprising an amino acid sequence having an amino acid sequence of 84-240 in the amino acid sequence represented by SEQ ID NO:1.
(5) The liver function controlling agent comprising a DNA containing a DNA having a base sequence encoding the protein according to (1) or the partial peptide according to (3).
(6) The agent according to (5), wherein the DNA is a DNA having a base sequence represented by any one of SEQ ID NO:4 to SEQ ID NO:10, or by SEQ ID NO:30.
(7) The liver function controlling agent comprising an antibody to the protein according to (1) or the partial peptide according to (3), or to a salt thereof.
(8) A method of screening a liver function controlling agent which comprises using (a) the protein according to (1) or the partial peptide according to (3), or a salt thereof, (b) the DNA according to (5), or (c) the antibody according to (7).
(9) A liver function controlling agent comprising a compound obtained using the method of screening according to (8), or a salt thereof.
(10) The agent according to (1), (3), (5), (7) or (9), which is a liver function promoting agent.
(11) The agent according to (1), (3), (5), (7) or (9), which is a liver regenerating agent.
(12) The agent according to (1), (3), (5), (7) or (9), which has an action for promoting transfer from the G0 phase to the G1 phase of the cell cycle.
(13) A protein comprising an amino acid sequence represented by SEQ ID NO:31, or a salt thereof.
(14) A DNA containing a DNA having a base sequence encoding the protein according to (13).
(15) The DNA according to (14) having a base sequence represented by SEQ ID NO:30.
(16) A recombinant vector containing the DNA according to (15).
(17) A transformant transformed by the recombinant vector according to (16).
(18) A process of producing the protein or its salt according to (13), which comprises culturing the transformant according to (17), producing, accumulating and collecting the protein according to (13).
(19) An antibody to the protein or its salt according to (13).
(20) A diagnostic agent comprising the DNA according to (14) or the antibody according to (19).
(21) An antisense DNA containing a base sequence complementary or substantially complementary to the DNA according to (14) and having an action capable of suppressing expression of the DNA.
(22) A method of screening a compound or its salt that accelerates or inhibits the activity of the protein or its salt according to (13), which comprises using the protein or its salt according to (13).
(23) A kit for screening a compound or its salt that accelerates or inhibits the activity of the protein or its salt according to (13), comprising the protein or its salt according to (13).
(24) A compound or its salt that accelerates or inhibits the activity of the protein or its salt according to (13), which is obtainable using the method of screening according to (22) or the kit for screening according to (23).
(25) A pharmaceutical composition comprising the compound or its salt according to (24).
(26) The pharmaceutical composition according to (25), which is a liver function controlling agent.
   The present invention further relates to the following features.
(27) The method of screening according to (8), which comprises determining DNA synthesis capabilities in the cases where (i) the protein according to (1), the partial peptide according to (3) or a salt thereof is brought in contact with a liver cell and (ii) the partial peptide according to (3) or a salt thereof and a test compound are brought in contact with a liver cell or liver tissue; and comparing the DNA synthesis capabilities.
(28) The method of screening according to (27), wherein the liver cell is a liver parenchymal cell.
(29) A compound or a salt thereof having a liver function controlling activity, obtainable by the method of screening according to (28).
(30) The compound or its salt according to (24) or (28),which is an agent for the prevention/treatment of impaired liver function, liver cancer, hepatitis (viral hepatitis, fulminant hepatitis, etc.) or liver cirrhosis.
(31) The compound or its salt according to (24) or (28).

### BRIEF DESCRIPTION OF THE DRAWINGS

FIG. 1 shows a base sequence of DNA encoding the human-derived protein of the present invention contained in plasmid pTB1939 obtained in REFERENCE EXAMPLE 1, and the amino acid sequence deduced therefrom of the human-derived protein of the present invention (continued to FIG. 2).
FIG. 2 shows a base sequence of DNA encoding the human-derived protein of the present invention contained in plasmid pTB1939 obtained in REFERENCE EXAMPLE 1, and the amino acid sequence deduced therefrom of the human-derived protein of the present invention (continued from FIG. 1 and to FIG. 3).
FIG. 3 shows a base sequence of DNA encoding the human-derived protein of the present invention contained in plasmid pTB1939 obtained in REFERENCE EXAMPLE 1, and the amino acid sequence deduced therefrom of the human-derived protein of the present invention (continued from FIG. 2).
FIG. 4 shows a base sequence of DNA encoding the human-derived protein of the present invention contained in plasmid pTB1940 obtained in REFERENCE EXAMPLE 1, and the amino acid sequence deduced therefrom of the human-derived protein of the present invention (continued to FIG. 5).
FIG. 5 shows a base sequence of DNA encoding the human-derived protein-of the present invention contained in plasmid pTB1940 obtained in REFERENCE EXAMPLE 1, and the amino acid sequence deduced therefrom of the human-derived protein of the present invention (continued from FIG. 4 and to FIG. 6).
FIG. 6 shows a base sequence of DNA encoding the human-derived protein of the present invention contained in plasmid pTB1940 obtained in REFERENCE EXAMPLE 1, and the amino acid sequence deduced therefrom of the human-derived protein of the present invention (continued from FIG. 5).
FIG. 7 shows a base sequence of DNA encoding the mouse-derived protein of the present invention contained in plasmid pTB1958 obtained in REFERENCE EXAMPLE 2, and the amino acid sequence deduced therefrom of the mouse-derived protein of the present invention (continued to FIG. 8).
FIG. 8 shows a base sequence of DNA encoding the mouse-derived protein of the present invention contained in plasmid pTB1958 obtained in REFERENCE EXAMPLE 2, and the amino acid sequence deduced therefrom of the mouse-derived protein of the present invention (continued from FIG. 7).
FIG. 9 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued to FIG. 10).
FIG. 10 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued from FIG. 9 and to FIG. 11).
FIG. 11 shows a base sequence of genomic DNA encoding themouse-derived protein of the present invention contained in plasmid pTB2011 obtained in REFERENCE EXAMPLE 3, and the amino acid sequence deduced therefrom of the mouse-derived protein of the present invention (continued from FIG. 10 and to FIG. 12).
FIG. 12 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued from FIG. 11 and to FIG. 13).
FIG. 13 shows a base sequence of genomic DNA encoding themouse-derived protein of the present invention contained in plasmid pTB2011 obtained in REFERENCE EXAMPLE 3, and the amino acid sequence deduced therefrom of the mouse-derived protein of the present invention (continued from FIG. 12 and to FIG. 14).
FIG. 14 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued from FIG. 13 and to FIG. 15).
FIG. 15 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued from FIG. 14 and to FIG. 16).
FIG. 16 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued from FIG. 15 and to FIG. 17).
FIG. 17 shows a base sequence of genomic DNA encoding the mouse-derived protein of the present invention contained in plasmid pTB2011 obtained in REFERENCE EXAMPLE 3, and the amino acid sequence deduced therefrom of the mouse-derived protein of the present invention (continued from FIG. 16 and to FIG. 18).
FIG. 18 shows a base sequence of genomic DNA encoding themouse-derivedprotein of thepresent invention contained in plasmid pTB2011 obtained in REFERENCE EXAMPLE 3, and the amino acid sequence deduced therefrom of the mouse-derived protein of the present invention (continued from FIG. 17).

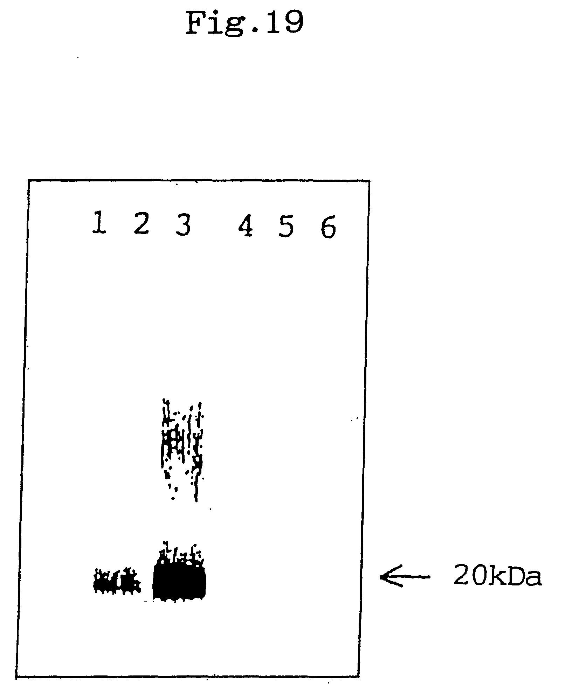

FIG. 19 shows the results of western blotting analysis on expression of a recombinant protein of human TL4 protein of the present invention in the extracellular region using Pichia yeast as a host in REFERENCE EXAMPLE 4, which analysis was made using antiserum to the protein. Lanes 1 to 3 designate the results obtained using the culture supernatant of human TL4 protein-expressed vector-inserted strain; and lanes 4 to 6 designate the results obtained using the culture supernatant of vector pPICZαA-inserted strain (wherein lanes 1 and 4 represent the results immediately after initiation of incubation in BMMY medium, lanes 2 and 5 represent 1 day after the incubation, and lanes 3 and 6 represent 2 days after the incubation). The band shows expression of the desired recombinant protein.
FIG. 20 shows a base sequence of DNA encoding the rat-derived protein of the present invention contained in plasmid pTB2012 obtained in REFERENCE EXAMPLE 5, and the amino acid sequence deduced therefrom of the rat-derived protein of the present invention (continued to FIG. 21).
FIG. 21 shows a base sequence of DNA encoding the rat-derived protein of the present invention contained in plasmid pTB2012 obtained in REFERENCE EXAMPLE 5, and the amino acid sequence deduced therefrom of the rat-derived protein of the present invention (continued from FIG. 20).
FIG. 22 is a schematic representation showing the construction of TL4 expression vector for insect cells used in EXAMPLE 1.
FIG. 23 shows the assay results of DNA synthesis promoting activity of normal human liver parenchymal cells by soluble human TL4, determined in EXAMPLE 2.
FIG. 24 shows the assay results of DNA synthesis promoting activity of normal human liver parenchymal cells by soluble human TL4 in starvation, determined in EXAMPLE 3.
FIG. 25 shows the assay results of cytotoxic activity of soluble human TL4 against liver parenchymal cells when used in combination with actinomycin D, determined in EXAMPLE 4.
FIG. 26 shows the detection results of apoptosis induction activity in EXAMPLE 5, using annexin V and propidium iodide.
FIG. 27 shows the assay results of synergistic promoting activity of soluble human TL4 and various growth factors in DNA synthesis of normal human liver parenchymal cells, determined in EXAMPLE 7.
FIG. 28 shows the assay results of change in expression of each gene in a mouse model with CCl₄-administered liver injury, determined in EXAMPLE 8.
FIG. 29 shows the results of change in gene expression in a mouse model with concanavalin A-administered liver injury, determined in EXAMPLE 10.
FIG. 30 shows the results of anti-apoptosis activity of soluble human TL4 against apoptosis of normal human liver parenchymal cells induced by actinomycin D and TNFα, determined in EXAMPLE 11.
FIG. 31 shows the results of anti-apoptosis activity of soluble human TL4 against apoptosis of normal human liver parenchymal cells induced by actinomycin D and TNFα, determined in EXAMPLE 11.

### BEST MODE FOR CARRYING OUT THE INVENTION

The protein contained in the liver function controlling agent of the present invention (hereinafter sometimes referred to as the protein of the present invention) contains the same or substantially the same amino acid sequence as the amino acid sequence represented by SEQ ID NO:1, SEQ ID NO:2, SEQ ID NO:3 or SEQ ID NO:31.

The protein of the present invention may be any protein derived from any cells of, for example, human and other warm-blooded animals (e.g., guinea pig, rat, mouse, chicken, rabbit, swine, sheep, bovine, horse, monkey, etc.) such as splenocyte, nerve cell, glial cell, β cell of pancreas, bone marrow cell, mesangial cell. Langerhans' cell, epidermic cell, epithelial cell, endothelial cell, fibroblast, fibrocyte, myocyte, fat cell, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, etc., the corresponding precursor cells, stem cells, cancer cells, etc.), or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine, small intestine and duodenum), blood vessel, heart, thymus, spleen, submandibular gland, peripheral blood, prostate, testis, ovary, placenta, uterus, bone, joint, skeletal muscle, etc. The protein of the present invention may also be a synthetic protein.

Examples of the protein in accordance with the present invention include proteins described in WO 98/03648 and WO 97/34911.

The protein of the present invention further includes proteins (proteins) having a ligand activity to the receptor proteins described in, e.g., J. Clin. Invest., 102, 1142-1151 (1998) and U.S. Patent No. 5,874,240, and the like.

The amino acid sequence which has substantially the same amino acid sequence as that represented by SEQ ID NO: 1, SEQ ID NO:2 or SEQ ID NO:3 includes an amino acid sequence having at least about 40% homology, preferably at least about 60% homology, more preferably at least about 80% homology, much more preferably at least about 90% homology, and most preferably at least about 95% homology, to the amino acid sequence represented by SEQ ID NO:1, SEQ ID NO:2 or SEQ ID NO:3.

It is particularly advantageous when the amino acid sequence has at least about 40% homology, preferably at least about 60% homology, more preferably at least about 80% homology and much more preferably at least about 90% homology, to the amino acid sequence of 84 - 240 in the amino acid sequence represented by SEQ ID NO:1, the amino acid sequence of 82-239 in the amino acid sequence represented by SEQ ID NO:2, or the amino acid sequence of 82-239 in the amino acid sequence represented by SEQ ID NO:3.

Preferred examples of substantially the same amino acid sequence as that represented by SEQ ID NO:1, SEQ ID NO:2 or SEQ ID NO:3 further include amino acid sequences containing, as the constituent amino acids, amino acid sequences of 8-21, 55-59, 93-102, 109-116, 118-126, 128-134, 144-149, 162-170, 176-182, 184-189, 193-213, 215-219 and 228-239 in the amino acid sequence represented by SEQ ID NO:1. These amino acid sequences are the amino acid sequences that are common to the amino acid sequences represented by SEQ ID NO:1, SEQ ID NO:2 and SEQ ID NO:3.

Preferred examples of substantially the same amino acid sequence as that represented by SEQ ID NO:1 or SEQ ID NO:2 further include amino acid sequences containing, as the constituent amino acids, amino acid sequences of 8-21, 54-59, 93-102, 109-116, 118-126, 128-134, 144-149, 162-170, 176-182, 184-189, 193-213, 215-219 and 228-240 in the amino acid sequence represented by SEQ ID NO:1. These amino acid sequences are the amino acid sequences corresponding to the amino acid sequences of 6-20, 52-57, 91-100, 107-114, 116-124, 126-132, 142-147, 162-170, 176-182, 184-189, 192-212, 214-218 and 227-239 that are common to the amino acid sequences represented by SEQ ID NO:1 and SEQ ID NO:2.

Examples of substantially the same amino acid sequence as that represented by SEQ ID NO:31 include amino acid sequences containing, as the constituent amino acids, amino acid sequences of 8-21, 57-66, 73-80, 82-90, 92-98, 108-111, 126-134, 140-146, 148-153, 157-177, 179-183 and 192-204 in the amino acid sequence represented by SEQ ID NO:31.

As described above, proteins containing substantially the same amino acid sequence as that represented by SEQ ID NO:1, SEQ ID NO:2, SEQ ID NO:3 or SEQ ID NO:31 and having the activity substantially equivalent to that of the protein containing the amino acid sequence represented by SEQ ID NO:1, SEQ ID NO:2, SEQ ID NO:3 or SEQ ID NO:31, and the like are preferred as the protein of the present invention containing substantially the same amino acid sequence as that represented by SEQ ID NO:1, SEQ ID NO:2, SEQ ID NO:3 or SEQ ID NO:31.

Examples of the substantially equivalent activity include a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc. The term "substantially equivalent" is used to mean that the nature of these activities is equivalent (for example, biochemically or pharmacologically). Therefore, it is preferred that the these activities such as a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specif ically, anactivity of promoting transfer from the G0 phase to the G1 phase in the cell cycle (preferably, the cell cycle of liver cells), etc. are equivalent in strength (e.g., about 0.01 to about 20 times, preferably about 0.2 to 5 times, and more preferably about 0.5 to about 2 times). Even differences among grades such as the strength of these activities and molecular weight of the proteins may be allowably present.

The activities such as a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), or the like can be determined by publicly known methods, or modified methods (methods described in, e.g., Eisuke Mekada, Yasuko Miyake and Yoshio Okada, "Gan to Kagaku Ryoho (Cancer & Chemotherapy)", 4, 407 (1977), etc.) and further by means of screening which will be later described.

The proteins of the present invention also include so-called muteins such as proteins containing (i) an amino acid sequence represented by SEQ ID NO:1, SEQ ID NO:2, SEQ ID NO:3 or SEQ ID NO:31, of which at least 1 or 2 (preferably 1 to 80, more preferably 1 to about 20, more preferably 1 to about 9, and most preferably several (e.g., 1 to 5)) amino acids are deleted; (ii) an amino acid sequence represented by SEQ ID NO:1, SEQ ID NO:2, SEQ ID NO:3 or SEQ ID NO:31, to which at least 1 or 2 (preferably 1 to 80, more preferably 1 to about 20, more preferably 1 to about 9, and most preferably several (e.g., 1 to 5)) amino acids are added; (iii) an amino acid sequence represented by SEQ ID NO:1, SEQ ID NO:2, SEQ ID NO:3 or SEQ ID NO:31, in which at least 1 or 2 (preferably 1 to 80, more preferably 1 to about 20, more preferably 1 to about 9, and most preferably several (e.g., 1 to 5)) amino acids are substituted by other amino acids; and (iv) a combination of the above amino acid sequences.

In the case that the amino acid sequence is deleted or substituted as described above, the positions of the deletion or substitution are not particularly restricted but examples of the positions are (i) the positions other than the amino acid sequences of 8-21, 55-59 (or 54-59), 93-102, 109-116, 118-126, 128-134, 144-149, 162-170, 176-182, 184-189, 193-213, 215-219 and 228-239 (or 228-240) in the amino acid sequence represented by SEQ ID NO:1, preferably the position other than the amino acid sequences of 93-102, 109-116, 118-126, 128-134, 144-149, 162-170, 176-182, 184-189, 193-213, 215-219 or 228-240 in the amino acid sequence represented by SEQ ID NO:1; (ii) the positions other than the amino acid sequences of 6-19, 53-57 (or 52-57), 91-100, 107-114, 116-124, 126-132, 142-147, 162-170, 176-182, 184-189, 192-212, 214-218 or 227-238 (or 227-239) in the amino acid sequence represented by SEQ ID NO:2, preferably the position other than the amino acid sequences of 91-100, 107-114, 116-124, 126-132, 142-147, 162-170, 176-182, 184-189, 192-212, 214-218 or 227-239 in the amino acid sequence represented by SEQ ID NO: 2; (iii) the positions other than the amino acid sequences of 6-19, 53-57 (or 52-57), 91-100, 107-114, 116-124, 126-132, 142-147, 162-170, 176-182, 184-189, 192-212, 214-218 or 227-238 (or 227-239) in the amino acid sequence represented by SEQ ID NO:3, preferably the position other than the amino acid sequences of 91-100, 107-114, 116-124, 126-132, 142-147, 162-170, 176-182, 184-189, 192-212, 214-218 or 227-239 in the amino acid sequence represented by SEQ ID NO:3; and (iv) the positions other than the amino acid sequences of 8-21, 57-66 73-80, 82-90, 92-98, 108-111, 126-134, 140-146, 148-153, 157-177, 179-183 and 192-204 in the amino acid sequence represented by SEQ ID NO:31.

As the protein containing substantially the same as the amino acid sequence represented by SEQ ID NO:1, SEQ ID NO:2 or SEQ ID NO:3, there is also preferably used a protein containing the amino acid sequence specifically shown by general formula [the amino acid sequence represented by SEQ ID NO:25]: 
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30
Cys Xaa Xaa Xaa Xaa Val Xaa Leu Xaa Leu Xaa Leu Leu Leu Xaa Gly
        35                  40                  45
Ala Gly Leu Ala Xaa Gln Gly Trp Phe Leu Leu Xaa Leu His Xaa Arg
    50                  55                  60
Leu Gly Xaa Xaa Vla Xaa Xaa Leu Pro Asp Gly Xaa Xaa Gly Ser Trp
65                  70                  75                  80
Glu Xaa Leu Ile Gln Xaa Xaa Arg Ser His Xaa Xaa Asn Pro Ala Ala
                85                  90                  95
His Leu Thr Gly Ala Asn Xaa Ser Leu Xaa Gly Xaa Gly Gly Pro Leu
            100                 105                 110
Leu Trp Glu Thr Xaa Leu Gly Leu Ala Phe Leu Arg Gly Leu Xaa Tyr
 
115 120 125
His Asp Gly Ala Leu Val Xaa Xaa Xaa Xaa Gly Tyr Tyr Tyr Xaa Tyr
130 135 140
Ser Lys Val Gln Leu Xaa Gly Val Gly Cys Pro Xaa Gly Leu Ala Xaa
145 150 155 160
Xaa Xaa Xaa Ile Thr His Gly Leu Tyr Lys Arg Thr Xaa Arg Tyr Pro
165 170 175
Glu Xaa Leu Glu Leu Leu Val Ser Xaa Xaa Ser Pro Cys Gly Arg Ala
180 185 190
Xaa Xaa Ser Ser Arg Val Trp Trp Asp Ser Ser Phe Leu Gly Gly Val
195 200 205
Val His Leu Glu Ala Gly Glu Xaa Val Val Val Arg Val Xaa Xaa Xaa
210 215 220
Arg Leu Val Arg Xaa Arg Asp Gly Thr Arg Ser Tyr Phe Gly Ala Phe
225 230 235 240
Met Val (I)
 (wherein Xaa is an optional amino acid residue or a bond).

In the general formula (I) described above, Xaa may be deleted at the position of at least 1 or 2 (e.g., 1 to 56, preferably 1 to 40, more preferably 1 to 20, much more preferably 1 to 9, and most preferably several (1 to 5) positions).

The amino acid shown by Xaa may be either hydrophilic or hydrophobic amino acid and may be any one of acidic, basic and neutral amino acids. Specific examples of the amino acid employed are Gly, Ala, Val, Leu, Ile, Ser, Thr, Cys, Met, Glu, Asp, Lys, Arg, His, Phe, Tyr, Trp, Pro, Asn, Gln, etc.

In the general formula (I), the third Xaa is preferably Glu or may be deleted.

The 7th Xaa is preferably a hydrophilic amino acid and specifically, Arg or Gln is preferred.

The 22nd Xaa is preferably a hydrophilic amino acid and specifically, Thr or Arg is preferred.

The 25th Xaa is preferably a hydrophilic amino acid and specifically, Gly or Glu is preferred.

The 26th Xaa is preferably a hydrophilic amino acid and specifically, Arg or Gln is preferred.

The 27th Xaa is preferably a hydrophilic amino acid and specifically, Ser or Asn is preferred.

The 31st Xaa is preferably a hydrophilic amino acid and specifically, GIn or Arg is preferred.

The 32nd Xaa is preferably a hydrophilic amino acid and specifically, Ser or Arg is preferred.

The 34th Xaa is preferably a hydrophilic amino acid and specifically, Ser or Gly is preferred.

The 35th Xaa is preferably, e.g., Val or Thr.

The 36th Xaa is preferably, e.g., a hydrophobic amino acid and specifically, Ala or Val is preferred.

The 37th Xaa is preferably, e.g., a hydrophilic amino acid and specifically, Arg or Gln is preferred.

The 39th Xaa is preferably, e.g., a hydrophilic amino acid and specifically, Gly or Ser is preferred.

The 41st Xaa is preferably, e.g., Gly or Ala.

The 43rd Xaa is preferably, e.g., a hydrophobic amino acid and specifically, Leu or Val is preferred.

The 47th Xaa is preferably Met or may be deleted.

The 53rd Xaa is preferably, e.g., Val or Thr.

The 60th Xaa is preferably, e.g., a hydrophilic amino acid and specifically, Gln or Arg is preferred.

The 63rd Xaa is preferably, e.g., Trp or Gln.

The 67th Xaa is preferably, e.g., an acidic amino acid and specifically, Glu or Asp is preferred.

The 68th Xaa is preferably, e.g., a hydrophobic amino acid and specifically, Met or Ile is preferred.

The 70th Xaa is preferably, e.g., Thr or Ala.

The 71st Xaa is preferably, e.g., a basic amino acid and specifically, Arg or His is preferred.

The 76th Xaa is preferably, e.g., Pro or Gly.

The 77th Xaa is preferably, e.g., Ala or Lys.

The 82nd Xaa is preferably, e.g., a hydrophilic amino acid and specifically, Gln or Lys is preferred.

The 86th Xaa is preferably, e.g., an acidic amino acid and specifically, Glu or Asp is preferred.

The 87th Xaa is preferably, e.g., a hydrophilic amino acid and specifically, Arg or Gln is preferred.

The 91st Xaa is preferably, e.g., a hydrophilic amino acid and specifically, Glu or Gln is preferred.

The 92nd Xaa is preferably, e.g., a hydrophobic amino acid and specifically, Val or Ala is preferred.

The 103rd Xaa is preferably, e.g., Ser or Ala.

The 106th Xaa is preferably, e.g., Thr or Ile.

The 108th Xaa is preferably, e.g., Ser or Ile.

The 117th Xaa is preferably, e.g., a hydrophilic amino acid and specifically, Gln or Arg is preferred.

The 127th Xaa is preferably, e.g., a hydrophilic amino acid and specifically, Ser or Thr is preferred.

The 135th Xaa is preferably, e.g., Val or Thr.

The 136th Xaa is preferably, e.g., Thr or Met.

The 137th Xaa is preferably, e.g., a hydrophilic amino acid and specifically, Lys or Glu is preferred.

The 138th Xaa is preferably, e.g., a hydrophobic amino acid and specifically, Ala or Pro is preferred.

The 143rd Xaa is preferably, e.g., a hydrophobic amino acid and specifically, Ile or Val is preferred.

The 150th Xaa is preferably, e.g., a hydrophilic amino acid and specifically, Gly or Ser is preferred.

The 156th Xaa is preferably, e.g., Leu or Gly.

The 160th Xaa is preferably, e.g., a hydrophilic amino acid and specifically, Ser or Asn is preferred.

The 161st Xaa is preferably, e.g., a hydrophilic amino acid and specifically, Thr or Gly is preferred.

The 162nd Xaa is preferably Leu or may be deleted.

The 163rd Xaa is preferably Pro or may be deleted.

The 173rd Xaa is preferably, e.g., Pro or Ser.

The 178th Xaa is preferably, e.g., a hydrophilic amino acid and specifically, Glu or Lys is preferred.

The 185th and 186th Xaa are preferably, e.g., a hydrophilic amino acid and specifically, Gln or Arg is preferred.

The 193rd Xaa is preferably Thr or may be deleted.

The 194th Xaa is preferably, e.g., a hydrophilic amino acid and specifically, Ser or Asn is preferred.

The 216th Xaa is preferably, e.g., a hydrophilic amino acid and specifically, Lys or Glu is preferred.

The 222nd Xaa is preferably, e.g., a hydrophobic amino acid and specifically, Leu or Pro is preferred.

The 223rd Xaa is preferably, e.g., a hydrophilic amino acid and specifically, Asp or Gly is preferred.

The 224th Xaa is preferably, e.g., a hydrophilic amino acid and specifically, Glu or Asn is preferred.

The 229th Xaa is preferably, e.g., a hydrophobic amino acid and specifically, Leu or Pro is preferred.

As the protein containing substantially the same as the amino acid sequence represented by SEQ ID NO:31. there is also preferably used a protein containing the amino acid sequence specifically shown by general formula [the amino acid sequence represented by SEQ ID NO:47]: 
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30
Cys Xaa Xaa Xaa Xaa Asp Gly Xaa Xaa Gly Ser Trp Glu Xaa Leu Ile
        35                  40                  45
Gln Xaa Xaa Arg Ser His Xaa Xaa Asn Pro Ala Ala His Leu Thr Gly
    50                  55                  60
Ala Asn Xaa Ser Leu Xaa Gly Xaa Gly Gly Pro Leu Leu Trp Glu Thr
65                  70                  75                  80
Xaa Leu Gly Leu Ala Phe Leu Arg Gly Leu Xaa Tyr His Asp Gly Ala
 
                85                  90                  95
Leu Val Xaa Xaa Xaa Xaa Gly Tyr Tyr Tyr Xaa Tyr Ser Lys Val Gln
            100                 105                 110
Leu Xaa Gly Val Gly Cys Pro Xaa Gly Leu Ala Xaa Xaa Ile Thr His
        115                 120                 125
Gly Leu Tyr Lys Arg Thr Xaa Arg Tyr Pro Glu Xaa Leu Glu Leu Leu
    130                 135                 140
Val Ser Xaa Xaa Ser Pro Cys Gly Arg Ala Xaa Xaa Ser Ser Arg Val
145                 150                 155                 160
Trp Trp Asp Ser Ser Phe Leu Gly Gly Val Val His Leu Glu Ala Gly
                165                 170                 175
Glu Xaa Val Val Val Arg Val Xaa Xaa Xaa Arg Leu Val Arg Xaa Arg
            180                 185                 190
Asp Gly Thr Arg Ser Tyr Phe Gly Ala Phe Met Val (II)
195 200 204
 (wherein Xaa is an optional amino acid residue or a bond).

In the general formula (II) described above, Xaa may be deleted at the position of at least 1 or 2 (e.g., 1 to 40, preferably 1 to 20, more preferably 1 to 9, and most preferably several (1 to 5) positions).

The amino acid shown by Xaa may be either hydrophilic or hydrophobic amino acid and may be any one of acidic, basic and neutral amino acids. Specific examples of the amino acid employed are Gly, Ala, Val, Leu, Ile, Ser, Thr, Cys, Met, Glu, Asp, Lys, Arg, His, Phe, Tyr, Trp, Pro, Asn, Gln, etc.

In the general formula (II), the third Xaa is preferably Glu or may be deleted.

The 7th Xaa is preferably a hydrophilic amino acid and specifically, Arg or Gln is preferred.

The 22nd Xaa is preferably a hydrophilic amino acid and specifically, Thr or Arg is preferred.

The 25th Xaa is preferably a hydrophilic amino acid and specifically, Gly or Glu is preferred.

The 26th Xaa is preferably a hydrophilic amino acid and specifically, Arg or Gln is preferred.

The 27th Xaa is preferably a hydrophilic amino acid and specifically, Ser or Asn is preferred.

The 31st Xaa is preferably a hydrophilic amino acid and specifically, Gln or Arg is preferred.

The 32nd Xaa is preferably a hydrophilic amino acid and specifically, Ser or Arg is preferred.

The 34th Xaa is preferably a hydrophilic amino acid and specifically, Ser or Gly is preferred.

The 35th Xaa is preferably, e.g., Val or Thr.

The 36th Xaa is preferably, e.g., a hydrophobic amino acid and specifically, Ala or Val is preferred.

The 37th Xaa is preferably, e.g., a hydrophilic amino acid and specifically, Arg or Gln is preferred.

The 40th Xaa is preferably, e.g., Pro or Gly.

The 41st Xaa is preferably, e.g., Ala or Lys.

The 46th Xaa is preferably, e.g., a hydrophilic amino acid and specifically, Gln or Lys is preferred.

The 50th Xaa is preferably, e.g., an acidic amino acid and specifically, Glu or Asp is preferred.

The 51st Xaa is preferably, e.g., a hydrophilic amino acid and specifically, Arg or Gln is preferred.

The 55th Xaa is preferably, e.g., a hydrophilic amino acid and specifically, Glu or Gln is preferred.

The 56th Xaa is preferably, e.g., a hydrophobic amino acid and specifically, Val or Ala is preferred.

The 67th Xaa is preferably, e.g., Ser or Ala.

The 70th Xaa is preferably, e.g., Thr or Ile.

The 72nd Xaa is preferably, e.g., Ser or Ile.

The 81st Xaa is preferably, e.g., a hydrophilic amino acid and specifically, Gln or Arg is preferred.

The 91st Xaa is preferably, e.g., a hydrophilic amino acid and specifically, Ser or Thr is preferred.

The 99th Xaa is preferably, e.g., Val or Thr.

The 100th Xaa is preferably, e.g., Thr or Met.

The 101st Xaa is preferably, e.g., a hydrophilic amino acid and specifically, Lys or Glu is preferred.

The 102nd Xaa is preferably, e.g., a hydrophobic amino acid and specifically, Ala or Pro is preferred.

The 107th Xaa is preferably, e.g., a hydrophobic amino acid and specifically, Ile or Val is preferred.

The 114th Xaa is preferably, e.g., a hydrophilic amino acid and specifically, Gly or Ser is preferred.

The 120th Xaa is preferably, e.g., Leu or Gln.

The 124th Xaa is preferably, e.g., a hydrophilic amino acid and specifically, Ser or Asn is preferred.

The 125th Xaa is preferably, e.g., a hydrophilic amino acid and specifically, Thr or Gly is preferred.

The 135th Xaa is preferably Pro or may be deleted.

The 140th Xaa is preferably, e.g., a hydrophilic amino acid and specifically, Glu or Lys is preferred.

The 147th and 148th Xaa are preferably, e.g., a hydrophilic amino acid and specifically, Gln or Arg is preferred.

The 155th Xaa is preferably Thr or may be deleted.

The 156th Xaa is preferably, e.g., a hydrophilic amino acid and specifically, Ser or Asn is preferred.

The 178th Xaa is preferably, e.g., a hydrophilic amino acid and specifically, Lys or Glu is preferred.

The 184th Xaa is preferably, e.g., a hydrophobic amino acid and specifically, Leu or Pro is preferred.

The 185th Xaa is preferably, e.g., a hydrophilic amino acid and specifically, Asp or Gly is preferred.

The 186th Xaa is preferably, e.g., a hydrophilic amino acid and specifically, Glu or Asn is preferred.

The 191st Xaa is preferably, e.g., a hydrophobic amino acid and specifically, Leu or Pro is preferred.

In the present specification, the protein is represented in accordance with the conventional way of describing peptides, that is, the N-terminus (amino terminus) at the left hand and the C-terminus (carboxyl terminus) at the right hand. In the proteins of the present invention, including the protein containing the amino acid sequence shown by SEQ ID NO:1, the C-terminus is usually in the form of a carboxyl group (-COOH) or a carboxylate (-COO⁻) but may also be in the form of an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a phenyl-C₁₋₂ alkyl group, e.g., benzyl, phenethyl, etc.; a C₇₋₁₄ aralkyl such as an α-naphthyl-C₁₋₂ alkyl group, e.g., α-naphthylmethyl, etc.; and the like. In addition, pivaloyloxymethyl or the like which is widely used as an ester for oral administration may be used as well.

Where the protein of the present invention contains a carboxyl group (or a carboxylate) at a position other than the C-terminus, it may be amidated or esterified and such an amide or ester is also included within the protein of the present invention. Examples of the ester group used are those described above with respect to the C-terminal esters.

Furthermore, examples of the protein of the present invention include variants of the above proteins, wherein the amino group at the N-terminus of the protein is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those whereinasubstituent (e.g., -OH, -SH, aminogroup, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formylgroup, acetyl group, etc.), orconjugated proteins such as glycoproteins having sugar chains.

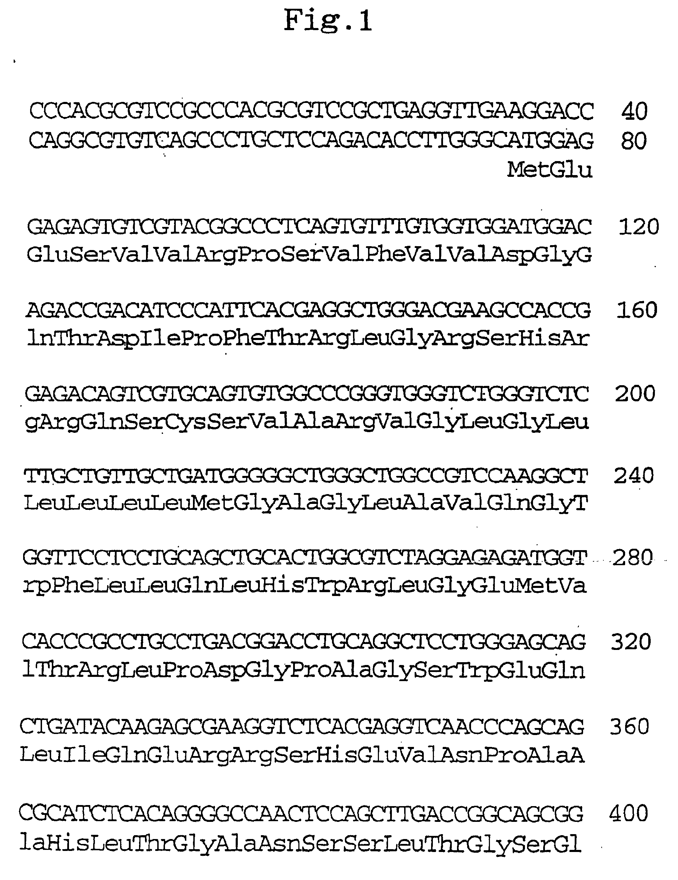

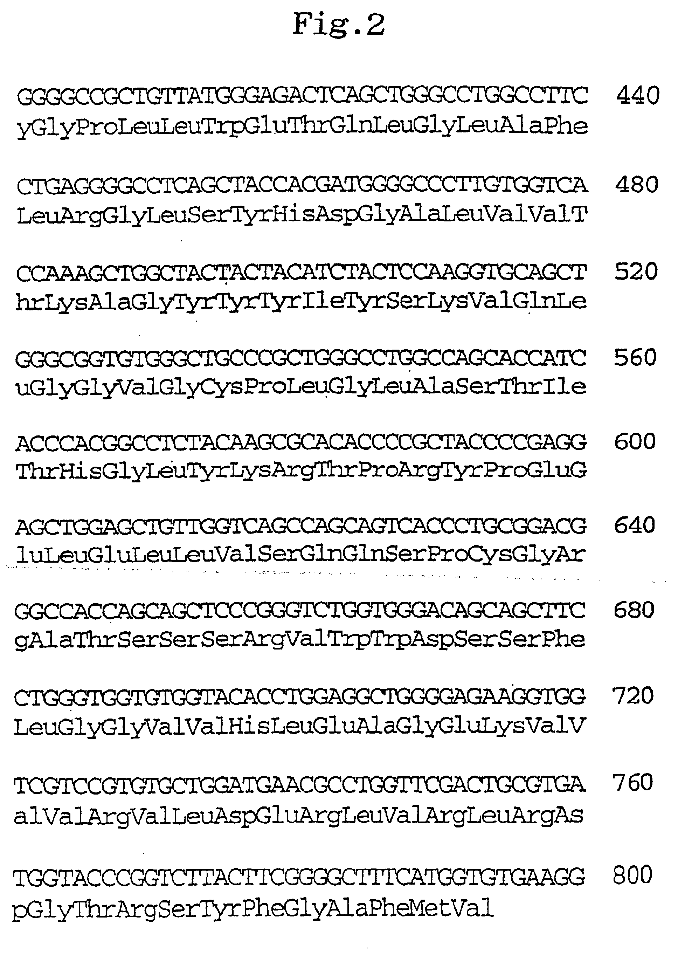

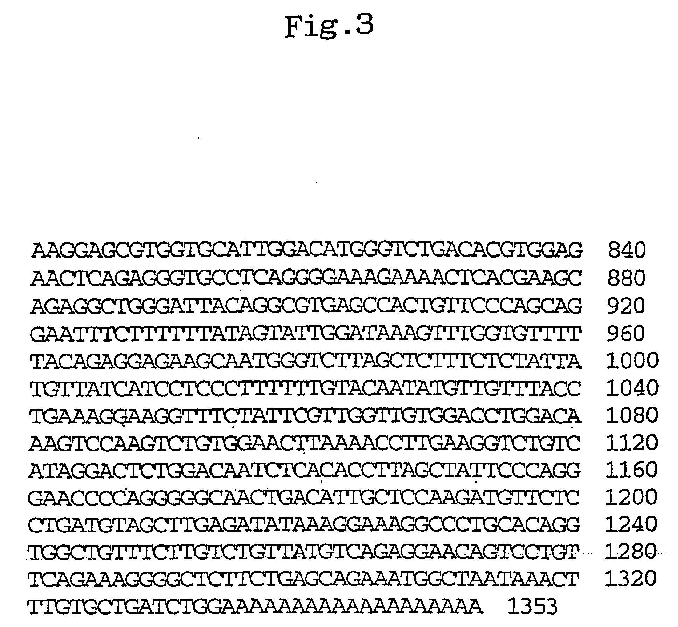

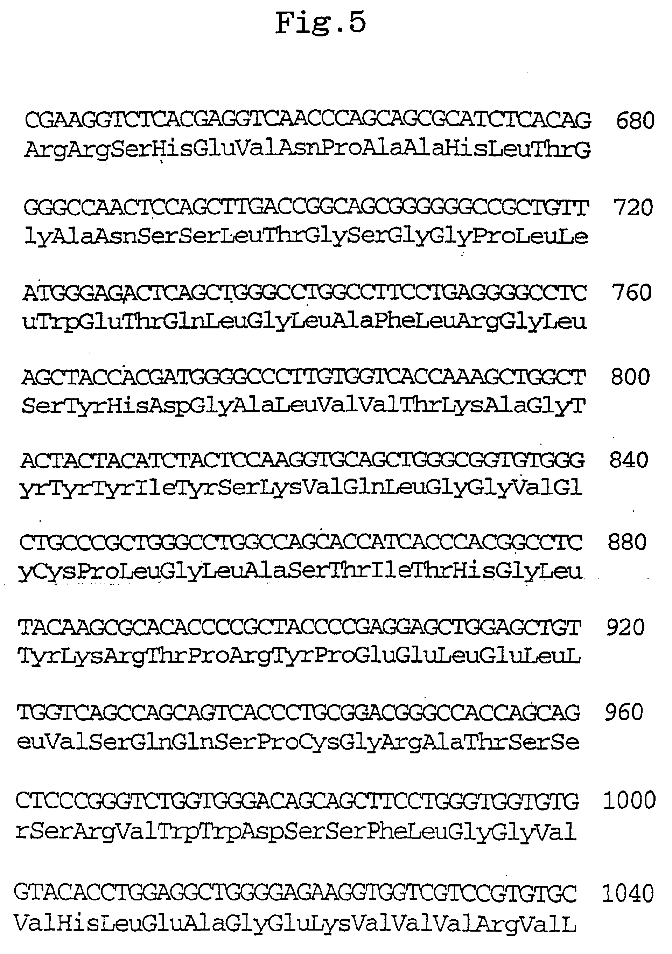

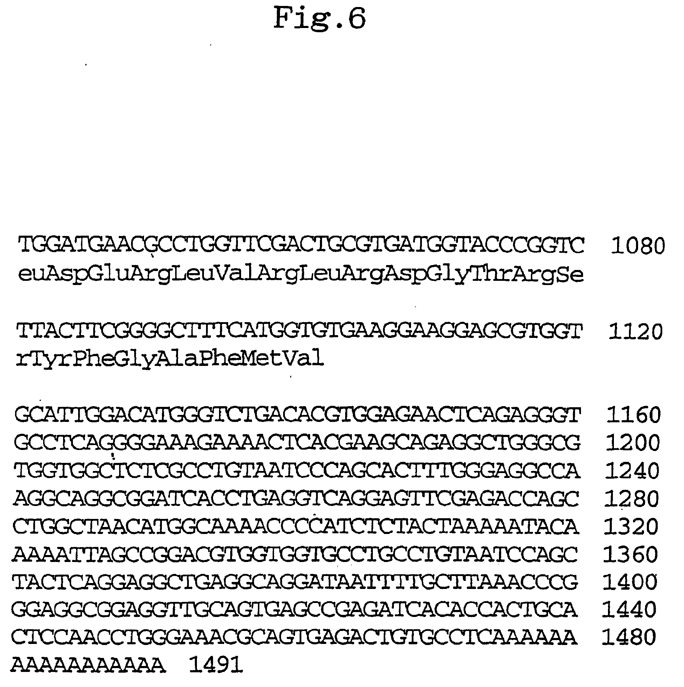

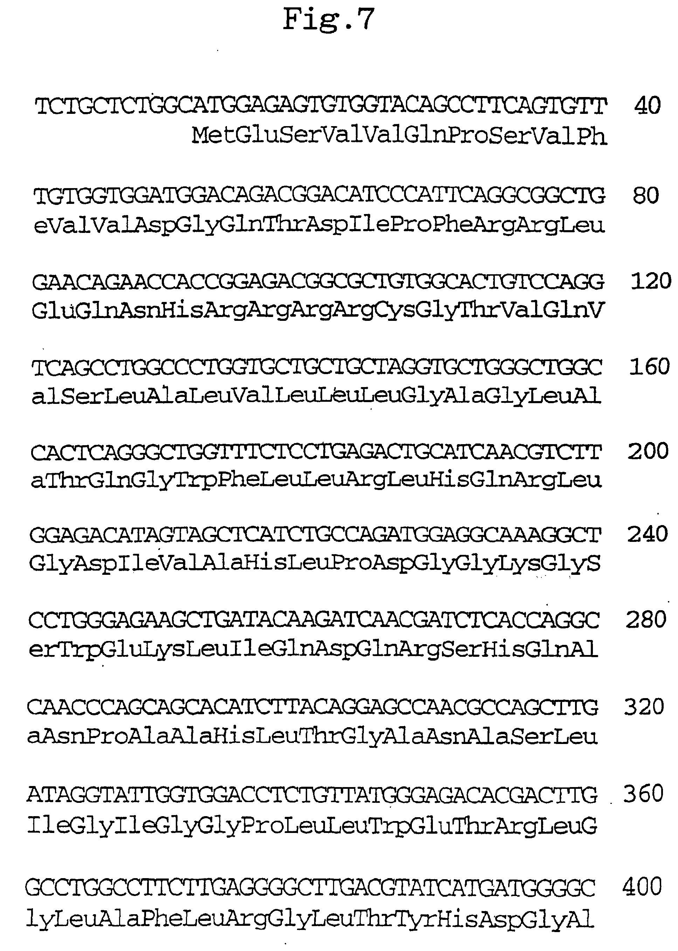

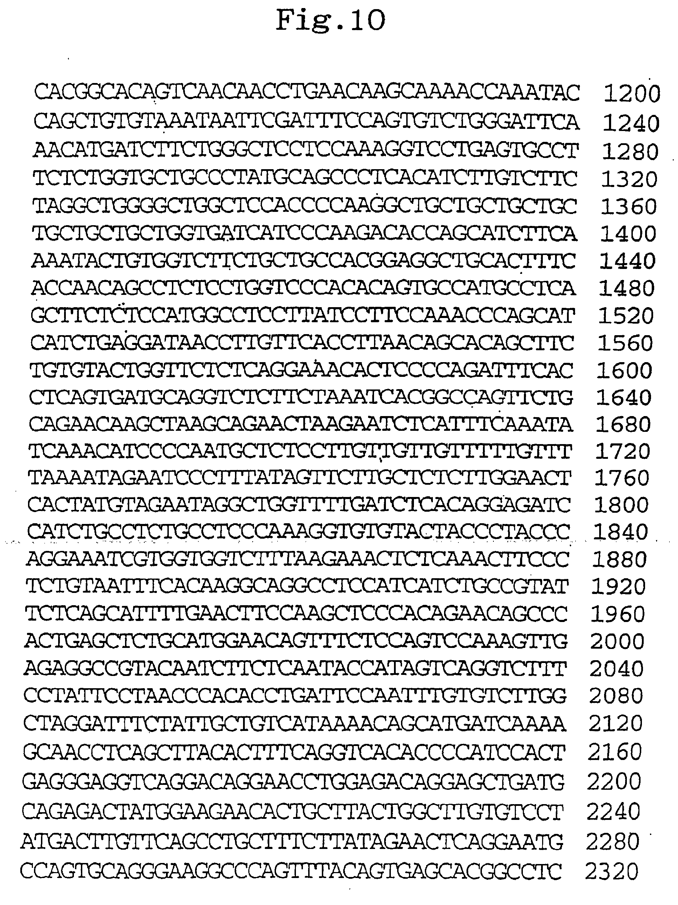

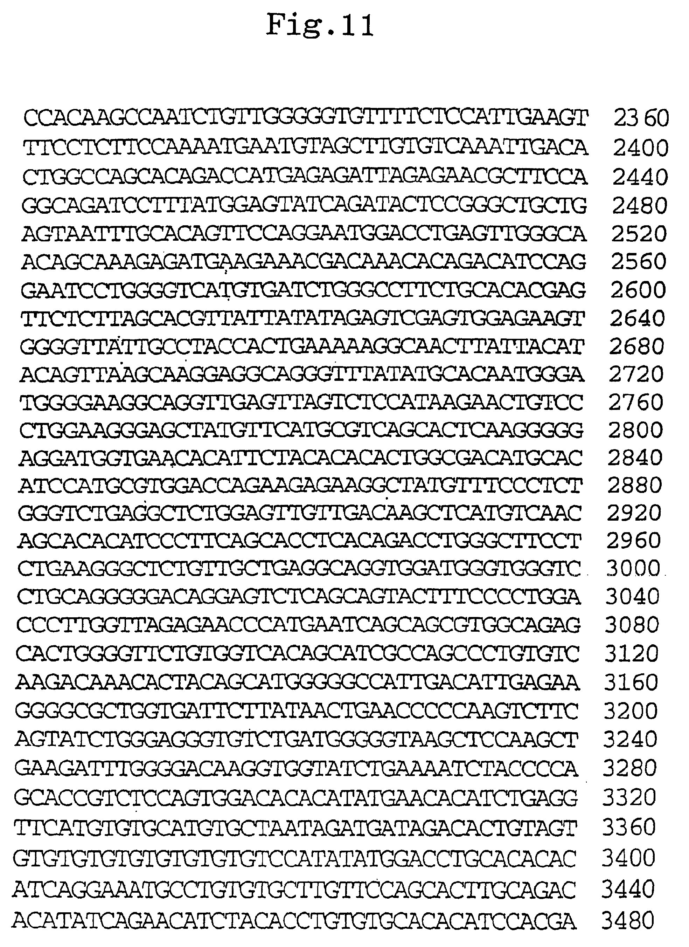

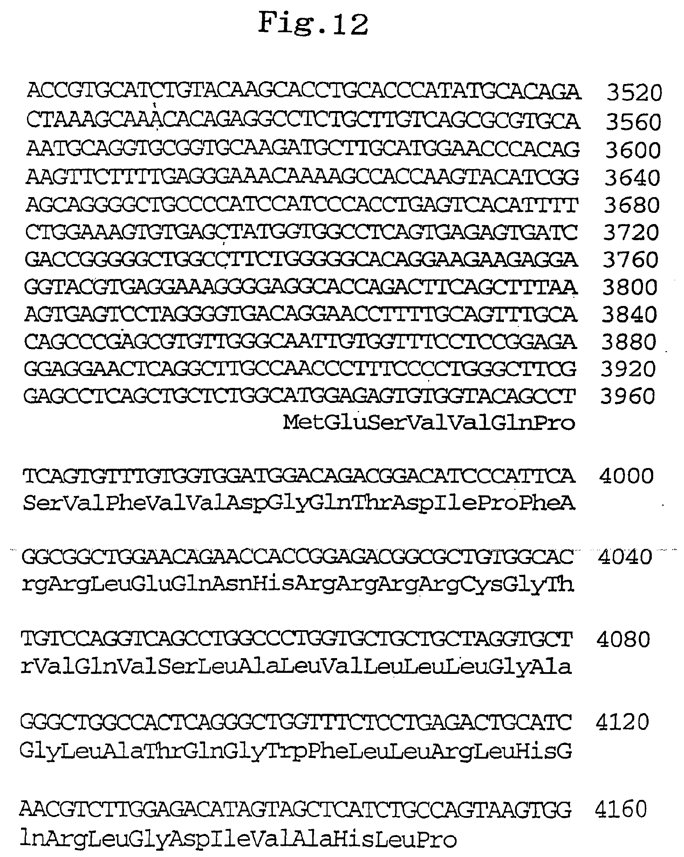

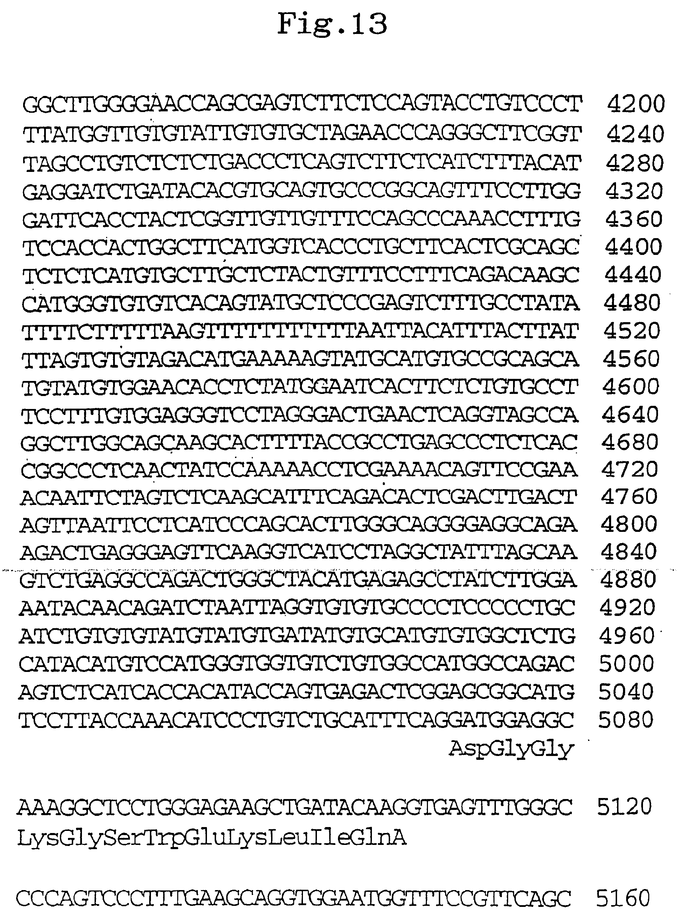

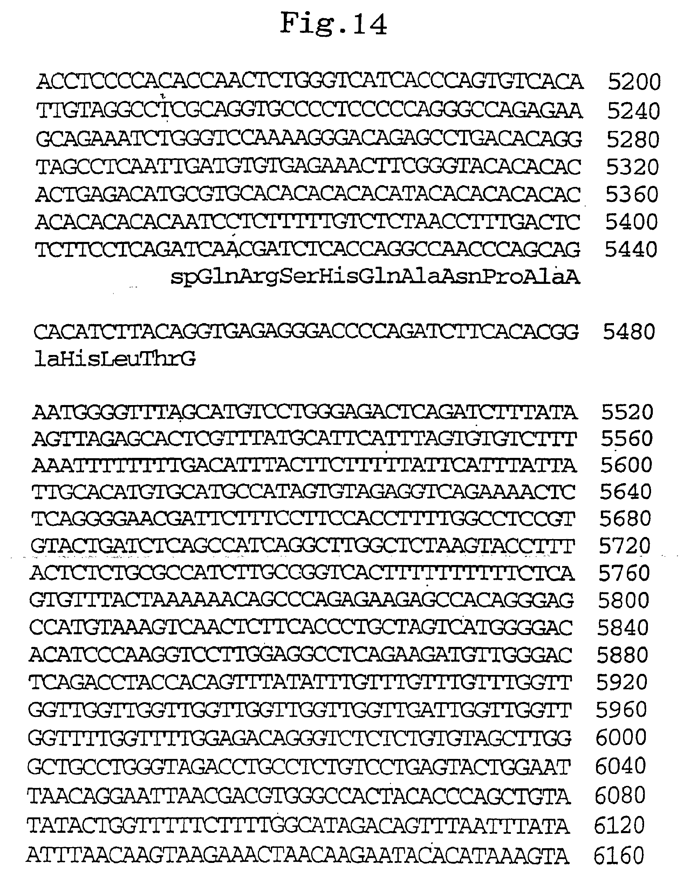

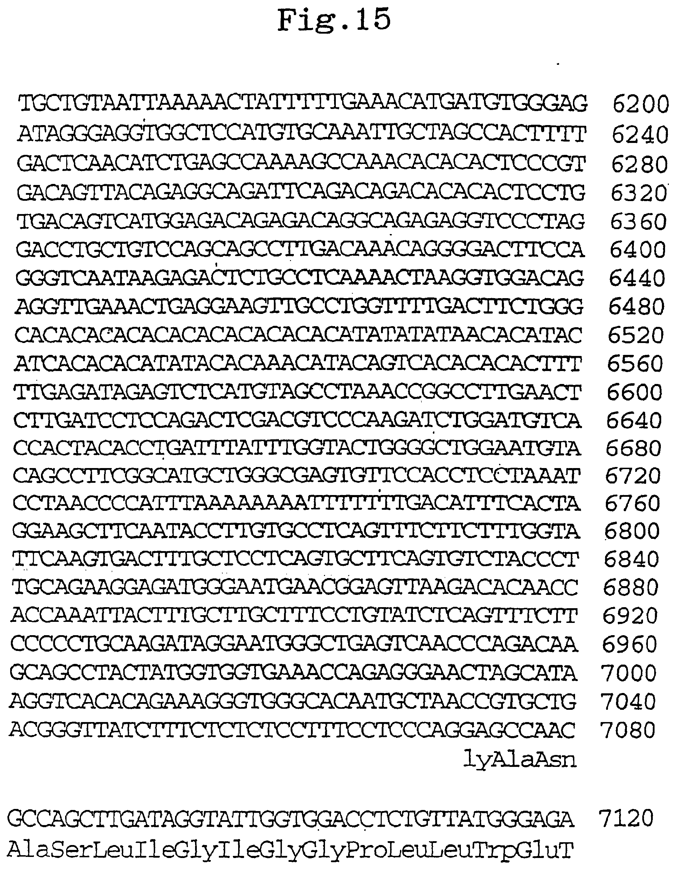

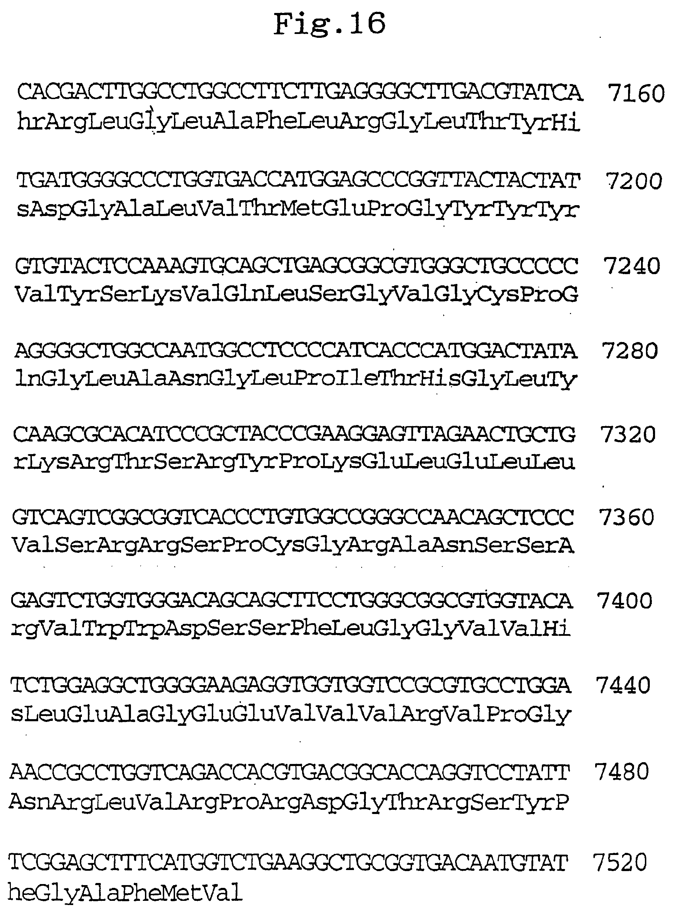

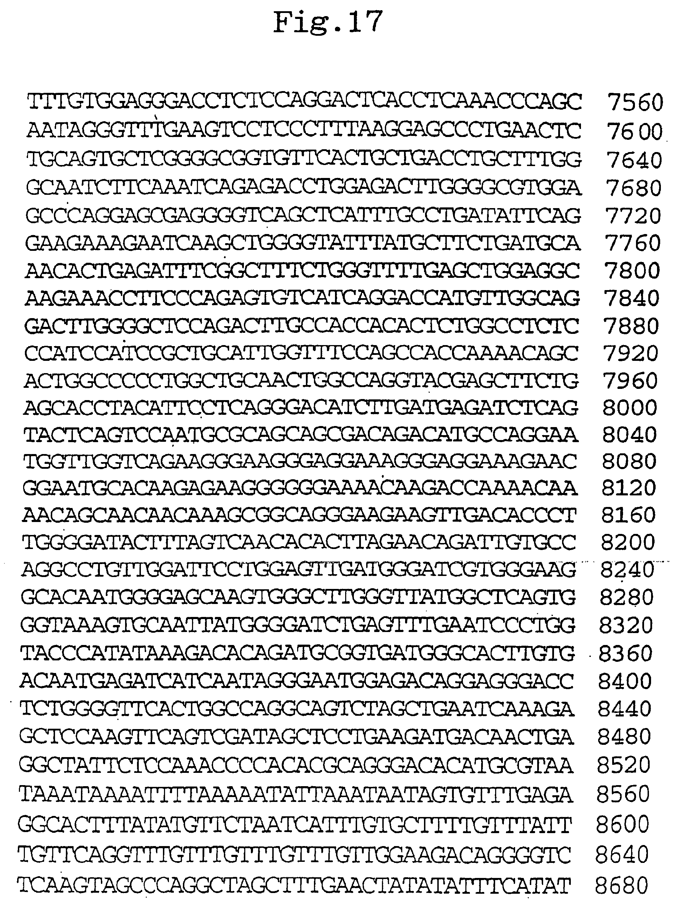

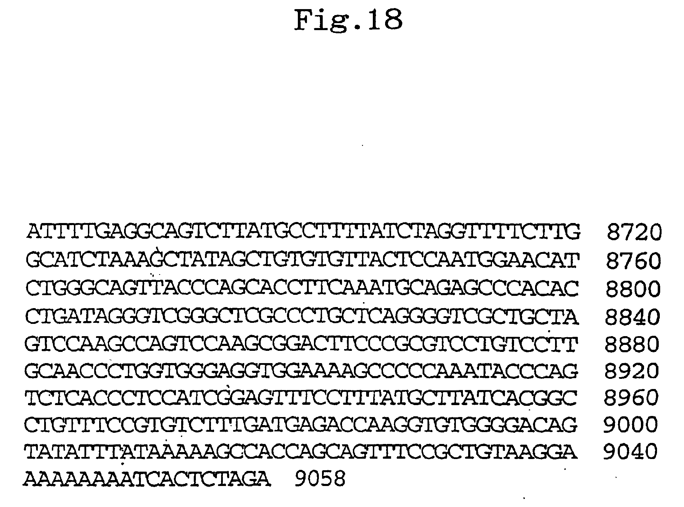

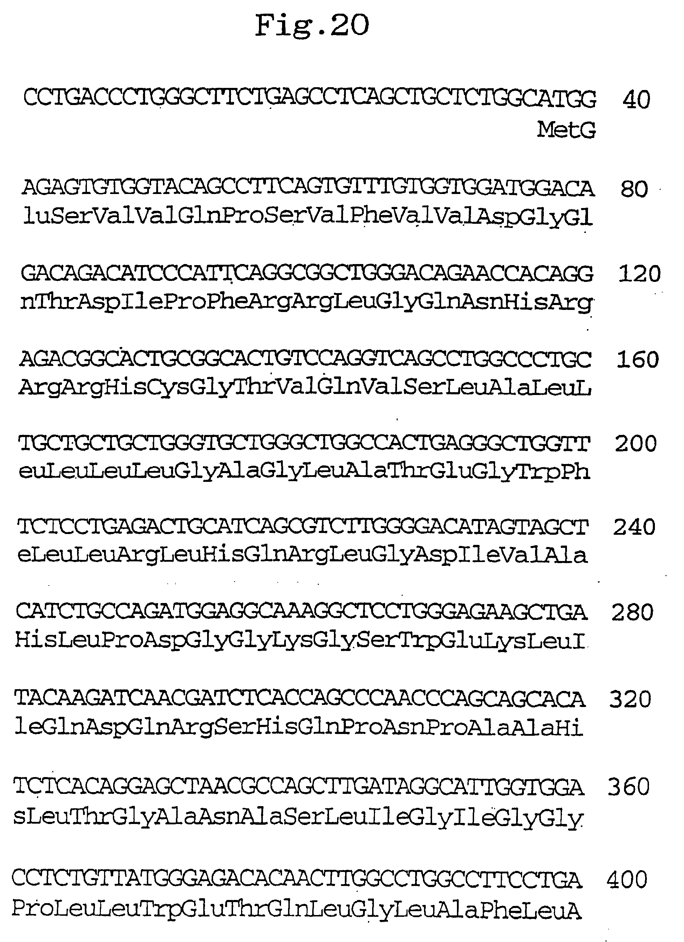

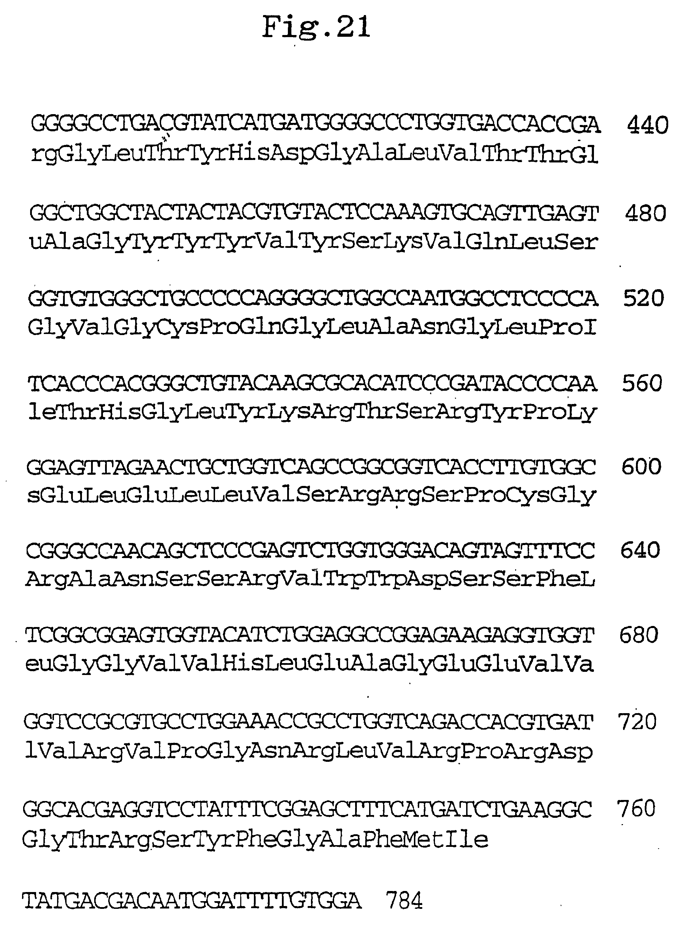

More specifically, a human liver-derived protein containing the amino acid sequence represented by SEQ ID NO:1 (FIGS. 1 through 3 or FIGS. 4 through 6), a mouse embryo-derived protein containing the amino acid sequence represented by SEQ ID NO:2 (FIGS. 7 and 8 or FIGS. 9 through 18), and a rat liver-derived protein containing the amino acid sequence represented by SEQ ID NO:3 (FIGS. 20 and 21) and a human liver-derived protein containing the amino acid sequence represented by SEQ ID NO:31, and the like are preferred as the protein of the present invention.

The partial peptides of the proteins in accordance with the present invention may be any peptides so long as they are peptides having activities equivalent to those of the proteins of the present invention described above, for example, a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc. For example, peptides containing at least about 20 amino acids, preferably about 50 or more, more preferably about 70 or more, much more preferably about 100 or more and most preferably about 200 or more, in the amino acid sequences of the proteins of the present invention are advantageously employed.

There are employed, for example, (1) a partial peptide containing at least one amino acid sequence selected from the amino acid sequences of 8-21, 55-59, 93-102, 109-116, 118-126, 128-134, 144-149, 162-170. 176-182, 184-189, 193-213, 215-219 and 228-239 in the amino acid sequence represented by SEQ ID NO:1 (i.e., a partial peptide containing at least one amino acid sequence selected from the amino acid sequences of 6-20, 53-57, 91-100, 107-114, 116-124, 126-132, 142-147, 162-170, 176-182, 184-189, 192-212, 214-218 and 227-238, in the amino acid sequence represented by SEQ ID NO:2 or SEQ ID NO:3); (2) a partial peptide containing at least one amino acid sequence selected from the amino acid sequences of 8-21, 54-59, 93-102, 109-116, 118-126, 128-134, 144-149, 162-170, 176-182, 184-189, 193-213, 215-219 and 228-240 in the amino acid sequence represented by SEQ ID NO:1 (i.e., a partial peptide containing at least one amino acid sequence selected from the amino acid sequences of 6-20, 52-57, 91-100, 107-114, 116-124, 126-132, 142-147, 162-170, 176-182, 184-189, 192-212, 214-218 and 227-239, in the amino acid sequence represented by SEQ ID NO:2 or SEQ ID NO: 3) ; and (3) a partial peptide containing at least one amino acid sequence selected from the amino acid sequences of 8-21, 57-66, 73-80, 82-90, 92-98,108-113,126-134, 140-146, 148-)53, 157-177, 179-183 and 192-203 in the amino acid sequence represented by SEQ ID NO:31.

More specifically, there are preferably employed a partial peptide containing the amino acid sequence of 84-240 in the amino acid sequence represented by SEQ ID NO:1, a partial peptide containing the amino acid sequence of 82-239 in the amino acid sequence represented by SEQ ID NO:2, a partial peptide containing the amino acid sequence of 82-239 in the amino acid sequence represented by SEQ ID NO:3, a partial peptide containing the amino acid sequence of 48-204 in the amino acid sequence represented by SEQ ID NO:31, and the like.

Preferred examples of the partial peptides of the present invention further include (i) a peptide containing substantially the same amino acid sequence as the amino acid sequence of 84-240 in the amino acid sequence represented by SEQ ID NO:1 and having an activity substantially equivalent to that of the peptide containing the amino acid sequence of 84-240 in the amino acid sequence represented by SEQ ID NO:1; (ii) a peptide containing substantially the same amino acid sequence as the amino acid sequence of 82-239 in the amino acid sequence represented by SEQ ID NO:2 and having an activity substantially equivalent to that of the peptide containing the amino acid sequence of 82-239 in the amino acid sequence represented by SEQ ID NO:2; (iii) a peptide containing substantially the same amino acid sequence as the amino acid sequence of 82-239 in the amino acid sequence represented by SEQ ID NO:3 and having an activity substantially equivalent to that of the peptide containing the amino acid sequence of 82-239 in the amino acid sequence represented by SEQ ID NO:2; (iv) a peptide containing substantially the same amino acid sequence as the amino acid sequence of 48-204 in the amino acid sequence represented by SEQ ID NO:31 and having an activity substantially equivalent to that of the peptide containing the amino acid sequence of 48-204 in the amino acid sequence represented by SEQ ID NO:1; and the like.

Examples of substantially the same amino acid sequence as the amino acid sequence of 84-240 in the amino acid sequence represented by SEQ ID NO:1 include an amino acid sequence having at least about 40% homology, preferably at least about 60% homology, more preferably at least about 80% homology, much more preferably at least about 90% homology, and most preferably at least about 95% homology, to the amino acid sequence of 84-240 in the amino acid sequence represented by SEQ ID NO:1, and the like.

Examples of substantially the same amino acid sequence as the amino acid sequence of 82-239 in the amino acid sequence represented by SEQ ID NO:2 include an amino acid sequence having at least about 40% homology, preferably at least about 60% homology, more preferably at least about 80% homology, much more preferably at least about 90% homology, and most preferably at least about 95% homology, to the amino acid sequence of 82-239 in the amino acid sequence represented by SEQ ID NO:2, and the like.

Examples of substantially the same amino acid sequence as the amino acid sequence of 82-239 in the amino acid sequence represented by SEQ ID NO:3 include an amino acid sequence having at least about 40% homology, preferably at least about 60% homology, more preferably at least about 80% homology, much more preferably at least about 90% homology, and most preferably at least about 95% homology, to the amino acid sequence of 82-239 in the amino acid sequence represented by SEQ ID NO:3, and the like.

Examples of substantially the same amino acid sequence as the amino acid sequence of 48 - 204 in the amino acid sequence represented by SEQ ID NO:31 include an amino acid sequence having at least about 40% homology, preferably at least about 60% homology, more preferably at least about 80% homology, much more preferably at least about 90% homology, and most preferably at least about 95% homology, to the amino acid sequence of 48-204 in the amino acid sequence represented by SEQ ID NO:31, and the like.

The term "substantially equivalent activity" means the same significance as defined above.

The activities such as a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), or the like can be determined by publicly known methods, or modified methods (methods described in, e.g., Eisuke Mekada, Yasuko Miyake and Yoshio Okada, "Gan to Kagaku Ryoho (Cancer & Chemotherapy)", 4, 407 (1977), etc.) and further by means of screening which will be later described.

The partial proteins of the present invention also include (i) partial proteins containing an amino acid sequence of 84-240 in the amino acid sequence represented by SEQ ID NO:1, of which at least 1 or 2 (preferably 1 to 80, more preferably 1 to about 20, more preferably 1 to about 9, and most preferably several (e.g., 1 to 5)) amino acids are deleted; an amino acid sequence of 84-240 in the amino acid sequence represented by SEQ ID NO:1, to which at least 1 or 2 (preferably 1 to 80, more preferably 1 to about 20, more preferably 1 to about 9, and most preferably several (e.g., 1 to 5)) amino acids are added; an amino acid sequence of 84-240 in the amino acid sequence represented by SEQ ID NO:1, in which at least 1 or 2 (preferably 1 to 80, more preferably 1 to about 20, more preferably 1 to about 9, and most preferably several (e.g., 1 to 5)) amino acids are substituted by other amino acids; or a combination of the above amino acid sequences; (ii) partial proteins containing an amino acid sequence of 82-239 in the amino acid sequence representedby SEQ ID NO:2, of which at least 1 or 2 (preferably 1 to 80, more preferably 1 to about 20, more preferably 1 to about 9, and most preferably several (e.g., 1 to 5)) amino acids are deleted; an amino acid sequence of 82-239 in the amino acid sequence represented by SEQ ID NO:2, to which at least 1 or 2 (preferably 1 to 80, more preferably 1 to about 20, more preferably 1 to about 9, and most preferably several (e.g., 1 to 5)) amino acids are added; an amino acid sequence of 82-239 in the amino acid sequence represented by SEQ ID NO:2, in which at least 1 or 2 (preferably 1 to 80, more preferably 1 to about 20, more preferably 1 to about 9, and most preferably several (e.g., 1 to 5)) amino acids are substituted by other amino acids; or a combination of the above amino acid sequences; (iii) partial proteins containing an amino acid sequence of 82-239 in the amino acid sequence represented by SEQ ID NO:3, of which at least 1 or 2 (preferably 1 to 80, more preferably 1 to about 20, more preferably 1 to about 9, and most preferably several (e.g., 1 to 5)) amino acids are deleted; an amino acid sequence of 82-239 in the amino acid sequence represented by SEQ ID NO:3, to which at least 1 or 2 (preferably 1 to 80, more preferably 1 to about 20, more preferably 1 to about 9, and most preferably several (e.g., 1 to 5)) amino acids are added; an amino acid sequence of 82-239 in the amino acid sequence represented by SEQ ID NO:3, inwhichatleastlor2 (preferably 1 to 80, more preferably 1 to about 20, more preferably 1 to about 9, and most preferably several (e.g., 1 to 5)) amino acids are substituted by other amino acids; or a combination of the above amino acid sequences; and, (iv) partial proteins containing an amino acid sequence of 48-204 in the amino acid sequence represented by SEQ ID NO:31, of which at least 1 or 2 (preferably 1 to 80, more preferably 1 to about 20, more preferably 1 to about 9, and most preferably several (e.g., 1 to 5)) amino acids are deleted; an amino acid sequence of 48-204 in the amino acid sequence represented by SEQ ID NO:31, to which at least 1 or 2 (preferably 1 to 80, more preferably 1 to about 20, more preferably 1 to about 9, and most preferably several (e.g., 1 to 5)) amino acids are added; an amino acid sequence of 48-204 in the amino acid sequence represented by SEQ ID NO:31, in which at least 1 or 2 (preferably 1 to 80, more preferably 1 to about 20, more preferably 1 to about 9, and most preferably several (e.g., 1 to 5)) amino acids are substituted by other amino acids; or a combination of the above amino acid sequences.

In case that the partial peptides are deleted or substituted as described above, a peptide having an amino acid sequence represented by general formula (I), from which 1-83 amino acids are removed is preferably used.

Also, the C-terminus is usually in the form of a carboxyl group (-COOH) or a carboxylate (-COO⁻) but may also be in the form of an amide (-CONH₂) or an ester (-COOR) (wherein R has the same significance as defined above).

Furthermore, as in the proteins of the present invention described above, the partial peptides of the present invention also include variants of the above partial peptides, wherein the amino group at the N-terminal amino acid residue is protected with a protecting group; those wherein the N-terminal region is cleaved in vivo and the glutamyl group thus formed is pyroglutaminated; those wherein a substituent on the side chain of an amino acid in themolecule is protected with a suitable protecting group; or conjugated peptides such as glycopeptides having sugar chains.

Preferred examples of the partial peptides of the present invention are peptides containing the amino acid sequence of 84-240 in the amino acid sequence represented by SEQ ID NO:1 and the amino acid sequence of 82-239 in the amino acid sequence represented by SEQ ID NO:2, and peptides containing the amino acid sequence of 82-239 in the amino acid sequence represented by SEQ ID NO:3 and the amino acid sequence of 48-204 in the amino acid sequence represented by SEQ ID NO:31.

As the salts of the protein or its partial peptide in accordance with the present invention, salts with physiologically acceptable acids (e.g., inorganic acids or organic acids) or bases (e.g., alkali metals) are employed, and physiologically acceptable acid addition salts are particularly preferred. Examples of such salts that are employed include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinicacid, tartaricacid, citric acid, malicacid, oxalic acid, benzoic acid, methanesulfonic acid, benzenesulfonic acid) and the like.

The protein or its salts of the present invention may be produced from the aforesaid cells or tissues of human or other warm-blooded animal by applying publicly known methods for purification of proteins, or may be produced by culturing a transformant bearing a DNA encoding the protein, which will be described hereinafter. Furthermore, the protein or its salts may be produced by the protein synthesis or its modifications, which will be also described later. Specifically, the protein or its salts can be produced by the method described in WO 98/03648 or WO 97/34911.

Where the protein or its salts are produced from the tissues or cells of human or other warm-blooded animal, the tissues or cells of human or other warm-blooded animal are homogenized, then extracted with an acid or the like, and the extract is isolated and purified by a combination of chromatography techniques such as reverse phase chromatography, ion exchange chromatography, and the like.

To synthesize the protein of the present invention, itspartialpeptide, or salts oramides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin,
4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the protein is excised from the resin and at the same time, the protecting groups are removed. Then, intramolecular disulfide bond-forming reaction is performed in a highly diluted solution to obtain the objective protein, partial peptide or amides thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide,
N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be chosen from solvents that are known to be usable for protein condensation reactions. Examples of such solvents are N,N-dimethylformamide, N,N-dimethylacetamide, N-methylpyrrolidone, chloroform, trifluoroethanol, dimethylsulfoxide, DMF, dimethylsulfoxide, pyridine, chloroform, dioxane, methylene chloride, tetrahydrofuran, acetonitrile, ethyl acetate, N-methylpyrrolidone, or appropriate mixtures of these solvents. The reaction temperature is appropriately chosen from the range known to be applicable to protein bond-forming reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect amino groups of the starting materials include Z, Boc, tertiary-amyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by converting the carboxyl group into, e.g., alkylesters (e.g., methyl, ethyl, propyl, butyl, tertiary-butyl, cyclopentyl, cyclohexyl, cycloheptyl, cyclooctyl or 2-adamantyl ester, etc.), benzyl ester, 4-nitrobenzyl ester, 4-methoxybenzyl ester, 4-chlorobenzyl ester, benzhydryl ester, phenacine esters, benzyloxycarbonyl hydrazide, tertiary-butoxycarbonyl hydrazide, trityl hydrazide, or the like.

The hydroxyl group of serine can be protected by, for example, its esterification or etherification. Examples of groups appropriately used for the esterification include a lower alkanoyl group such as acetyl group, etc., an aroyl group such as benzoyl group, etc., and a group derived from carbon such as benzyloxycarbonyl group and ethoxycarbonyl group. Examples of the group appropriately used for the etherification include benzyl group, tetrahydropyranyl group, t-butyl group, and the like.

Examples of groups for protecting the phenolic hydroxyl group of tyrosine include Bzl. Cl₂-Bzl, 2-nitrobenzyl, Br-Z, tertiary-butyl, etc.

Examples of groups used to protect the imidazole moiety of histidine include Tos,
4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters (esters with alcohols (e.g., pentachlorophenol, 2,4,5-trichlorophenol,
2,4-dinitrophenol, cyanomethyl alcohol, p-nitrophenol, HONB, N-hydroxysuccimide, N-hydroxyphthalimide, HOBt)). As the activated form of the amino groups in the starting material, the corresponding phosphoric amides are employed.

To eliminate (split off) the protecting groups, there are employed catalytic reduction in a hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol or the like, as well as by a treatment with an alkali such as a diluted sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group has been eliminated from the peptide chain and a protein in which only the protecting group of the C-terminal carboxyl group has been eliminated are manufactured. The two proteins are condensed in a mixture of the solvents described above. The details of the condensation reaction are the same as described hereinabove. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the desired esterified protein.

The partial peptide or salts of the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part of the partial peptide of the present invention. Where the product contains protecting groups, these protecting groups are removed to give the desired peptide. Publicly known methods for condensation and elimination of the protecting groups are described in 1) - 5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu (A* sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the protein of the present invention. When the protein obtained by the above methods is in a free form, the protein can be converted into an appropriate salt by a publicly known method; when the protein is obtained in a salt form, it can be converted into a free form or a different salt form by a publicly known method.

The DNA encoding the protein of the present invention maybe any DNA so long as it contains the base sequence encoding the protein of the present invention described above. Such a DNA may also be any one of genomic DNA, genomic DNA library, cDNA derived from the cells or tissues described above, cDNA library derived from the cells or tissues described above and synthetic DNA. The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

DNAs containing the base sequences encoding the protein of the present invention, which are described in WO 98/03648 or WO 97/34911, are also given as examples of the DNAs of the present invention.

Specifically, as the DNA encoding the protein of the present invention having the amino acid sequence represented by SEQ ID NO:1, there are employed, e.g., (i) a DNA having the base sequence represented by SEQ ID NO:4, (ii) a DNA hybridizable to a DNA having the base sequence represented by SEQ ID NO:4 under high stringent conditions and encoding a protein having an activity substantially equivalent to the activity (e.g., a liver function controlling activity (e.g., a liver cell maintenance activity, a liver cell death inhibiting activity, a liver function promoting activity, etc.), specifically, a liver regeneration activity (preferably, aliverparenchymal cell growth activity), etc., more specifically, an activity of promoting transfer from the G0 phase to the G1 phase in the cell cycle (preferably, the cell cycle of liver cells), etc.) of a protein having the amino acid sequence represented by SEQ ID NO:1; and the like.

Examples of the DNA that is hybridizable to the DNA having the base sequence represented by SEQ ID NO:4 under high stringent conditions include a DNA having at least about 40% homology, preferably at least about 60% homology, more preferably at least about 80% homology, much more preferably at least about 90% homology and most preferably at least about 95% homology, to the base sequence represented by SEQ ID NO:4.

As the DNA encoding the protein of the present invention having the amino acid sequence represented by SEQ ID NO:2, there are employed, for example, (i) a DNA having the base sequence represented by SEQ ID NO:7, (ii) a DNA hybridizable to a DNA having the base sequence represented by SEQ ID NO:7 under high stringent conditions and encoding a protein having an activity substantially equivalent to that of a protein having the amino acid sequence represented by SEQ ID NO:2, or the like.

Examples of the DNA that is hybridizable to the DNA having the base sequence represented by SEQ ID NO:7 under high stringent conditions include a DNA having at least about 40% homology, preferably at least about 60% homology, more preferably at least about 80% homology, much more preferably at least about 90% homology and most preferably at least about 95% homology, to the base sequence represented by SEQ ID NO:7.

As the DNA encoding the protein of the present invention having the amino acid sequence represented by SEQ ID NO:3, there are employed, for example, (i) a DNA having the base sequence represented by SEQ ID NO:10, (ii) aDNAhybridizable to a DNA having the base sequence represented by SEQ ID NO: 10 under high stringent conditions and encoding a protein having an activity substantially equivalent to that of a protein having the amino acid sequence represented by SEQ ID NO:3, or the like.

Examples of the DNA that is hybridizable to the DNA having the base sequence represented by SEQ ID NO:10 under high stringent conditions include a DNA having at least about 40% homology, preferably at least about 60% homology, more preferably at least about 80% homology, much more preferably at least about 90% homology and most preferably at least about 95% homology, to the base sequence represented by SEQ ID NO:10.

As the DNA encoding the protein of the present invention having the amino acid sequence represented by SEQ ID NO:31, there are employed, for example, (i) a DNA having the base sequence represented by SEQ ID NO: 30. (ii) aDNAhybridizable to a DNA having the base sequence represented by SEQ ID NO: 30 under high stringent conditions and encoding a protein having an activity substantially equivalent to that of a protein having the amino acid sequence represented by SEQ ID NO:2, or the like.

Examples of the DNA that is hybridizable to the DNA having the base sequence represented by SEQ ID NO:30 under high stringent conditions include a DNA having at least about 40% homology, preferably at least about 60% homology, more preferably at least about 80% homology, much more preferably at least about 90% homology and most preferably at least about 95% homology, to the base sequence represented by SEQ ID NO:30.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attachedmanufacturer's protocol. More preferably, the hybridization can be carried out under high stringent conditions.

The high stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration at about 19 mM at a temperature of about 65°C are most preferred.

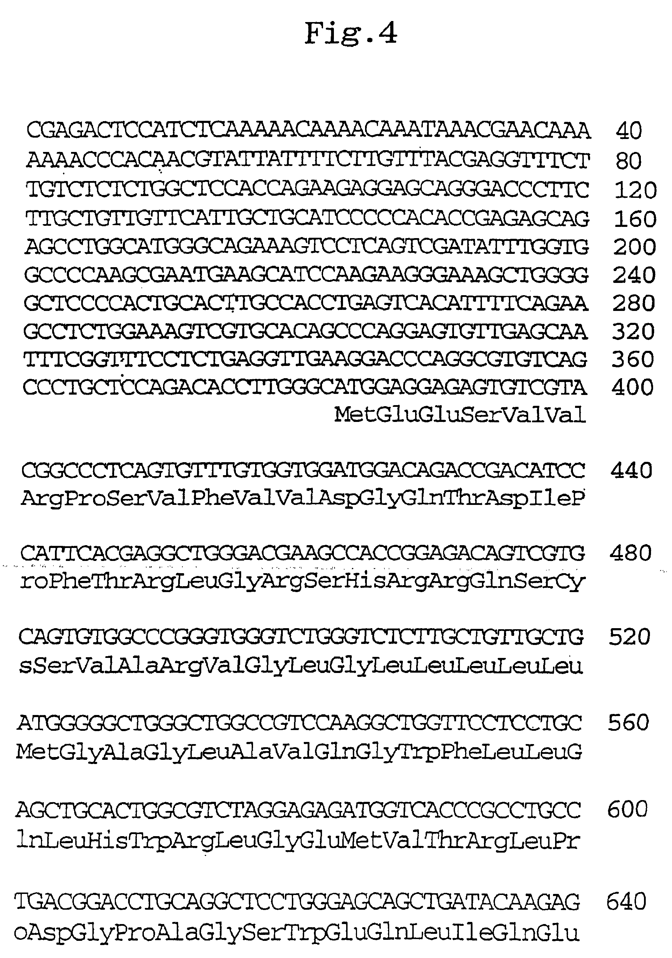

More specifically, for the DNA encoding the protein having the amino acid sequence represented by SEQ ID NO:1, there may be employed a DNA having the base sequence represented by SEQ ID NO:4. For the DNA containing a DNA encoding the protein of the present invention having the amino acid sequence represented by SEQ ID NO:1, e.g., a DNA having the base sequence represented by SEQ ID NO:5 [FIGS. 1 through 3] or SEQ ID NO:6 [FIGS. 4 through 6] may be employed.

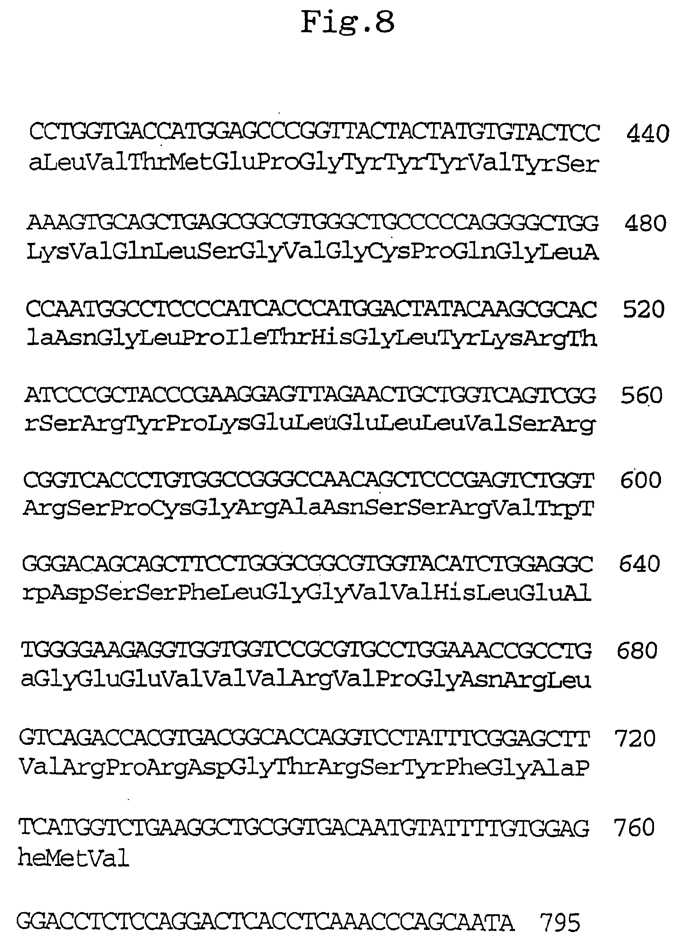

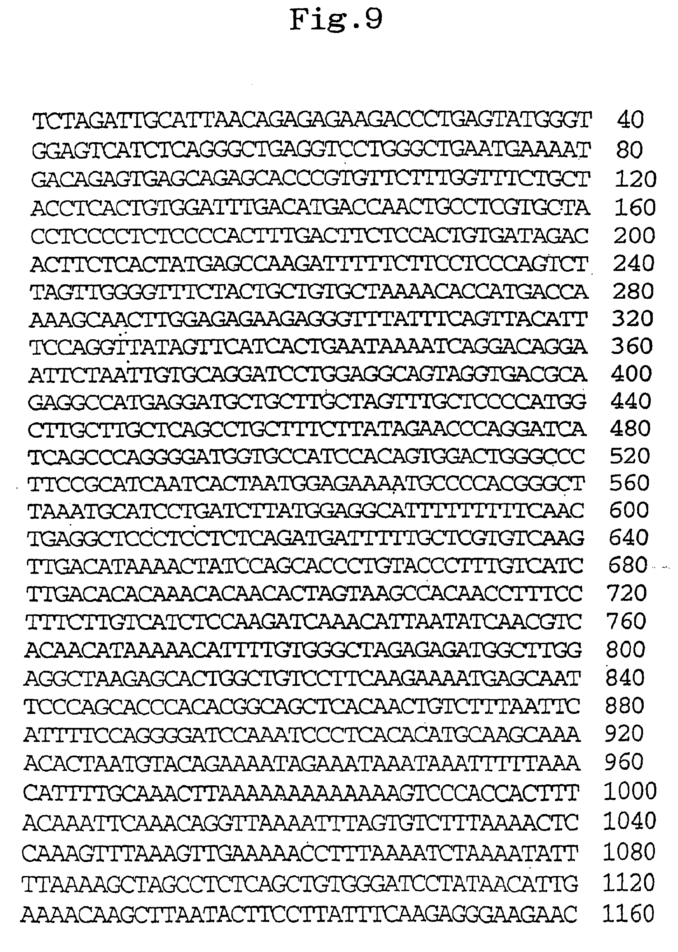

For the DNA encoding the protein containing the amino acid sequence represented by SEQ ID NO:2, there may be employed a DNA having the base sequence represented by SEQ ID NO:7. For the DNA containing a DNA encoding the protein of the present invention having the amino acid sequence represented by SEQ ID NO:2, for example, a DNA having the base sequence represented by SEQ ID NO:8 [FIGS. 7 and 8] or SEQ ID NO: 9 [FIGS. 9 through 18] or the like may be employed.

For the DNA encoding the protein containing the amino acid sequence represented by SEQ ID NO:3, there may be employed a DNA having the base sequence represented by SEQ ID NO:10, or the like.

For the DNA encoding the protein containing the amino acid sequence represented by SEQ ID NO:31, there may be employed a DNA having the base sequence represented by SEQ ID NO:30, or the like.

The DNA encoding the partial peptide of the present invention may be any DNA so long as it contains the base sequence encoding the partial peptide of the present invention described above. The DNAmay also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid, phagemid and the like. Furthermore, the DNA can also be amplified directly by RT-PCR using the mRNA fraction prepared from the cells or tissues described above.

Specifically, as the DNA encoding the partial peptide of the present invention that contains at least one amino acid sequence selected from the amino acid sequences of 8-21, 55-59 (or 54-59), 93-102, 109-116, 118-126, 128-134, 144-149. 162-170, 176-182, 184-189, 193-213, 215-219 and 228-239 (or 228-240) in the amino acid sequence represented by SEQ ID NO:1, there may be used, for example, a DNA having at least one base sequence selected from the base sequences of 22 - 63, 163-177 (or 160-177), 277-306, 325-348, 352-378, 382-402, 430-447, 484-510, 526-546, 550-567, 577-639, 643-657 and 682-717 (or 682-720) in the base sequence represented by SEQ ID NO:4; and the like.

As the DNA encoding the partial peptide containing at least one amino acid sequence selected from the amino acid sequences of 6-20, 53-57 (or 52-57), 91-100, 107-114, 116-124, 126-132, 142-147, 162-170, 176-182, 184-189, 192-212, 214-218 and 227-238 (or 227-239) in the amino acid sequence represented by SEQ ID NO:2, there may be used, for example, a DNA having at least one base sequence selected from the base sequences of 16-60, 157-171 (or 154-171), 271-300, 319-342, 346-372, 376-396, 424-441, 484-510, 526-546, 550-567, 574-636, 640-654 and 678-714 (or 678-717) in the base sequence represented by SEQ ID NO:7; and the like.

As the DNA encoding the partial peptide containing at least one amino acid sequence selected from the amino acid sequences of 6-20, 53-57 (or52-57), 91-100, 107-114, 116-124, 126-132, 142-147, 162-170, 176-182, 184-189, 192-212, 214-218 and 227-238 (or 227-239) in the amino acid sequence represented by SEQ ID NO:3, there may be used, for example, a DNA having at least one base sequence selected from the base sequences of 16-60, 157-171 (or 154-171), 271-300, 319-342, 346-372, 376-396, 424-441, 484-510, 526-546, 550-567, 574-636, 640-654 and 678-714 (or 678-717) in the base sequence represented by SEQ ID NO:10; and the like.

As the DNA encoding the partial peptide containing at least one amino acid sequence selected from the amino acid sequences of 8-21, 57-66, 73-80, 82-90, 92-98, 108-113, 126-134, 140-146, 148-153, 157-177, 179-183 and 192-203 in the amino acid sequence represented by SEQ ID NO:31, there may be used, for example, a DNA having at least one base sequence selected from the base sequences of 22-63, 169-198, 217-240, 244-270, 274-294, 322-339, 376-402, 418-438, 442-459, 469-531, 535-549 and 574-607 in the base sequence represented by SEQ ID NO:30; and the like.

As the DNA encoding the partial peptide having the amino acid sequence of 84-240 in the amino acid sequence represented by SEQ ID NO:1, there are employed, e.g., (i) a DNA having the base sequence of 250-720 in the base sequence represented by SEQ ID NO:4, (ii) a DNA hybridizable to a DNA having the base sequence of 250-720 in the base sequence represented by SEQ ID NO:4 under high stringent conditions and encoding a partial peptide having an activity substantially equivalent to the activity (e.g., a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the GO phase to the G1 phase in the cell cycle (preferably, the cell cycle of liver cells), etc.) of the partial peptide having the amino acid sequence of 84-240 in the amino acid sequence represented by SEQ ID NO:1; and the like.

Examples of the DNA that is hybridizable to the base sequence of 250-720 in the base sequence represented by SEQ ID NO:4 under high stringent conditions include a DNA containing a base sequence having at least about 40% homology, preferably at least about 60% homology, more preferably at least about 80% homology, much more preferably at least about 90% homology and most preferably at least about 95% homology, to the base sequence of 250-720 in the base sequence represented by SEQ ID NO:4; and the like.

As the DNA encoding the partial peptide having the amino acid sequence of 82-239 in the amino acid sequence represented by SEQ ID NO:2, there are employed, for example, (i) a DNA having the base sequence of 244-717 in the base sequence represented by SEQ ID NO:7, (ii) a DNA hybridizable to a DNA having the base sequence of 244-717 in the base sequence represented by SEQ ID NO:7 and encoding a partial peptide having an activity substantially equivalent to the activity (e.g., a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc.) of the partial peptide having the amino acid sequence of 82-239 in the amino acid sequence represented by SEQ ID NO:2, and the like.

Examples of the DNA that is hybridizable to the base sequence of 244-717 in the base sequence represented by SEQ ID NO:7 under high stringent conditions include a DNA containing a base sequence having at least about 40% homology, preferably at least about 60% homology, more preferably at leastabout 80% homology, much more preferably at least about 90% homology andmost preferably at least about 95% homology, to the base sequence of 244-717 in the base sequence represented by SEQ ID NO:7; and the like.

As the DNA encoding the partial peptide having the amino acid sequence of 82-239 in the amino acid sequence represented by SEQ ID NO:3, there are employed, for example, (i) a DNA having the base sequence of 244-717 in the base sequence represented by SEQ ID NO:10, (ii) aDNAhybridizable to a DNA having the base sequence of 244-717 in the base sequence represented by SEQ ID NO: 10 and encoding a partial peptide having an activity substantially equivalent to the activity (e.g., a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc.) of the partial peptide having the amino acid sequence of 82-239 in the amino acid sequence represented by SEQ ID NO:3, and the like.

Also, as the DNA encoding the partial peptide having the amino acid sequence of 48-204 in the amino acid sequence represented by SEQ ID NO:31, there are employed, for example, (i) a DNA having the base sequence of 142-612 in the base sequence represented by SEQ ID NO : 30, (ii) a DNA hybridizable to a DNA having the base sequence of 142-612 in the base sequence represented by SEQ ID NO:30 under high stringent conditions and encoding a partial peptide having an activity substantially equivalent to the activity (e.g., a liver function controlling activity (e.g., a liver cell maintenance activity, a liver cell death inhibiting-activity, a liver function promoting activity, etc.), specifically, a liver regeneration activity (preferably, a liver parenchymal cell growth activity), etc., more specifically, an activity of promoting transfer from the GO phase to the G1 phase in the cell cycle (preferably, the cell cycle of liver cells), etc.) of the partial peptide having the amino acid sequence of 48-204 in the amino acid sequence represented by SEQ ID NO:31, and the like.

Examples of the DNA that is hybridizable to the base sequence of 244-717 in the base sequence represented by SEQ ID NO:10 under high stringent conditions include a DNA containing a base sequence having at least about 40% homology, preferably at least about 60% homology, more preferably at least about 80% homology, much more preferably at least about 90% homology and most preferably at least about 95% homology, to the base sequence of 244-717 in the base sequence represented by SEQ ID NO:10; and the like.

The method of hybridization and the high stringent conditions are the same as those described above.

More specifically, a DNA having the base sequence of 250-720 in the base sequence represented by SEQ ID NO:4, etc. are employed as the DNA encoding the partial peptide having the amino acid sequence of 84-240 in the amino acid sequence represented by SEQ ID NO:1. A DNA having the base sequence of 244-717 in the base sequence represented by SEQ ID NO:7, etc. are employed as the DNA encoding the partial peptide having the amino acid sequence of 82-239 in the amino acid sequence represented by SEQ ID NO:2. A DNA having the base sequence of 244-717 in the base sequence represented by SEQ ID NO:10, etc. are employed as the DNA encoding the partial peptide having the amino acid sequence of 82-239 in the amino acid sequence represented by SEQ ID NO:3. A DNA having the base sequence of 142-612 in the base sequence represented by SEQ ID NO:30, and the like are employed as the DNA encoding the partial peptide having the amino acid sequence of 48-204 in the amino acid sequence represented by SEQ ID NO:31.

For cloning of the DNA encoding the protein or its partial peptide of the present invention, the DNA may be either amplified by publicly known PCR using synthetic DNA primers containing a part of the base sequence of the DNA encoding the protein of the present invention, or the DNA inserted into an appropriate vector can be selected by hybridization with a labeled DNA fragment or synthetic DNA that encodes apart or entire region of the protein of the present invention. The hybridization can be carried out. for example, according to the method described in Molecular Cloning, 2nd (J. Sambrook et al., Cold Spring Harbor Lab. Press, 1989). In the case of using commercially available library, hybridization may be performed in accordance with the protocol described in the attached instructions.

Conversion (deletion, addition or substitution) in the base sequence of the DNA can be made by publicly known methods such as the Gapped duplex method or the Kunkel method or its modification by using a publicly known kit available as Mutan™-G (manufactured by Takara Shuzo Co., Ltd., trademark) or Mutan™-K (manufactured by Takara Shuzo Co., Ltd., trademark).

The cloned DNA encoding the protein or its partial peptide of the present invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the protein or its partial peptide of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV (cytomegalovirus) promoter, HSV-TK promoter, etc. Among them, CMV promoter or SRα promoter is preferably used. Where the host is bacteria of the genus Escherichia, preferred examples of the promoter include trp promoter, lac promoter, recA promoter, λP_{L} promoter, lpp promoter, etc. In the case of using bacteria of the genus Bacillus as the host, preferred example are SPO1 promoter, SPO2 promoter and penP promoter. When yeast is used as the host, preferred examples are AOX1 promoter, PHO5 promoter, PGK promoter, GAP promoter and ADH promoter. When insect cells are used as the host, preferred examples include polyhedrin prompter and P10 promoter.

In addition to the foregoing examples, the expression vectormayfurtheroptionallycontainanenhancer, asplicing signal, a poly A addition signal, a selection marker, SV40 replication origin (hereinafter sometimes abbreviated as SV40ori) etc. Examples of the selection marker employed include dihydrofolate reductase (hereinafter sometimes abbreviated as dhfr) gene, ampicillin resistant gene (hereinafter sometimes abbreviated as Amp^{r}), neomycin resistant gene (hereinafter sometimes abbreviated as Neo, G418 resistance), etc. The dhfr gene and Neo impart methotrexate (MTX) resistance and G418 resistance, respectively. In particular, when the dhfr gene is used as the selection marker together with dhfr gene-deficient Chinese hamster cell CHO, the objective gene can be selected also in a thymidine free medium.

If necessary and desired, a signal sequence that matches with a host is added to the N-terminus of the protein. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

By introducing the vector containing the DNA encoding the protein of the present invention thus constructed, transformants can be produced.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of MolecularBiology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cerevisiae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711) and Sf21 cell (both cells are described in Vaughn, J. L. et al., In Vivo, 13, 213-217 (1977).

As the insect, for example, a larva of Bombyx mori can be used (Maeda et al., Nature, 315, 592 (1985)).

Examples of animal cells include monkey cell COS-7, Vero, Chinese hamster cell CHO (hereinafter simply referred to as CHO cell), dhfr gene deficient Chinese hamster cell CHO (hereinafter simply referred to as CHO(dhfr⁻) cell), mouse L cell, mouse AtT-20, mouse myeloma cell, rat GH3, human FL cell, 293 cell, C127 cell, BALB3T3 cell, Sp-2 cell, etc. Among these cells, CHO cell, CHO(dhfr⁻) cell, 293 cell, etc. are preferred.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991) or Proc. Natl. Acad. Sci. U.S.A., 75, 1929 (1978).

Insect cells or insects can be transformed, for example, according to the method described in Bio/Technology, 6, 47-55(1988).

Animal cells can be transformed, for example, according to the method described in *Saibo Kogaku* (Cell Engineering) , extra issue 8, *Shin Saibo Kogaku Jikken Protocol* (New Cell Engineering Experimental Protocol), 263-267 (1995), published by Shujunsha, or Virology, vol. 52, 456 (1973).

For introducing the expression vectors into cells, there may be employed, for example, the calcium phosphate method [Graham, F. L. and van der Eb, A. J., Virology, 52, 456-467 (1973)], the electroporation method (Nuemann, E. et al., EMBO J., 1, 841-845 (1982)), etc.

As described above, the transformant transformed with the expression vector containing the DNA encoding the protein of the present invention can be obtained.

Methods for stably expressing the protein of the present invention using animal cells include methods of selecting the cells by clone selection in which the expression vectors described above are introduced into chromosomes. Specifically, transformants can be selected based on the selection markers described above. Further, repeated clone selections on the transformants thus obtained using the selection markers enable to acquire stable animal cell lines capable of highly expressing the protein of the present invention. Furthermore, when the dhfr gene is used as the selection marker, incubation may be carried out with a gradually increased concentration of MTX to select resistant cells, whereby the introduced gene is amplified in the cells to obtain higher expression of animal cell lines.

The transformants described above are cultured under conditions capable of expressing the DNA encoding the protein or its partial peptide of the present invention to produce and accumulate the protein or its partial peptide of the present invention, whereby the protein of the present invention, its partial peptide or salts thereof can be produced.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean lees, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate promoters efficiently.

Where the bacteria belonging to the genus Escherichia are used as the host, the transformant is usually cultured at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is incubated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

In particular, when the CHO (dhfr-) cell and the dhfr gene are used as the selection markers, it is preferred to use DMEM medium containing dialyzed bovine fetal serum substantially free of thymidine.

When the protein of the present invention is extracted from the culture or cells, after incubation the transformant or cell is collected by a publicly known method and suspended in a appropriate buffer. The transformant or cell is then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™, etc.

When the protein is secreted in the culture broth, after completion of the cultivation the supernatant can be separated from the transformant or cell to collect the supernatant by a publicly known method. The thus obtained supernatant or the protein of the present invention contained in the extract can be purified by appropriately combining publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein of the present invention thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein of the present invention produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase and the like.

The activity of the thus produced protein of the present invention or salts thereof can be determined by an enzyme immunoassay using a specific antibody.

Antibodies to the protein of the present invention, its partial peptide, or salts thereof may be any of polyclonal antibodies and monoclonal antibodies, as long as they are capable of recognizing the protein of the present invention, its partial peptide, or salts thereof.

The antibodies to the protein of the present invention (hereinafter sometimes merely referred to as the antibodies of the present invention) may be manufactured by publicly known methods for manufacturing antibodies or antisera, using as antigens the protein of the present invention.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every 2 to 6 weeks and 2 to 10 times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out. for example, by reacting a labeled protein, which will be described later, with the antiserum followed by assaying the binding activity of the labeling agent bound to the antibody. The fusion may be carried out, for example, by the known method by Koehler and Milstein (Nature, 256, 495, 1975). Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. Apreferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins (for example. salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.

### [Preparation of polyclonal antibody)

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every 2 to 6 weeks and 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described above.

The antisense DNA having a complementary or substantial complementary base sequence to the DNA encoding the protein of the present invention can be any antisense DNA so long as it is an oligonucleotide or derivatives thereof, having a base sequence substantially complementary to the entire or part of the base sequence of DNA or mRNA encoding the protein of the present invention or its partial peptide and capable of suppressing expression of the protein or the partial peptide.

The base sequence substantially complementary to the DNA or mRNA may, for example, be a base sequence having at least about 40% homology, preferably at least about 60% homology, more preferably at least about 80% homology, and most preferably at least about 90% homology, to the full - length base sequence of the base sequence complementary to the DNA or mRNA (i.e., complementary strand to the DNA or mRNA) or its partial base sequence. In the entire base sequence of the complementary strand to the DNA or mRNA of the present invention, an antisense DNA having at least about 40% homology, preferably at least about 60% homology, more preferably at least about 80% homology, and most preferably at least about 90% homology, to the complementary strand of the base sequence which encodes the N-terminal region of the protein of the present invention (e.g., the base sequence around the initiation codon) . These antisense DNAs can be synthesized using a publicly known DNA synthesizer, etc.

The protein of the present invention, its partial peptide, or salts thereof have, e.g., a liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc. Therefore, the protein of the present invention, its partial peptide, or salts thereof are applicable to a variety of utilities based on the activities described above.

Hereinafter these utilities are described on the protein of the present invention, its partial peptide, or salts thereof (hereinafter sometimes merely referred to as the protein of the present invention), the DNAs encoding the protein, etc. of the present invention (hereinafter sometimes merely referred to as the DNA of the present invention), the antibodies to the protein, etc. of the present invention (hereinafter sometimes merely referred to as the antibodies of the present invention), and the antisense DNA.

### (1) Therapeutic/prophylactic medicaments for various diseases based on the liver function controlling activity

In case that there are patients who do not exhibit sufficiently or normally the liver function controlling activity (e.g., a liver cell maintenance activity, a liver cell death inhibiting activity, a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc.) due to decrease or deficiency in the protein of the present invention in vivo, the role of the protein in accordance with the present invention can be exhibited sufficiently or normally in the patients, by (a) administering the DNA of the present invention to the patients thereby to express the protein of the present invention in vivo, (b) inserting the DNA of the present invention into cells to express the protein of the present invention in the cells, and then transplanting the cells to the patients, or (c) administering the protein of the present invention to the patients.

As will be demonstrated later in EXAMPLES 4 and 5, the protein of the present invention has such an excellent property that is free of any action considered to be side effects such as liver apoptosis inductivity, liver cytotoxicity, etc. markedly observed when other TNF-family ligand molecules are used as medicaments.

Therefore, the protein of the present invention and the DNA of the present invention are useful as agents for the treatment/prevention of diseases, e.g., impaired liver function, liver cancer, hepatitis (viral hepatitis, fulminant hepatitis, etc.), liver cirrhosis, etc. The protein and DNA of the present invention are also useful as medicaments for liver regeneration in the patient with liver cancer after partial hepatectomy (removal).

Where the DNA of the present invention is used as prophylactic/therapeutic agents or as agents for liver regeneration described above, the DNA per se is administered directly to human or other warm-blooded animal; alternatively, the DNA is inserted into an appropriate vector such as retrovirus vector, adenovirus vector, adenovirus-associated virus vector, etc. and then administered to human or other warm-blooded animal in a conventional manner. The DNA of the present invention may also be administered as intact DNA, in its intact form, or may be prepared into a pharmaceutical composition together physiologically acceptable carrier such as an aid to assist its uptake, which composition may be then administered by gene gun or through a catheter such as a catheter with a hydrogel.

Where the protein of the present invention is used as the therapeutic/prophylactic agents described above, the protein is advantageously used on a purified level of at least 90%, preferably at least 95%, more preferably at least 98% and most preferably at least 99%.

Where the protein of the present invention is used as the medicaments described above, the protein can be used orally, for example, in the form of tablets which may be sugar coated if necessary and desired, capsules, elixirs, microcapsules etc., orparenterally in the form of injectable preparations such as a sterile solution and a suspension in water or with other pharmaceutically acceptable liquid. These preparations can be manufactured by mixing the protein of the present invention with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given. When the DNA of the present invention is applied, the DNA can be administered per se directly, or the DNA is inserted into an appropriate vector such as retrovirus vector, adenovirus vector, adenovirus-associated virus vector, etc. and then administered, to human or other warm-blooded animal in a conventional manner.

Additivesmisciblewith tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, *e.g.*, by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), anonionic surfactant (e.g., polysorbate 80™ and HCO-50), etc. Examples of the oily medium include sesame oil and soybean oil, which may also be used in combination with a dissolution aid such as benzyl benzoate, benzyl alcohol, etc. The prophylactic/therapeutic agent may further be formulated withabuffer (e.g., phosphatebuffer, sodiumacetatebuffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector in which the DNA of the present invention is inserted may also be prepared into pharmaceutical preparations in a manner similar to the procedures above. Such preparations are generally used parenterally.

Since the thus obtained pharmaceutical preparation is safe and low toxic, the preparation can be administered to human or other mammal (e.g., rat, mouse, guinea pig, rabbit, sheep, swine, bovine, horse, cat, dog, monkey, etc.).

The dose of the protein of the present invention varies depending on target disease, subject to be administered, route for administration, etc.; when the protein of the present invention is orally administered for the treatment of, e.g., liver cirrhosis, the dose is normally about 0.1 mg to about 100 mg, preferably about 1.0 to about 50 mg, and more preferably about 1.0 to about 20 mg per day for adult (as 60 kg body weight) . In parenteral administration, the single dose may also vary depending on subject to be administered, target disease, etc. but when the protein of the present invention is administered to adult (as 60 kg body weight) as an agent for liver regeneration in the form of injection, it is advantageous to inject the protein into the affected site in a daily dose of about 0.01 to about 30mg, preferably about 0.1 to about 20 mg, and more preferably about 0.1 to about 10 mg. For other animal species, the corresponding dose as converted per 60 kg body weight can be administered.

### (2) Gene diagnostic agent

By using the DNA of the present invention as a probe, an abnormality (gene abnormality) of the DNA encoding the protein of the present invention in human or warm-blooded animal (e.g., rat, mouse, guinea pig, rabbit, sheep, swine, bovine, *horse, cat,* dog, monkey, etc.)can be detected. Therefore, the DNA of the present invention is useful *as* a gene diagnostic agent for various diseases associated with the protein of the present invention.

For example, when the damage to the DNA or mRNA encoding the protein of the present invention, its deficiency, or its decreased expression is detected, it can be diagnosed that diseases are, e.g., impaired liver function, hepatitis (viral hepatitis, fulminant hepatitis, etc.), liver cancer, liver cirrhosis, etc.

The gene diagnosis described above using the DNA of the present invention can be performed by, for example, the publicly known Northern hybridization assay or the PCR-SSCP assay (Genomics, 5, 874-879 (1989); proceedings of the National Academy of Sciences of the United States of America, 86, 2766-2770 (1989)), etc.

In case that decreased expression of the mRNA is detected by, e.g., Northern hybridization, it can be diagnosed that impaired liver function, hepatitis (viral hepatitis, fulminant hepatitis, etc.), liver cancer, liver cirrhosis, etc. are involved or it is highly likely to suffer from these disease in the future.

### (3) Diagnosis of various diseases based on the liver function controlling function of the protein of the present invention, through quantification for the protein of the present invention, its partial peptide, or salts thereof:

The antibody of the present invention is capable of specifically recognizing the protein of the present invention and thus, can be used for a quantification of the protein of the present invention in a test sample fluid, in particular, for a quantification by sandwich immunoassay. Thus, the present invention can be used for diagnosis of various diseases based on the liver function controlling function of the protein in accordance with the present invention.

Quantification of the present invention involves the following methods: (i) a method for quantification of the protein of the present invention in a test sample fluid, which comprises competitively reacting the antibody of the present invention, a test sample fluid and the labeled protein of the present invention, and measuring the ratio of the labeled protein of the present invention bound to the antibody; and, (ii) a method for quantification of the protein of the present invention in a test sample fluid, which comprises reacting the test sample fluid simultaneously or continuously with the antibody of the present invention immobilized on a carrier and a labeled antibody of the present invention, and then measuring the activity of the labeling agent on the insoluble carrier.

In the method (ii) for the quantification described above, it is preferred that one antibody is capable of recognizing the N-terminal region of the protein of the present invention, while another antibody is capable of recognizing the C-terminal region of the protein of the present invention.

The monoclonal antibody to the protein of the present invention (hereinafter sometimes simply referred to as the monoclonal antibody) may be used to assay the protein of the present invention. Moreover, the protein of the present invention can be detected by means of a tissue staining as well. For these purposes, the antibody molecule per se may be used or F(ab')₂, Fab' or Fab fractions of the antibody molecule may also be used.

There is no particular limitation for the assay method using the antibody to the protein of the present invention; any method may be used so far as it relates to a method in which the amount of an antibody, antigen or antibody-antigen complex can be detected by a chemical or a physical means, depending on or corresponding to the amount of an antigen (e.g., the amount of a protein) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method, which will be described later, is particularly preferred.

Examples of the labeling agent used in the assay method using the labeling substance are radioisotopes, enzymes, fluorescent substances and luminescent substances, etc. Examples of the radioisotope are [¹²⁵I], [¹³¹I], [³H], [¹⁴C], etc. Preferred examples of the enzyme are those that are stable and have a high specific activity, which include β-galactosidase, β-glucosidase, alkaline phosphatase, peroxidase, malate dehydrogenase, etc. As the labeling agents, fluorescent substances such as fluorescamine, fluorescein isothiocyanate, etc.; and luminescent substances such as luminol, aluminol derivative, luciferin, lucigenin, etc.; are employed, respectively. Furthermore, the biotin-avidin system may also be used for binding of an antibody or antigen to a labeling agent.

In immobilization of antigens or antibodies, physical adsorption may be used. Alternatively, chemical binding that is conventionally used for immobilization of proteins or enzymes may be used as well. Examples of the carrier include insoluble polysaccharides such as agarose, dextran and cellulose; synthetic resins such as polystyrene, polyacrylamide and silicone; glass; etc.

In the sandwich method, a test sample fluid is reacted with an immobilized monoclonal antibody of the present invention (first reaction), then reacted with another labeled monoclonal antibody of the present invention (second reaction) and the activity of the labeling agent on the insoluble carrier is assayed, whereby the amount of the protein of the present invention in the test sample fluid can be quantified. The first and second reactions may be carried out in a reversed order, simultaneously or sequentially with an interval. The type of the labeling agent and the method for immobilization may be the same as those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.

In the method for assaying the protein of the present invention by the sandwich method according to the present invention, preferred monoclonal antibodies of the present invention used for the first and the second reactions are antibodies, which binding sites to the protein of the present invention are different from one another. Thus, the antibodies used in the first and the second reactions are those wherein, when the antibody used in the second reaction recognizes the C-terminal region of the protein of the present invention, the antibody recognizing the site other than the C-terminal regions, e.g., recognizing the N-terminal region, is preferably used in the first reaction.

The monoclonal antibody of the present invention may be used in an assay system other than the sandwich method, such as a competitive method, an immunometric method, a nephrometry, or the like.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody is used, and a solid phase method in which an immobilized antibody is used as the first antibody or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a *gel* or in a *solution*, is measured. Even when the amount of an antigen in a test sample fluid is small and only a small amount of the sediment is obtained, a laser nephrometry utilizing laser scattering can be suitably used.

In applying each of those immunoassays to the assay method for the present invention, any special conditions or operations are not required to set forth. The assay system for the protein of the present invention may be constructed in addition to conditions or operations conventionally used for each of the methods, taking the technical consideration of one skilled in the art into account consideration. For the details of such conventional technical means, a variety of reviews, reference books, etc. may be referred to, for example, Hiroshi Irie (ed.): "Radioimmunoassay" (published by Kodansha, 1974); Hiroshi Irie (ed.) : "Radioimmunoassay; Second Series" (published by Kodansha, 1979) ; Eiji Ishikawa, etal. (ed.) : "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.) : "Enzyme Immunoassay" (ThirdEdition) (published by Igaku Shoin, 1987); "Methods in Enzymology" Vol. 70 (Immuochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), and ibid., Vol. 121 (Immunochemical Techniques (Part I: Hybridoma Technology and Monoclonal Antibodies)) (all published by Academic Press); etc.

As described above, the protein of the present invention can be quantified with high sensitivity, using the antibody of the present invention.

By applying the quantification methods described above, various diseases associated with the protein of the present invention can be diagnosed.

In case that decreased level of the protein of the present invention is detected by, e.g., Northern hybridization, it can be diagnosed that impaired liver function, hepatitis (viral hepatitis, fulminant hepatitis, etc.), liver cancer, liver cirrhosis, etc. are involved or it is highly likely to suffer from these disease in the future.

### (4) Screening of drug candidate compounds

### (A) Method of screening compounds having a liver function controlling activity:

Since the protein of the present invention possesses a liver function controlling activity (e.g., a liver cell maintenance activity, a liver cell death inhibiting activity. a liver function promoting activity, etc.), specifically, a liver regeneration activity (preferably, a liver parenchymal cell growth activity), etc., more specifically, an activity of promoting transfer from the G0 phase to the G1 phase in the cell cycle (preferably, the cell cycle of liver cells), etc., the method of screening a compound or its salt that accelerates or inhibits the liver cell maintenance activity of the protein of the present invention is provided by constructing an assay system using the protein of the present invention and liver cells (preferably liver parenchymal cells, etc.).

That is, the present invention provides:

a method of screening a compound or its salt that accelerates or inhibits a liver function controlling activity of the protein of the present invention, which comprises comparing (i) the case in which the protein of the present invention is brought in contact with a liver cell (preferably, a liver parenchymal cell), with (ii) the case in which the protein of the present invention and a test compound are brought in contact with a liver cell (preferably, a liver parenchymal cell.

Specifically, the screening method of the present invention is characterized by measuring, for example, the DNA synthesis capabilities of liver cells (preferably, liver parenchymal cells) or the like and comparing the DNA synthesis capabilities.

In the screening method described above, a test compound that is observed to accelerate the DNA synthesis capability of liver cells (preferably, liver parenchymal cells) in the protein of the present invention canbe selected as an agonist, and a test compound that is observed to inhibit the DNA synthesis capability can be selected as an antagonist.

Any liver cell may be used for the method of screening of the present invention, so long as it is a liver cell (especially a liver parenchymal cell) derived from human or other warm-blooded animals (e.g., monkey, rabbit, dog, guinea pig, mouse, rat, sheep, goat, chicken, etc.). The liver cell (preferably a liver parenchymal cell) can be isolated from normal tissues of human or other warm-blooded animals by the established collagenase infusion method.

As media for the liver cell described above, there may be used, for example, commercially available media such as CSC serum-free medium (Cell System, Inc.), Ham F-12 Medium (GIBCO BRL), LEIBOVITZ L-15 medium (GIBCO BRL), etc.; MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc.; a suitable medium mixture of these media; and the like.

In the screening method of the present invention, the liver cells maybe fixed using glutaraldehyde, formalin etc. The fixation can be performed by a publicly known method.

In case that liver cells are contacted with the protein of the present invention, it is also possible to employ cells capable of expressing the protein of the present invention or cell membrane fractions of the cells capable of expressing the protein of the present invention.

Any cell is usable as the cell capable of expressing the protein of the present invention, so long as it is capable of expressing the protein of the present invention. Specific examples of such cells are those exemplified as the transformants described above.

The cell membrane fraction of the cell capable of expressing the protein of the present invention is a fraction abundant in the cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica, Inc.), disruption by ultrasonication, and disruption by cell spraying through thin nozzles under an increased pressure using a French press or the like. Cell membrane fractionation is effected chiefly by fractionation using a centrifugal force, such as centrifugation for fractionation, density gradient centrifugation, etc. For example, cell disrupted fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the expressed protein or the protein of the present invention, and many membrane components such as cell-derived phospholipids and membrane proteins.

Examples of the test compounds include peptides, proteins, non-peptide compounds, synthetic compounds, fermentation products, cell extracts, vegetable extracts, animal tissue extracts, blood plasma and the like and these compounds maybe novel compounds or publicly known compounds.

According to the screening method of the present invention, the reaction of the protein of the present invention with a liver cell (preferably a liver parenchymal cells) may be carried out normally at about 37°C for several ten hours (e.g., 36-72 hours, preferably 48-72 hours).

In more detail, to perform the screening method above, liver cells (preferably liver parenchymal cells) are first suspended in a medium suitable for screening (specifically; media described above, etc.) to prepare the liver cells (preferably liver parenchymal cells).

The liver cells (preferably liver parenchymal cells) suspended in a medium are plated on a culture plate (e.g., a multi-well plate, etc. commercially available). As such a plate suitable for seeding, a plate with the surface coated with collagen, etc. to accelerate adhesion to the plate is preferably used. The concentration of liver cells (preferably liver parenchymal cells) to be plated is in the range of approximately 1,000 to 5,000 cells/well, preferably approximately 2, 000 to 5, 000 cells/well, and more preferably approximately 3,000 to 5,000 cells/well.

Next, the protein of the present invention purified by the publicly known method described above is charged in a plate in a final concentration of approximately 0.01 to 1,000 ng/ml, preferably approximately 0.1 to 1,000 ng/ml, and more preferably approximately 0.1 to 100 ng/ml, to culture liver cells (preferably liver parenchymal cells). The liver cells (preferably liver parenchymal cells) are used as control.

On the other hand, the protein of the present invention purified by the publicly known method and a test compound are added to the liver cells (preferably liver parenchymal cells) plated on a multi-plate, in the same manner as above, in a final concentration of approximately 0.01 to 1, 000 ng/ml, preferably approximately 0.05 to 500 ng/ml, and more preferably approximately 0.1 to 100 ng/ml, to culture the liver cells (preferably liver parenchymal cells) under culture conditions similar to those described above.

The DNA synthesis activity in each liver cell after culture can be detected by a modification from publicly known methods (e.g., the method described in Burton, K: Biochem. J., 62, 315 (1956), etc.).

Specifically, about 0.5 ml of a DNA fraction obtained by, e.g., the Schmidt-Thanhauser method is taken and about 0.5 ml of 5% perchloric acid is added thereto. When a color formed is too strong, the mixture is further diluted with 5% perchloric acid. After about 1.0 ml of diphenylamine reagent (about 1.5 g of diphenylamine, about 100 ml of glacial acetic acid, about 1.5 ml of conc. sulfuric acid; prepared upon use) is added to the mixture, about 0.05 ml of acetaldehyde (obtained by diluting about 10.3 ml with 100 ml of water) is further added thereto. The mixture is allowed to stand at approximately 27-37°C for about 16 hours and then A₆₀₀ nm is measured.

As the standard, commercially available calf thymus-derived DNA is completely dissolved in water in a concentration of about 1 mg/ml, a part of the solution is taken and made about 0.2N NaOH. Then A₂₆₀ nm is measured. Since commercially available DNA might be contaminated with water, proteins, salts, etc., the original DNA concentration is previously corrected from the measurement data of A₂₆₀ nm above, as 1 µg/ml of DNA is A₂₆₀ = 0.023.

In the screening method described above, when the ability of DNA synthesis in liver cells (preferably liver parenchymal cells) added with a test compound is higher than the ability of DNA synthesis in liver cells (preferably liver parenchymal cells) added with no test compound, the test compound can be selected as an agonist candidate compound. On the other hand, when the ability of DNA synthesis in liver cells (preferably liver parenchymal cells) added with a test compound is lower than the ability of DNA synthesis in liver cells (preferably liver parenchymal cells) added with no test compound, the test compound can be selected as an antagonist candidate compound.

The kit for screening according the present invention comprises liver cells (preferably liver parenchymal cells) and the protein, etc. of the present invention.

Examples of the kit for screening of the present invention include the following.

### [Reagent for screening]

### (1) Medium for liver cells (preferably liver parenchymal cells)

(a) CSS serum-free medium (Cell System, Inc.) or,
(b) Basal medium as an equimolar mixture of Ham F-12 (GIBCO BRL) and LEIBOVITZ L-15 medium (GIBCO BRL), supplemented with 1% BSA, 5 mM glucose (WAKO), 10⁻⁸ M dexamethasone (WAKO) and 10⁻⁸ M bovine insulin (GIBCO BRL), all in a final concentration.

### (2) Liver cell (preferably liver parenchymal cell) specimen

Normal human liver parenchymal cell (Cell System, Inc., #3716)

### (3) Specimen protein of the present invention

The aforesaid protein of the present invention, its partial peptide, or salts thereof.

### [Method for assay]

(1) Normal human liver parenchymal cells (Cell System, Inc., #3716) suspended in serum-free CSC medium (Cell System, Inc.) are plated on a 96-well culture plate (FALCON, Inc.) coated with collagen type I, in 2,500 cells/well/50 ml.
(2) The protein of the present invention purified is added by a 3-fold common ratio in 50 ml/well (n-2) to have a final concentration of 0.1 ng/ml to 10 ng/ml followed by incubation in a CO₂ incubator for 3 days (37°C, 5% CO₂).
(3) Separately from (2) above, the protein of the present invention purified and a test compound are added by a 3 - fold common ratio to the plate (1) in 50 ml/well (n = 2) to have a final concentration of 0.1 ng/ml to 10 ng/ml followed by incubation in a CO₂ incubator for 3 days (37°C, 5% CO₂).
(4) After incubation, bromodeoxyuridine (BrdU) is added to each well in a final 1000-fold dilution followed by incubation overnight. Then, each culture broth is removed and 200 ml/well of Fix Denat solution is added followed by fixing the cells at room temperature for 30 minutes.
(4) Thereafter, the solution is removed and HRP-labeled anti-BrdU antibody solution is added in 100 ml/well followed by reacting them for 90 minutes at room temperature. After washing with PBS, substrate is added in 50 ml/well for color formation for 30 minutes. Absorbance is measured at a wavelength of 340 nm and absorbance is measured for control at 492 nm, using a 96-well plate reader (Multiscan Multisoft : Dai-Nippon Pharmaceutical Co., Ltd.).
(5) Uptake of BrdU in the culture cells of (2) above, i.e., the ability of DNA synthesis, is compared to uptake of BrdU in the culture cells of (3) above, i.e., the ability of DNA synthesis.
   As stated above, the protein of the present invention is useful as a reagent for screening of a compound (agonist) that accelerates, or a compound (antagonist) that inhibits the liver function controlling activity possessed by the protein of the present invention.
   The compound or its salt that is obtainable using the screening method or screening kit of the present invention is a compound that accelerates or inhibits the liver function controlling activity the protein of the present invention possesses. Specifically, these compounds accelerate or inhibit a liver regeneration activity (preferably, a liver parenchymal cell growth activity) or an activity of promoting transfer from the G0 phase to the G1 phase in the cell cycle (preferably, the cell cycle of liver cells).
   These compounds are obtained from the test compounds described (e.g., peptides, proteins, non-peptide compounds; synthetic compounds, fermentation products, cell extracts, vegetable extracts, animal tissue extracts, blood plasma and the like) and these compounds may be novel compounds or publicly known compounds.
   The agonist described above has wholly or partly the physiological activities possessed by the protein of the present invention, and is useful as a safe and low-toxic medicament depending upon the physiological activities. That is, the agonist is useful as a medicament for the prevention/treatment of impaired liver function, hepatitis (viral hepatitis, fulminanthepatitis, etc.), liver cancer, liver cirrhosis or the like. The agonist is also useful as a medicament for liver regeneration in the patient with liver cancer after partial hepatectomy.
   The compounds obtained by the screening method described above may be in the form of salts. As the salts of the compounds, preferred are salts with physiologically acceptable acids (e.g., inorganic acids or organic acids) or bases (e.g., alkali metals), with physiologically acceptable acid addition salts being particularly preferred. Examples of such salts that are employed include salts with inorganic acids (e.g., hydrochloric acid, phosphoric acid, hydrobromic acid, sulfuric acid), salts with organic acids (e.g., acetic acid, formic acid, propionic acid, fumaric acid, malefic acid, succinicacid, tartaricacid, citric acid, malicacid, oxalicacid, benzoic acid, methanesulfonic acid, benzenesulfonic acid) and the like.
   When these compounds are employed as therapeutic/prophylactic agents for the diseases described above, the compounds may be prepared in the form of pharmaceutical preparations as in medicaments containing the protein of the present invention described above, which are provided for use.
   Since the thus obtained pharmaceutical preparations are safe and low toxic, these preparations can be administered to human or other mammal (e.g., rat, rabbit, sheep, swine, bovine, cat, dog, monkey, etc.).
   The dose of the compounds obtained by the screening above or salts thereof varies depending on target disease, subject to be administered, route for administration, etc.; when these agonists are orally administered for the treatment of, e.g., liver cirrhosis, the dose is normally about 0.1 mg to about 100 mg, preferably about 1.0 to about 50 mg, and more preferably about 1.0 to about 20 mg per day for adult (as 60 kg body weight) . In parenteral administration, a single dose may also vary depending on subject to be administered, target disease, etc. but when the agonist is administered to adult (as 60 kg body weight) as an agent for the treatment of cirrhosis in the form of injection, it is advantageous to inject the agonist in a daily dose of about 0.01 to about 30 mg, preferably about 0.1 to about 20 mg, and more preferably about 0.1 to about 10 mg. For other animal species, the corresponding dose as converted per 60 kg body weight can be administered.
(6) Pharmaceutical composition comprising antisense DNA
   An antisense DNA that binds to the DNA of the present invention complementarily to inhibit expression of the DNA can be used as the agent for the treatment/prevention of, e.g., diseases caused by overexpression of the protein, etc. of the present invention.

When the antisense DNA described above is used as the therapeutic/prophylactic agent described above, the antisense DNA may be used similarly to the therapeutic/prophylactic agent comprising the DNA of the present invention for various diseases described above.

Where the antisense DNA is used, the antisense DNA alone is administered directly to human or other warm-blooded animal; alternatively, the antisense DNA is inserted into an appropriate vector such as retrovirus vector, adenovirus vector, adenovirus-associated virus vector, etc. and then administered to human or other warm-blooded animal in a conventional manner. The antisense DNA may also be administered in its intact form, or may be prepared into a pharmaceutical composition together physiologically acceptable carrier such as an aid to assist its uptake, which composition may be then administered by gene gun or through a catheter such as a catheter with a hydrogel.

In addition, the antisense DNA may also be employed as an oligonucleotide probe for diagnosis to examine the presence of the DNA of the present invention in tissues or cells and states of its expression.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A :: adenine
- T :: thymine
- G :: guanine
- C :: cytosine
- I :: inosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS :: sodium dodecyl sulfate
- Gly :: glycine
- Ala :: alanine
- Val :: valine
- Leu :: leucine
- Ile :: isoleucine
- Ser :: serine
- Thr :: threonine
- Cys :: cysteine
- Met :: methionine
- Glu :: glutamic acid
- Asp :: aspartic acid
- Lys :: lysine
- Arg :: arginine
- His :: histidine
- Phe: phenylalanine
- Tyr :: tyrosine
- Trp :: tryptophan
- Pro :: proline
- Asn :: asparagine
- Gln :: glutamine
- pGlu :: pyroglutamic acid

Substituents, protecting groups, and reagents used in this specification are presented as the codes below.
- Me :: methyl group
- Et :: ethyl group
- Bu :: butyl group
- Ph :: phenyl group
- TC :: thiazolidine-4(R)-carboxamide group
- Tos :: p-toluenesulfonyl
- CHO :: formyl
- Bzl :: benzyl
- Cl₂Bzl:: 2,6-dichlorobenzyl
- Bom :: benzyloxymethyl
- Z :: benzyloxycarbonyl
- Cl-Z :: 2-chlorobenzyl oxycarbonyl
- Br-Z :: 2-bromobenzyl oxycarbonyl
- Boc :: t-butoxycarbonyl
- DNP :: dinitrophenol
- Trt :: trityl
- Bum :: t-butoxymethyl
- Fmoc :: N-9-fluorenyl methoxycarbonyl
- HOBt :: 1-hydroxybenztriazole
- HOOBt :: 3,4-dihydro-3-hydroxy-4-oxo-1,2,3-benzotriazine
- HONB :: 1-hydroxy-5-norbornene-2,3-dicarboxyimide
- DCC :: N,N'-dichlorohexylcarbodiimide

The sequence identification numbers in the sequence listing of the specification indicates the following sequence.

### [SEQ ID NO:1]

This shows the amino acid sequence of the protein of the present invention derived from human.

### [SEQ ID NO:2]

This shows the amino acid sequence of the protein of the present invention derived from mouse.

### [SEQ ID NO:3]

This shows the amino acid sequence of the protein of the present invention derived from rat.

### [SEQ ID NO:4]

This shows the base sequence of cDNA encoding the protein of the present invention derived from human having the amino acid sequence represented by SEQ ID NO:1.

### [SEQ ID NO:5]

This shows the base sequence of DNA containing cDNA encoding the protein of the present invention derived from human having the amino acid sequence represented by SEQ ID NO:1, which is inserted in plasmid pTB1939.

### [SEQ ID NO:6]

This shows the base sequence of DNA containing cDNA encoding the protein of the present invention derived from human having the amino acid sequence represented by SEQ ID NO:1, which is inserted in plasmid pTB1940.

### [SEQ ID NO:7]

This shows the base sequence of cDNA encoding the protein of the present invention derived from mouse having the amino acid sequence represented by SEQ ID NO:2.

### [SEQ ID NO:8]

This shows the base sequence of DNA containing cDNA encoding the protein of the present invention derived from mouse having the amino acid sequence represented by SEQ ID NO:2, which is inserted in plasmid pTB1958.

### [SEQ ID NO:9]

This shows the base sequence of genomic DNA encoding the protein of the present invention derived from mouse having the amino acid sequence represented by SEQ ID NO:2.

### [SEQ ID NO:10]

This shows the base sequence of cDNA encoding the protein of the present invention derived from rat having the amino acid sequence represented by SEQ ID NO:3.

### [SEQ ID NO:11]

This shows the base sequence of a synthetic oligonucleotide used for cloning DNA encoding the protein of the present invention derived from human.

### [SEQ ID NO:12]

This shows the base sequence of a primer used for cloning DNA encoding the protein of the present invention derived from human.

### [SEQ ID NO:13]

This shows the base sequence of a primer used for cloning DNA encoding the protein of the present invention derived from human.

### [SEQ ID NO:14]

This shows the base sequence of an oligonucleotide used for cloning DNA encoding the protein of the present invention derived from mouse.

### [SEQ ID NO:15]

This shows the base sequence of an oligonucleotide used for cloning DNA encoding the protein of the present invention derived from mouse.

### [SEQ ID NO:16]

This shows the base sequence of a synthetic oligonucleotide used for analysis of the base sequence around initiation codon of DNA encoding the protein of the present invention derived from mouse.

### [SEQ ID NO:17]

This shows the base sequence of a synthetic oligonucleotide used for analysis of the base sequence around initiation codon of DNA encoding the protein of the present invention derived from mouse.

### [SEQ ID NO:18]

This shows the base sequence of an adapter bound to the both ends of a mouse chromosomal DNA fragment used for analysis of the base sequence around initiation codon of DNA encoding the protein of the present invention derived from mouse.

### [SEQ ID NO:19]

This shows the base sequence of a synthetic oligonucleotide used for analysis of the base sequence around initiation codon of DNA encoding the protein of the present invention derived from mouse.

### [SEQ ID NO:20]

This shows the base sequence of a synthetic oligonucleotide used for analysis of the base sequence around initiation codon of DNA encoding the protein of the present invention derived from mouse.

### [SEQ ID NO:21]

This shows the base sequence of a primer used for cloning DNA encoding the extracellular region of the protein of the present invention derived from human.

### [SEQ ID NO:22]

This shows the base sequence of a primer used for cloning DNA encoding the extracellular region of the protein of the present invention derived from human.

### [SEQ ID NO:23]

This shows the base sequence of a primer used for cloning DNA encoding the protein of the present invention derived from rat.

### [SEQ ID NO:24]

This shows the base sequence of a primer used for cloning DNA encoding the protein of the present invention derived from rat.

### [SEQ ID NO:25]

This shows a general formula (I) of the amino acid sequence for the protein of the present invention.

### [SEQ ID NO:26]

This shows the base sequence of a primer used in EXAMPLE 1, which will be described later.

### [SEQ ID NO:27]

This shows the base sequence of a primer used in EXAMPLE 1, which will be described later.

### [SEQ ID NO:28]

This shows the base sequence of Primer 1 used in EXAMPLE 6, which will be described later.

### [SEQ ID NO:29]

This shows the base sequence of Primer 2 used in EXAMPLE 6, which will be described later.

### [SEQ ID NO:30]

This shows the base sequence of cDNA composed of 612 bases obtained in EXAMPLE 6, which will be described later.

### [SEQ ID NO:31]

This shows the amino acid sequence of hTL4-2 obtained in EXAMPLE 6, which will be described later.

### [SEQ ID NO:32]

This shows the base sequence of a probe used in EXAMPLE 8, which will be described later.

### [SEQ ID NO:33]

This shows the base sequence of a primer used in EXAMPLE 8, which will be described later.

### [SEQ ID NO:34]

This shows the base sequence of a primer used in EXAMPLE 8, which will be described later.

### [SEQ ID NO:35]

This shows the base sequence of a probe used in EXAMPLE 8, which will be described later.

### [SEQ ID NO:36]

This shows the base sequence of a primer used in EXAMPLE 8, which will be described later.

### [SEQ ID NO:37]

This shows the base sequence of a primer used in EXAMPLE 8, which will be described later.

### [SEQ ID NO:38]

This shows the base sequence of a probe used in EXAMPLE 8, which will be described later.

### [SEQ ID NO:39]

This shows the base sequence of a primer used in EXAMPLE 8, which will be described later.

### [SEQ ID NO:40]

This shows the base sequence of a primer used in EXAMPLE 8, which will be described later.

### [SEQ ID NO:41]

This shows the base sequence of a probe used in EXAMPLE 8, which will be described later.

### [SEQ ID NO:42]

This shows the base sequence of a primer used in EXAMPLE 8, which will be described later.

### [SEQ ID NO:43]

This shows the base sequence of a primer used in EXAMPLE 8, which will be described later.

### [SEQ ID NO:44]

This shows the base sequence of a probe used in EXAMPLE 8, which will be described later.

### [SEQ ID NO:45]

This shows the base sequence of a primer used in EXAMPLE 8, which will be described later.

### [SEQ ID NO:46]

This shows the base sequence of a primer used in EXAMPLE 8, which will be described later.

### [SEQ ID NO:47]

This shows a general formula (II) of the amino acid sequence for the protein of the present invention.

### [SEQ ID NO:48]

This shows the base sequence of a primer used in EXAMPLE 9, which will be described later.

### [SEQ ID NO:49]

This shows the base sequence of a primer used in EXAMPLE 9, which will be described later.

### [SEQ ID NO:50]

This shows the base sequence of a primer used in EXAMPLE 10, which will be described later.

### [SEQ ID NO:51]

This shows the base sequence of a primer used in EXAMPLE 10, which will be described later.

### [SEQ ID NO:52]

This shows the base sequence of a primer used in EXAMPLE 10, which will be described later.

### [SEQ ID NO:53]

This shows the base sequence of a primer used in EXAMPLE 10, which will be described later.

### [SEQ ID NO:54]

This shows the base sequence of a primer used in EXAMPLE 9, which will be described later.

*Escherichia coli* DH10B/pTB1939 and *Escherichia* coli DH10B/pTB1940, which are transformants obtained in REFERENCE EXAMPLE 1 later described, have been deposited with the Ministry of International Trade and Industry, Agency of Industrial Science and Technology, National Institute of Bioscience and Human Technology (NIBH) located at 1-1-3, Higashi, Tsukuba-shi, Ibaraki, Japan, as the Accession Numbers FERM BP-5595 and FERM BP-5596, respectively, since July 17, 1996 and with Institute for Fermentation, Osaka (IFO) located at 2-17-85, Juso Honcho, Yodogawa-ku, Osaka-shi, Osaka, Japan, as the Accession Numbers IFO 15997 and IFO 15998 since July 11, 1996.

*Escherichia coli* DH5α/pTB1958, which is a transformant obtained in REFERENCE EXAMPLE 2 later described, has been deposited with the Ministry of International Trade and Industry, Agency of Industrial Science and Technology, National Institute of Bioscience and Human Technology (NIBH) as the Accession Number FERM BP-5805 since January 30, 1997 and with Institute for Fermentation, Osaka (IFO) as the Accession Number IFO 16054 since January 31, 1997.

*Escherichia coli* DH5α/pTB2011, which is a transformant obtained in REFERENCE EXAMPLE 3 later described, has been deposited with the Ministry of International Trade and Industry, Agency of Industrial Science and Technology, National Institute of Bioscience and Human Technology (NIBH) as the Accession Number FERM BP-6012 since July 8, 1997 and with Institute for Fermentation, Osaka (IFO) as the Accession Number IFO 16109 since July 7, 1997.

*Escherichia coli* DH5α/pTB2012, which is a transformant obtained in REFERENCE EXAMPLE 5 later described, has been deposited with the Ministry of International Trade and Industry, Agency of Industrial Science and Technology, National Institute of Bioscience and Human Technology (NIBH) as the Accession Number FERM BP-6013 since July 8, 1997 and withlnstitute for Fermentation, Osaka (IFO) as the Accession Number IFO 16110 since July 7, 1997.

*Escherichia coli* DH5α/hTL4-pCR2.1 encoding hTL4-2, which is a transformant obtained in REFERENCE EXAMPLE 2 later described, has been deposited with the Ministry of International Trade and Industry, Agency of Industrial Science and Technology, National Institute of Bioscience and Human Technology (NIBH) as the Accession Number FERM BP-6958 since December 6, 1999 and with Institute for Fermentation, Osaka (IFO) as the Accession Number IFO 16329 since October 27, 1999.

### EXAMPLES

The present invention is described in detail below with reference to EXAMPLES, but not intended to limit the scope of the present invention there to. The gene manipulation procedures using *Escherichia coli* were performed according to the methods described in the Molecular Cloning.

### REFERENCE EXAMPLE 1

### Cloning of cDNA encoding human-derived TL4 protein

Cloning of cDNA was carried out using the GENETRAPPER™ cDNA positive selection system (GIBCO BRL). *Escherichia coli* DH12S strain from the SUPERSCRIPT™ human liver cDNA library (GIBCO BRL) was incubated at 30°C for 16 hours in Terrific Broth (12 g/l Bacto-tryptone (Difco Co.), 24 g/l Bacto-yeast extract (Difco Co.), 2.3 g/l potassium monophosphate, 12.5 g/l potassium diphosphate, 0.4% glycerol) supplemented with 100 µg/ml of ampicillin. After collecting the cells, plasmid cDNA library was prepared using Qiagen Plasmid Kit (Qiagen, Inc.). The plasmid cDNA library was digested, after purification, with GeneII, ExoIII (both GIBCO BRL) to produce single stranded cDNA library.

On the other hand, a synthetic oligonucleotide (SEQ ID NO:11) was used as a probe for screening of cDNA library. The probe was labeled through biotinylation at the 3' end using TdT, biotin-14-dCTP (GIBCO BRL). After treating at 95°C for a minute, the single stranded cDNA library was quenched on ice and the biotinylated probe was added thereto followed by hybridization at 37°C for an hour at room temperature. After hybridization, GENETRAPPER cDNA positive selection system/strepto-avidin beads (GIBCO BRL) were added, which was allowed to stand for 30 minutes at room temperature while agitating every 2 minutes. Then the beads were put into a GENETRAPPER™ cDNA positive selection system/magnet rack (GIBCO BRL), which was allowed to stand for 2 minutes. The supernatant was discarded and the magnet beads were rinsed with a GENETRAPPER cDNA positive selection system/washbuffer. Rinsing with the wash buffer was repeated 3 times. Then, the beads were put in the magnet rack, allowed to stand and the supernatant was discarded. After adding a GENETRAPPER cDNA positive selection system/elution buffer, the beads were allowed to stand for 5 minutes at room temperature. The beads were put in the magnet rack and allowed to stand for 5 minutes. The supernatant DNA solution was then recovered.

The synthetic oligonucleotide (SEQ ID NO:11) was added as a primer to the DNA solution collected, which was then treated at 95°C for a minute. After a GENETRAPPER cDNA positive selection system/repair enzyme was added to the DNA solution, the mixture was allowed to stand for 15 minutes at 70°C to synthesize double-stranded DNA. The double-stranded DNA thus synthesized was introduced into *Escherichia coli* DH10B strain using an electroporation device (BioRad, Inc.).

Using the transformant obtained, screening by colony PCR was performed using two oligonucleotides (SEQ ID NO:12, SEQ ID NO:13) as primers. Three colonies (#9, #33, #81) with 434 bp amplification fragment were selected as positive clones using PCR.

After culturing the selected *Escherichia coli*, DNA was extracted from the culture and reacted using Taq Dideoxy Terminator Cycle Sequencing Kit (Perkin-Elmer, Inc.) to determine the base sequence of cDNA fragment using ABI PRISM™ 377 DNA Sequencer (Perkin-Elmer, Inc.). Clones #9 and #33 in the 3 clones acquired contained the same DNA fragment and had a base sequence of 1491 bases with poly(A)⁺ chain, represented by SEQ ID NO:5 [FIGS. 1 through 3]. Clone #81 had a base sequence of 1353 bases represented by SEQ ID NO: 6 containing poly(A)⁺ chain and poly (A)⁺-added signal (AATAA) [FIGS. 4 through 6]. These three clones contained the same gene in the cDNA fragment, which gene encoded TL4 protein of 240 amino acids shown by SEQ ID NO:1. Also from the Kyte-Doolittle analysis, it was assumed that the hydrophobic region from 35th valine (Val) to 63rd tryptophan (Trp) would be a transmembrane region of this protein. This protein was most highly homologous to human lymphotoxin β, and 33% homology was noted on an amino acid level. While 31% homology on an amino acid level was noted with human Fas ligand, higher homology to human Fas ligand than human lymphotoxin β was observed by the taxonomic tree analysis by the J. Hein method (based on PAM50 residue weight table).

Plasmid pTB1939 bearing clone #9 and plasmid pTB1940 bearing clone #81, encoding the protein of the present invention, were introduced into *Escherichia coli* DH108 to obtain transformants: *Escherichia coli* DH10B/pTB1939 and *Escherichia coli* DH10B/pTB1940.

### REFERENCE EXAMPLE 2

### Cloning of cDNA encoding mouse-derived TL4 protein

Cloning of cDNA was performed according to the PCR method. *Escherichia coli* DH12S strain from SUPERSCRIPT™ mouse 8.5 day-embryo-derived cDNA library (GIBCO BRL) was incubated at 30°C for 16 hours in Terrific Broth (32 g/l Bacto-tryptone (Difco Co.), 20 g/l Bacto-yeast extract (Difco Co.), 0.2 g/l NaCl) supplemented with 100 µg/ml of ampicillin. Then, plasmid cDNA library was prepared using Qiagen Plasmid Kit (Qiagen, Inc.) and employed as a template.

As primers, the following two synthetic oligonucleotides were employed.

5'-TCTGCTCTGGCATGGAGAGTGTGGT-3' (SEQ ID NO:14)

5'-CTATTGCTGGGTTTGAGGTGAGTC-3' (SEQ ID NO:15)

By applying Thermal Cycler (GeneAmp^{R} PCR System 2400, Perkin-Elmer Co.) to the system TaKaRa Ex Taq (Takara Shuzo Co., Ltd.), PCR was carried out by one cycle set to include 94°C for aminute, 30 cycles set to include 94°C for 20 seconds, then 55°C for 30 seconds and then 72°C for 2 minutes, and then allowing to stand at 4°C.

The thus amplified fragment was inserted into pT7 Blue T-vector (Novagen, Inc.) using DNA ligation kit Version 2 (Takara Shuzo Co., Ltd.), which was transfected to *Escherichia coli* DH5α.

Plasmid DNA was extracted from the transformant obtained , and reacted using Dye Terminator Cycle Sequence FS Ready Reaction Kit (Perkin-Elmer, Inc.). The base sequence of cDNA fragment was determined using DNA sequencer 373A (Perkin-Elmer, Inc.).

The clone acquired had a base sequence of 795 bases shown by SEQ ID NO:8 containing the base sequence of 717 bases shown by SEQ ID NO:7, and encoded mouse-derived TL4 protein of 239 amino acids shown by SEQ ID NO:2 [FIGS. 7 and 8] This mouse-derived TL4 protein and human-derived TL4 protein having the amino acid sequence shown by SEQ ID NO:1 obtained in REFERENCE EXAMPLE 1 had 78% homology on an amino acid level, and the DNA encoding the protein had 77% homology on a base level. The thus obtained plasmid pTB1958 bearing the DNA encoding the mouse-derived TL4 protein was introduced into *Escherichia coli* DH5α to obtain transformant, *Escherichia coli* DH5α/pTB1958.

Next, the sequence around initiation codon of the DNA encoding the protein of the present invention derived from mouse was analyzed using Promoter Finder DNA Walking Kit (Clontech, Inc.).

Mouse genomic DNA used is previously digested with restriction enzyme ScaI, wherebyan adaptor sequence capable of ligating Primer AP1 (Clontech, Inc.) or Primer AP2 (Clontech, Inc.) has been ligated to the 5' and 3' ends. 
(1) Primer AP1: (SEQ ID NO:16)
5'-GTAATACGACTCACTATAGGGC-3'
 
(2) Primer AP2: (SEQ ID NO:17)
5'-ACTATAGGGCACGCGTGGT-3'
 
(3) Adaptor sequence: (SEQ ID NO:18)
5'-GTAATACGACTCACTATAGGGCACGCGTGGTC
GACGGCCCGGGCTGGT-3'
 Using this mouse genomic DNA solution, TaKaRa LA PCR Kit Version 2 (Takara Shuzo Co., Ltd.), AP1 and synthetic oligonucleotide GAP1, a first PCR was carried out by 7 cycles set to include 94°C for 2 seconds and 72°C for 3 minutes, 37 cycles set to include 94°C for 2 seconds and 68°C for 3 minutes and, 68°C for 4 minutes, and then allowing to stand at 4°C.

(4) Synthetic oligonucleotide GSP1: (SEQ ID NO:19)
5'-CAGCCCAGCACCTAGCAGCAGCACCAG-3'

Next, this reaction solution was diluted to 50-fold with sterile water and the dilution was used for a second PCR. Using this PCR reaction solution, TaKaRa LA PCR Kit Version 2 (Takara Shuzo Co., Ltd.), primer AP2 and synthetic oligonucleotide GAP2 described above, the second PCR was carried out by 5 cycles set to include 94°C for 2 seconds and 72°C for 3 minutes, 25 cycles set to include 94°C for 2 seconds and 68°C for 3 minutes and, 68°C for 4 minutes, and then allowing to stand at 4°C.

(5) Synthetic oligonucleotide GSP2: (SEQ ID NO:20)
5'-GCCGCCTGAATGGGATGTCCGTCTGTC-3'

The amplified fragment of about 1.1 kbp, which was obtained from the genomic DNA solution digested with ScaI, was inserted into pT7 Blue T-Vector (Novagen, Inc.) using DNA Ligation Kit Version 2 (Takara Shuzo Co., Ltd.), which was introduced into *Escherichia coli* DH5α to obtain a transformant.

PlasmidDNAwas extracted from the transformant obtained and reacted using Dye Terminator Cycle Sequence FS Ready Reaction Kit (Perkin-Elmer). A part of the base sequence for the amplified fragment was determined using DNA sequencer 373A (Perkin-Elmer). The clone acquired had a sequence fully coincident with the base sequence (1st to 39th base sequence in the base sequence shown by SEQ ID NO:7) encoding 1st Met (initiation codon) to 13th Asp of the protein of the present invention derived from mouse having the amino acid sequence shown by SEQ ID NO:2. It was thus confirmed that the sequence of the synthetic oligonucleotide (SEQ ID NO:14) used for cloning of cDNA encoding the protein of the present invention derived from mouse was a part of the sequence of DNA encoding the actual protein of the present invention derived from mouse.

### REFERENCE EXAMPLE 3

### Cloning of chromosomal gene containing the coding region of mouse-derived TL4 protein gene

A chromosomal DNA fragment encoding the region containing the open reading frame of the mouse-derived TL4 protein gene was isolated by the plaque hybridization method using as a probe labeled mouse-derived TL4 protein cDNA, using lambda FIX^{R} II library, into which 129SVJ mouse chromosomal DNA fragment partially digested with Sau2AI had been incorporated. First, *Escherichia coli* XL1-Blue MRA was incubated in LB medium supplemented with 0.2% maltose and 10 mM MgSO₄ at 30°C overnight. The same volume of the culture was mixed with a phage solution diluted in 1-10 x 10⁴ pfu (plaque-forming unit)/ml, followed by incubation at 37°C for 10 minutes. To 200 µl of the solution mixture was added 5 ml of top agarose (NZY medium [5 g/l NaCl, 2 g/l MgSO₄.7H₂O, 5 g/l yeast extract, 10 g/l NZ amin (pH was adjusted to 7.5)] added with agarose to become 0.7% of agarose), which had been previously warmed to 50°C. The resulting mixture was uniformly laminated on an NZY plate (1.5%agarose, 9 cm dish), which was then allowed to stand for 9 hours at 37°C. Nylon transfer membrane Hybond™-N+ (Amersham), which had been marked to locate the position of the plate, was closely contacted on the plate for a minute so that phage particles came out and transferred onto the membrane. The membrane was put on a sheet of 3MM paper filter (Whatman International Co.) soaked with a denaturation solution (1.5M NaCl, 0.5M NaOH) for 7 minutes, with the phage-attached surface up. Then, the membrane was put on a filter paper soaked with a neutralizing solution (1.5M NaCl, 0.5M Tris hydrochloride (pH 7.2), 1 mM EDTA), with the phage-attached surface up, and allowed to stand for 3 minutes. The neutralizing treatment was repeated again followed by washing with 2 x SSC solution (0.3M NaCl, 0.03M sodium citrate). After air-drying, the membrane was put on a filter paper soaked with a neutralizing 0.4M NaOH, for 20 minute with the phage-attached surface up, followed by washing with 5 x SSC solution (0.75MNaCl, 75 mM sodium citrate), which was then filled up in a hybridization pack. After 5 ml of a hybridization buffer of ECL Gene Detection System (Amersham) was added to the pack, hybridization was performed for an hour at 42°C.

On the other hand, the open reading frame region (720 bp) of the mouse-derived TL4 protein cDNA was amplified by PCR and the amplified DNA fragment was thermally denatured. Then, by adding the labeling reagent of the ECL Gene Detection System and glutaraldehyde in an equal volume, the mixture was incubated at 37°C for 5 minutes for labeling. A 10 µl aliquot was added to a pre-hybridization pack followed by incubation at 42°C for an hour. The membrane was then taken out of the pack and washed for 20 minutes with a primary wash buffer (6M urea, 4 g/l SDS, 25 ml/l 20 x SSC) previously kept at 42°C. After repeating the procedure again, the membrane was washed with a secondary wash buffer (2 x SSC) for 5 minutes at room temperature. After repeating this once again, the membrane was soaked in the detection reagent of the ECL Gene Detection System for a minute. Thereafter, the membrane was put on an X ray film and exposed to light. After an hour, the membrane was taken out and developed, and positive clones were screened. The clones screened at this stage were subjected further to secondary screening similarly to the screening above. Finally, five candidate clones (#2, 3, 4, 5 and 6) could be obtained. The results of PCR reveal that among these five candidate clones, #1 and #6 were the clones embrace the entire region of the gene encoding mouse-derived TL4 protein.

Next, for the purpose of clarifying the base sequence of chromosomal DNA bearing the coding region of the mouse-derived TL4 protein gene, subcloning was performed. First, the clone #6 obtained was digested with restriction enzyme XbaI followed by electrophoresis using 0.7% agarose gel. The DNA fragment of about 9 kb, that was suspected of containing the coding region of the mouse-derived TL4 protein gene, was excised out and recovered/purified using QIAquick Gel Extraction Kit (Qiagen). On the other hand, cloning vector pUC19 was digested with restriction enzyme XbaI followed by electrophoresis using 1.0% agarose gel. The DNA fragment corresponding to about 2.7 kb was excised out and recovered/purified using QIAquick Gel Extraction Kit (Qiagen), followed by terminal dephosphorylation using bovine small intestine-derived alkaline phosphatase CIAP (Takara Shuzo Co., Ltd.). This CIAP-treated pUC19 was ligated to the DNA fragment derived from the clone #6 prepared above, using DNA Ligation Kit Ver. 2 (Takara Shuzo Co., Ltd.), which was introduced into *Escherichia coli* DH5α. From the ampicillin-resistant strains, plasmid DNA inserted with the objective DNA fragment was screened and isolated. With respect to the base sequence of the cloned clone #6-derived XbaI DNA fragment, sequencing using Dye Terminator Cycle Sequence FS Ready Reaction Kit (Perkin-Elmer) was first performed on GeneAmp^{R} PCR System 2400 following the conditions instructed by the attached brochure, using various synthetic oligo DNAs as primers. Subsequently, the specimens were sequenced with DNA Sequencer 373A (Perkin-Elmer). The base sequence obtained was verified by a gene analysis software, Lasergene (DYNASTAR Co.). The results reveal that the chromosomal gene encoding the mouse-derived TL4 protein was constructed with 4 exons [FIGS. 9 through 18].

The plasmid bearing the cone #6-derived XbaI DNA fragment containing the coding region of the mouse-derived TL4 protein was named pTB2011, and the transformant obtained by transfecting the plasmid to *Escherichia coli* DH5α was named *Escherichia coli* DH5α/pTB2011.

### REFERENCE EXAMPLE 4

### Expression of the extracellular region of human-derived TL4 protein using Pichia yeast as host and Western blot analysis

pPICZαA (Invitrogen Co.) was used as a vector to express the extracellular region of the human-derived TL4 protein of the present invention in yeast Pichia pastoris. This vector bears a gene encoding a secretory signal α-factor of budding yeast, Saccharomyces cerevisiae, which is functional in the Pichia yeast, downstream the promoter for alcohol oxidase gene (AOX1) of the Pichia yeast, and a multicloning site after the gene, and thus the recombinant protein can be secreted into a medium.

First, the DNA fragment encoding the extracellular region of the human-derived TL4 protein of the present invention was prepared by PCR. The following 2 primers used in the preparation of the DNA fragment were synthesized by a DNA synthesizer (Oligo1000M, Beckman Instruments, Inc.). 
(1) 5'-Primer (SEQ ID NO:21)
5'-ACGAATTCCAAGAGCGAAGGTCTCACGAGGTC-3'
 (This primer has EcoRI recognition sequence and at the 3' end, 24 bases encoding 8 amino acids from 85th Gln at the N terminus, in the extracellular region of the human-derived TL4 protein.) 
(2) 3'- Primer (SEQ ID NO:21)
5'-AGTCTAGACTCCTTCCTTCACACCATGAAAGCCCC-3'
 (This primer has XbaI recognition sequence and at the 3' end, termination codon (TGA) and a complementary sequence to 15 bases encoding the C-terminal 5 amino acids in the extracellular region of the human-derived TL4 protein.)

A solution of 50 µl containing 50 pmols of each primer obtained, 100 ng of plasmid pTB1939 obtained in REFERENCE EXAMPLE 1, 10 nmols each of dATP, dCTP, dGTP and dTTP, 2.5 units of native Pfu DNA polymerase (Stratagene, Inc.) and 5 µl of native Pfu DNA buffer (Stratagene, Inc.) was prepared. Using Thermal Cycler (GeneAmp^{R} PCR System 2400, Perkin-Elmer Co.), PCR was carried out by 30 cycles in which one cycle is set to include 94°C for a minute, 98°C for 20 seconds, then 55°C for 30 seconds and then 68°C for 2 minutes, and finally by the condition at 72°C for 5 minutes. The PCR product was recovered from the reaction-completed solution. After digesting with EcoR1 and XbaI, the digestion product was ligated to pPICZαA, which had been previously made linear by digestion with EcoRI and XbaI, to obtain a cyclic plasmid. The plasmid DNA was again cleaved at the SacI unique cleavage site at the AOX1 locus to make linear, followed by transfection to Pichia pastoris KM71 strain through electroporation. Several clones were screened from Zeocin™-resistant strains acquired there, that could grow on 100 µg/ml Zeocin™ (Invitrogen)-containing YPD agar medium (1% yeast extract (Difco), 2% glucose (Wako Pure Chemicals), 2% agar powders (Wako Pure Chemicals). After preparing each chromosomal DNA, PCR was carried out to confirm incorporation of the transfected plasmid DNA into chromosome, using the chromosomal DNA preparedas a template. The clone for which the incorporation was confirmed was screened as a transformant for expression of the objective recombinant protein.

The recombinant protein was expressed by the following procedures. First, one platinum loop of a colony of the transformant for expression of human-derived TL4 protein was inoculated on 25 ml of BMGY medium (1% yeast extract, 2% peptone, 100 mM potassium phosphate (pH 6.0), 1.34% yeast nitrogen base with ammonium sulfate without amino acids (Difco), 4x10⁻⁵% biotin, 1% glycerol) followed by incubation at 30°C for 20 hours. The cells were collected by centrifugation. Next, the cell were resuspended in BMMY (1% yeast extract, 2% peptone, 100 mM potassium phosphate (pH 6.0), 1.34% yeastnitrogen base with ammonium sulfate without amino acids (Difco), 4 x 10⁻⁵% biotin, 0.5% methanol) medium, followed by incubation at 30°C. One or two days after, the culture broth was subjected to sampling and centrifuged to obtain the culture supernatant.

Using the supernatant, Western blotting was carried out as follows. First, a peptide containing a part of the amino acid sequence (amino acid sequence of 166-180 in the amino acid sequence shown by SEQ ID NO :1) of the extracellular region of the human-derived TL4 protein was synthesized, and rabbit antiserum capable of recognizing the synthetic peptide was prepared by a publicly known method. Next, 5 µl of the culture supernatant described above was mixed with 5 µl of a sample treatment solution (0.25 M Tris-HCl, 2% SDS, 30% glycerol, 10% β-mercaptoethanol, 0.01%bromophenol blue, pH 6.8). After treating 95°C for 5 minutes, the mixture was subjected to SDS-polyacrylamide electrophoresis (10-20% gradient gel). After completion of the electrophoresis, the protein electrophoresed was transferred onto a nitrocellulose membrane (Pharmacia) using SemiPhor™, Hoefer Pharmacia BioTech, Inc.). The membrane was blocked with 3% gelatin-containing TBS (20 mM Tris, 500 mM NaCl, pH 7.5), washed with TTBS (0.05% Tween-20-containing TBS), and then reacted with the aforesaid rabbit antiserum diluted to 2000-fold with 1.0% gelatin-containing TTBS, at room temperature for 2 hours. After completion of the reaction, the membrane was washed twice with TTBS, and then reacted with alkaline phosphatase (AP)-labeled goat anti-rabbit IgG antibody diluted to 3000-fold with 1.0% gelatin-containing TTBS, at room temperature for an hour. After the membrane was washed twice with TTBS and then with TBS once, detection was performed using an AP color forming kit (BioRad, Inc.).

[FIG. 19] shows the results of Western blotting. A main band was noted at about 20 kD with the culture supernatant of the strain inserted with the expression vector and its signal intensity increased with passage of time, whereas no signal was noted with the culture supernatant of the pPICZαA-introducing strain for control.

### REFERENCE EXAMPLE 5

### Cloning of cDNA encoding rat-derived TL4 protein

Cloning of cDNA encoding the rat-derived TL4 protein was carried by PCR.

*Escherichia coli* DH12S strain from the SUPERSCRIPT™ rat liver cDNA library (GIBCO BRL) was incubated at 30°C for 16 hours in Terrific Broth (12 g/l Bacto-tryptone (Difco Co.), 24 g/l Bacto-yeast extract (Difco Co.), 2.3 g/l potassium monophosphate, 12.5 g/l potassium diphosphate, 0.4% glycerol) supplemented with 100 µg/ml of ampicillin. After collecting the cells, plasmid cDNA library was prepared using Qiagen Plasmid Kit (Qiagen, Inc.).

Using the DNA as a template and also using the following two synthetic oligonucleo tide as primer DNAs, PCR was carried out in the reaction system further using TaKaRa LA Taq (Takara Shuzo Co., Ltd.) as a DNA polymerase.

5'-CCTGACCCTGGGCTTCTGAGCCTC-3' (SEQ ID NO:23)

5'-TCCACAAAATCCATTGTCGTCATAGCC-3' (SEQ ID NO:24)

Using Thermal Cycler (GeneAmp^{R} PCR System 2400, Perkin-Elmer Co.), PCR was carried out by such a program that one cycle was set to include 94°C for a minute, 35 cycles set to include 98°C for 20 seconds, then 55°C for 30 seconds and then 72°C for 3 minutes, one cycle set to include 72°C for 2 minutes, and then allowed to stand at 4°C. After completion of the reaction, a part of the reaction solution was subjected to electrophoresis using 1.0% agarose gel. After a single band corresponding to the DNA fragment amplified by the PCR was confirmed, the DNA fragment was recovered using QIAquick Gel Extraction Kit (Qiagen). In order to determine the base sequence, the DNA fragment was inserted into and ligated to the T cloning site of pT7 Blue T-vector (Novagen, Inc.), using DNA Ligation Kit Version 2 (Takara Shuzo Co., Ltd.). After the ligation solution was introduced into *Escherichia coli* DH5α, two clones were screened from colonies of the ampicillin-resistant transformants that came out on an ampicillin-containing LB agar medium. From each of the clones, plasmid DNA was prepared. In order to determine the base sequence of each of the inserted DNAs for the clones, cycle sequencing using Thermo Sequenase™ dye terminator cycle sequencing pre-mix kit (Amersham) was performed on GeneAmp^{R} PCR System 2400, using each plasmid DNA as a template and further using as primers two primer DNAs (PRM-007, PRM-008) commercially available (from Toyobo, Inc.) and other oligo DNAs synthesized with a DNA synthesizer (Oligo 1000M, Beckman, Inc.), following the conditions instructed by the attached brochure. Then, the specimens were sequenced by DNA Sequencer 373A (Perkin-Elmer).

The base sequence obtained was verified by a gene analysis software, Lasergene (DYNASTAR Co.). The results reveal that the both clones contained, in their T cloning sites, the DNA fragment having the base sequence of 784 base pairs comprising open reading frame composed of the base sequence of 717 shown by SEQ ID NO:10, encoding the rat-derived TL4 protein comprising 239 amino acids represented by SEQ ID NO: 3 [FIGS. 20 and 21]. This rat-derived TL4 protein had 75% homology on an amino acid level to the human-derived TL4 protein having the amino acid sequence represented by SEQ ID NO:1, which was obtained in REFERENCE EXAMPLE 1, and the DNAs encoding these proteins were homologous by 74% on a base level. Furthermore, this rat-derived TL4 protein had 96% homology on an amino acid level to the mouse-derived TL4 protein having the amino acid sequence represented by SEQ ID NO:2, which was obtained in REFERENCE EXAMPLE 1, and the DNAs encoding these proteins were homologous by 94% on a base level.

The plasmid pTB2012 bearing the DNA encoding the rat-derived TL4 protein was transfected to *Escherichia coli* DH5α to obtain the transformant: *Escherichia coli* DH5α/pTB2012.

### EXAMPLE 1

### Production of soluble human TL4 using the insect cell expression system

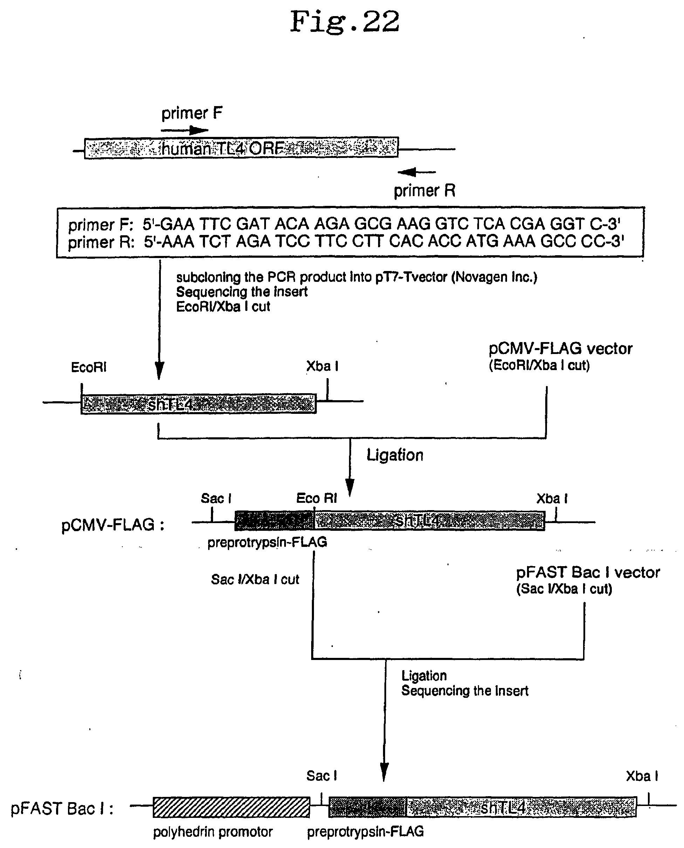

Using as a template plasmid pTB1940 inserted with the DNA encoding human TL4 protein, PCR was carried out using, as primers, a synthetic oligonucleotide added with the cleavage site of restriction enzyme EcoRI at the 5' end (5' GAATTCGATACAAGAGCGAAGGTCTCACGAGGTC 3'(SEQ ID NO:26)) and a synthetic oligonucleotide added with the cleavage site of restriction enzyme XbaI at the 3' end (5' AAATCTAGATCCTTCCTTCACACCATGAAAGCCCC 3'(SEQ ID NO:27)) to obtain the amplified DNA fragment of soluble TL4 encoding 84th isoleucine to 240th valine corresponding to the extracellular region of TL4. PCR was performed by treating at 94°C for a minute using DNA Thermal Cycler 9600, then repeating 25 cycles set to include 98°C for 10 seconds, 55°C for 5 seconds and 72°C for a minute, using ExTaq DNA polymerase. The amplified fragment thus obtained was treated with restriction enzymes EcoRI and XbaI. Furthermore, pCMV-FLAG plasmidwas treated similarly with restriction enzymes EcoRI and XbaI to acquire the DNA fragment encoding a signal sequence of preprotrypsin and FLAG protein added as a tag for facilitating purification and detection, respectively. The amplified DNA fragment of soluble TL4 treated with restriction enzyme was ligated to the preprotrypsin-FLAG protein-encoding DNA fragment at the 3' end. The obtained DNA fragment encoding the preprotrypsin-FLAG protein-soluble human TL4 protein was treated with restriction enzymes SacI and XbaI, which was inserted into vector pFAST Bacl (GIBCO BRL Lifetech, Inc.) for expression of inset cells, similarly treated with restriction enzymes SacI and XbaI (FIG. 22). The obtained TL4-expressed plasmid pFASTBac1/shTL4 was secreted in the cell culture supernatant in insect cells using preprotrypsin, and it was expected to be produced as a fused protein added wit the FLAG tag.

In the following procedures, Bac-to-Bac Baculovirus Expression System (GIBCO BRL Lifetech, Inc.) was used and the experimental procedures were conducted in accordance with the protocol attached. That is, recombinant plasmid pFAST Bacl/shTL4 inserted with DNA encoding the human-derived TL4 protein obtained was transfected to *Escherichia coli* DH10Bac attached. After a transformant was obtained, the recombinant bacmid was recovered from the transformant. The obtained recombinant bacmid was transfected to Sf9 insect cell using Celfectin reagent attached to obtain a recombinant baculovirus. After again infecting Sf9 insect cell with the recombinant baculovirus, incubation was continued for 4 to 5 days. FLAG-human TL4 fused protein secreted into the culture supernatant was purified using an anti-FLAG antibody column, which was named shTL4 and provided for the following experiments.

### EXAMPLE 2

### Confirmation of DNA synthesis promoting activity of normal human liver parenchymal cells by soluble human TL4

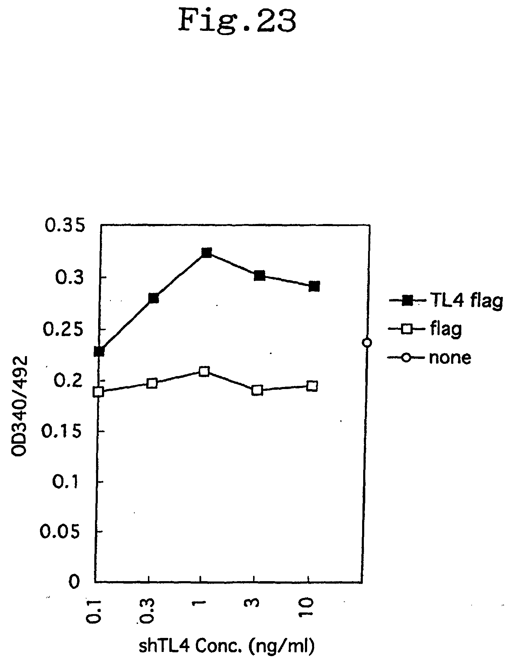

In order to examine the activity of shTL4 on DNA synthesis for normal human liver parenchymal cells, the following experiment was carried out. That is, normal human liver parenchymal cells (Cell Systems, Inc., #3716) suspended in serum-free CSC medium (Cell Systems, Inc.) were plated on a 96-well culture plate (FALCON, Inc.) coated with collagen type I, in 2,500 cells/well/50 ml. At the same time, shTL4 acquired and purified by the method of EXAMPLE 1 was added by a 3-fold common ratio in 50 ml/well (n = 2) to have a final concentration of 0.1 ng/ml to 10 ng/ml followed by incubation in a CO₂ incubator for 3 days (37°C, 5% CO₂). For detection of DNA synthesis, Cell Proliferation ELISA, BrdU kit (BOEHRINGER Inc.) was employed. That is, bromodeoxyuridine (BrdU) was added to each well in a final 1000-fold dilution followed by incubation overnight. Then, the culture broth was removed and 200 ml/well of Fix Denat solution was added followed by fixing the cells at room temperature for 30 minutes. Thereafter, the solution was removed and HRP-labeled anti-BrdU antibody solution was added in 100 ml/well followed by reacting them for 90 minutes at room temperature. After washing with PBS, the substrate was added in 50 ml/well to form a color for 30 minutes. Absorbance at a wavelength of 340 nm and absorbance at 492 nm for control was measured, using a 96-well plate reader (Multiscan Multisoft: Dai-Nippon Pharmaceutical Co., Ltd.). The results reveal that shTL4 dose-dependently promoted the uptake of BrdU in liver parenchymal cells, that is, DNA synthesis (FIG. 23).

### EXAMPLE 3

### DNA synthesis promoting activity of soluble human TL4 in normal human liver parenchymal cells under starvation

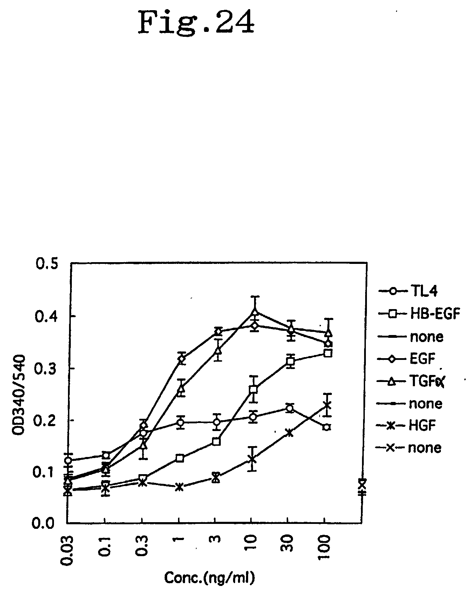

In order to examine the activity of shTL4 under starvation, basal medium obtained by mixing Ham F-12 medium and LEIBOVITZ L-15 medium (GIBCO BRL) in an equal volume was supplemented with 1% BSA, 5 mM glucose (WAKO), 10⁻⁸M dexamethasone (WAKO), 10⁻⁸M bovine insulin (GIBCO BRL) in a final concentration, respectively, which was used as a basic medium. Normal human liver parenchymal cells suspended in this medium were plated on a 96-well culture plate (FALCON, Inc.) coated with collagen type I, in 2,500 cells/well/50 ml, followed by incubation in a CO₂ incubator for 24 hours. After shTL4, human EGF, human heparin-bound EGF (HB-EGF), human TGFα or human HGF (each growth factor: R & D, Inc.) was added by a 3-fold common ratio in 50 ml/well (n-3) to have a final concentration of 0.03 ng/ml to 100 ng/ml followed by incubation in a CO₂ incubator for 3 days, DNA synthesis was detected as in EXAMPLE 1. As the result, even under starvation, shTL4 promoted the uptake of BrdU in liver parenchymal cells, that is, DNA synthesis, as in the other growth factors. It was thus shown that shTL4 had a promoting activity in the growth process of liver parenchymal cells (FIG. 24).

### EXAMPLE 4

### Cytotoxicity of soluble human TL4 against liver parenchymal cells when used in combination with actinomycin D (ActD)

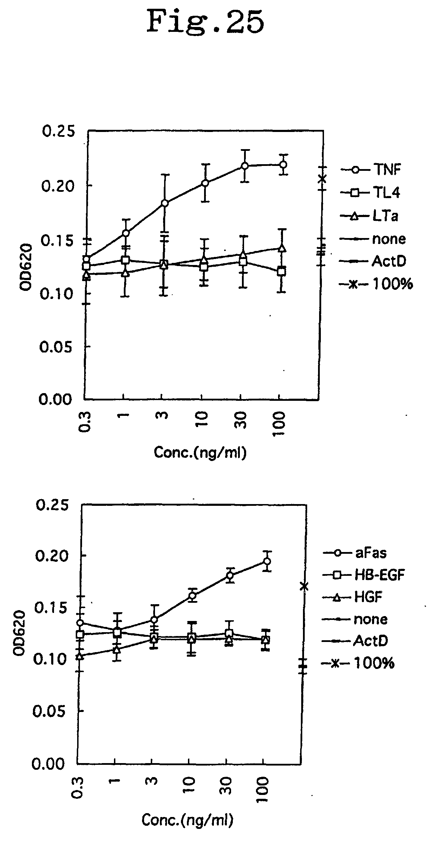

Normal human liver parenchymal cells were suspended in the basic medium described in EXAMPLE 3 supplemented with newly born calf serum (GIBCO BRL) in a final concentration of 10%. The cell suspension was plated on a 96-well culture plate (FALCON, Inc.) coated with collagen type I, in 5,000 cells/well/100 ml, followed by incubation in a CO₂ incubator overnight. Then, the medium was replaced by the basic medium supplemented with newly born calf serum (GIBCO BRL) in a final concentration of 1%, and 1.33 mM ActD (WAKO) was added to the medium in 50 ml/well, followed by incubation in a CO₂ incubator for 30 minutes. Thereafter shTL4, human TNFα (Genzyme, Inc.), human LTα (Genzyme, Inc.), anti-human Fas antibody (CH-11: MBL Inc.), human HB-EGF (R & D, Inc.) or human HGF (R & D, Inc.) was added by a 3-fold common ratio in 50 ml/well to have a final concentration of 0.3 ng/ml to 100 ng/ml followed by incubation in a CO₂ incubator for 24 hours. As an index for cytotoxicity, lactose dehydrogenase (LDH) activity in the culture supernatant was determined using LDH-Cytotoxic Test Wako (WAKO). That is, 10 ml of the culture supernatant, 40 ml of PBS and 50 ml of substrate were mixed with each other. After allowing to stand at room temperature for 45 minutes, absorbance was measured at 620 nm with a plate reader. The LDH activity was determined using as a blank the value obtained using medium in place of the culture supernatant, and as 100% when the cells were lysed in a medium containing 0.5% Tween 20 (BioRad, Inc.). TNFα and anti-Fas antibody dose-dependently increased the LDH activity in the culture supernatant, whereas no appreciable increase was noted with the addition of shTL4. That is, it was found that TNFα and anti-Fas antibody showed a remarkable cytotoxic activity, but shTL4 had not such activity. With respect to LTα, no appreciable increase of the LDH activity was noted, but some dead cells were observed microscopically (FIG. 25).

### EXAMPLE 5

### Detection of apoptosis induction activity using annexin-V and Propidium Iodide (P.I.)

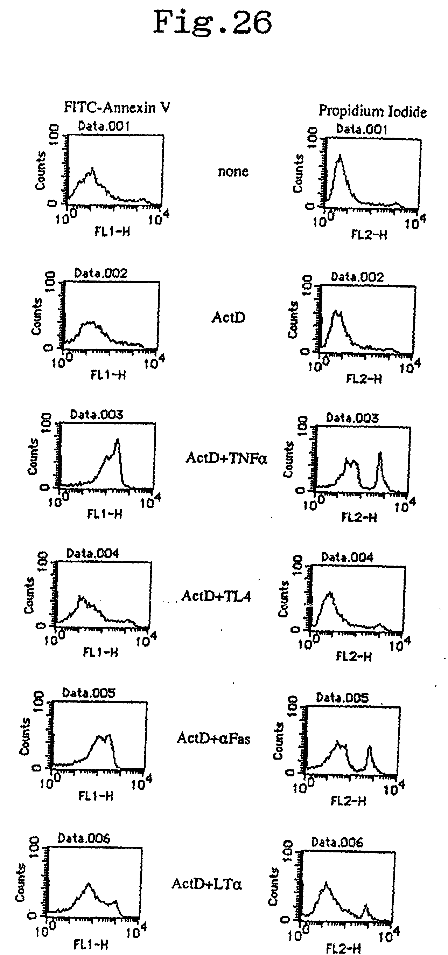

The difference between shTL4 and TNFα, anti-Fas antibody or LTα found in EXAMPLE 4 was examined in the apoptosis detection system using annexin-V and P.I. Normal human liver parenchymal cells were plated on a 6-well plate (FALCON, Inc.) coated with collagen type I, in 150,000 cells/well/2 ml, followed by incubation in a CO₂ incubator overnight. Then, after the medium was replaced as in EXAMPLE 4, ActD was added in a final concentration of 333 nM, followed by incubation in a CO₂ incubator for 30 minutes. Thereafter, shTL4, TNFα, LTα and anti-human Fas antibody was added to each well to have a final concentration of 100 ng/ml, respectively, followed by incubation for 15 hours. The cells were then recovered. For detection of apoptosis, Early Apoptosis detection kit (KAMIYA BIOMEDICAL, Inc.) was used for apoptosis detection. That is, after the cells were recovered by trypsin treatment, the cells were suspended in 400 ml of a binding buffer, and 10ml of FITC-annexin-V solution and 10 ml of P.I. solution were added to the suspension. The mixture was then allowed to stand for 30 minutes in the dark. Stained cells were analyzed by FACScan (Becton Dekinson). The conditions used for the analysis were FSC: E-1, 9.5, lin, SSC: 330, 1.0, lin, FL-1: 320, log, FL-2: 320, log, comp: FL1-1.1% FL2, FL2-24.4% FL1. When compared to the cells for control, the fluorescent intensity of FL-1 and FL-2 increased in the cells added with TNFα, LTα or anti-Fas antibody, whereas no increase was noted in the cells added with shTL4. That is, the cells added with TNFα, LTα or anti-Fas antibody were stained with annexin-V and P.I., whereas the shTL4-added cells were not stained. This indicates that TNFα, LTα and anti-Fas antibody had an apoptosis induction ability on liver parenchymal cells, but shTL4 had no such induction ability. The foregoing results reveal that shTL4 has no cytotoxic activity against liver parenchymal cells, unlike other ligands of the TNF family (FIG. 26).

### EXAMPLE 6

### Cloning of cDNA encoding novel Fas ligand-like soluble protein derived from human liver and determination of base sequence

Using human liver-derived cDNA as a template, PCR was performed using two primers, i.e., primer 1 (SEQ ID NO:28) and primer 2 (SEQ ID NO:29). Composition of the reaction solution in this reaction was obtained as follows: 33.5 ng of the above cDNA was used as a template; 1/50 volume of Advantage 2 Polymerase Mix (CLONTECH), 20 µM each of primer 1 (SEQ ID NO:28) and primer 2 (SEQ ID NO:29), 2.5 mM dNTPs and 1/10 buffer attached to enzymes, were added thereto, and the resulting mixture was made a total volume of 50 µl. PCR was effected by repeating one cycle set to include (1) 95°C for 30 seconds, then (2) 30 cycles set to include 95°C for 10 seconds, 58°C for 10 seconds and 72°C for 45 seconds and (3) finally extension at 72°C for 2 minutes. After completion of PCR, the reaction products obtained were the two of 723 bases and 615 bases. The reaction product of 615 base pairs was recovered from the gel and purified following the procedure of QIAquick Gel Extraction Kit (QIAGEN). The purified product was subcloned to plasmid vector pCR2.1-TOPO vector according to the formulation of TA Cloning Kit (Invitrogen, Inc.), and transfected to *Escherichia coli* DH5α. After the clones bearing the objective DNA were screened in ampicillin-supplemented LB agar medium, the respective clones were sequenced to obtain a cDNA sequence (SEQ ID NO:30) of 612 base pairs encoding a novel Fas ligand-like soluble protein. The novel Fas ligand-like soluble protein having the amino acid sequence (SEQ ID NO:31) deduced from this cDNA was named hTL4-2.

### EXAMPLE 7

### Synergistic DNA synthesis promoting activity of soluble human TL4 in DNA synthesis induction of normal human liver parenchymal cells by various growth factors

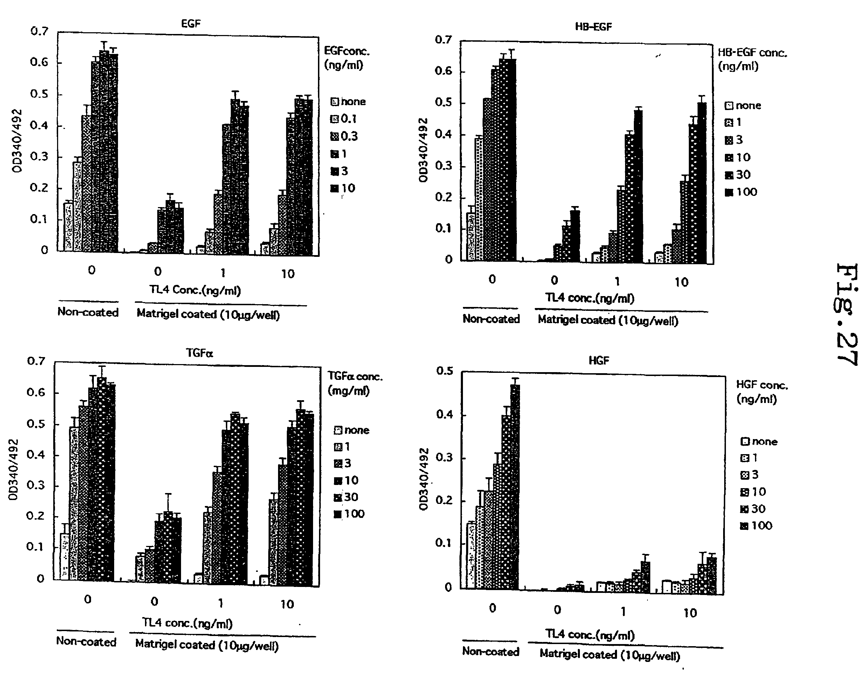

In order to clarify how the DNA synthesis promoting activity of soluble human TL4 (shTL4) observed in EXAMPLE 3 is exhibited in the presence of growth factors, the effect was monitored in the presence of various growth factors. First, basal medium obtained by mixing Ham F-12 medium and LEIBOVITZ L-15 medium (GIBCO BRL) in an equal volume was supplemented with 1% BSA, 5 mM glucose, 10⁻⁸M dexamethasone (all by WAKO), 10⁻⁸M bovine insulin (GIBCO BRL) in a final concentration, respectively, which was used as a basic medium. Normal human liver parenchymal cells were suspended in this medium were plated in 2,500 cells/well/50 ml on a 96-well culture plate (FALCON, Inc.) previously coated with a solution of EHS cell-derived extracellular substrate matrigel (FALCON, Inc.) in a concentration of 10 µg/well, followed by incubation in a CO₂ incubator for 24 hours. After shTL4 was added in a final dose of 1 or 10 ng/ml, human EGF in a dose of 0.1, 0.3, 1, 3 or 10 ng/ml; human heparin-bound EGF (HB-EGF), human TGFα or human HGF (all by R & D, Inc.) was added to have a final concentration of 1, 3, 10, 30 or 100 ng/ml, respectively. After incubation for 3 days, DNA synthesis was detected as in EXAMPLE 1 (n=3). As the result, low response to DNA synthesis by the growth factor for liver parenchymal cells was noted on the plate coated with matrigel, which is considered because dedifferentiation was inhibited by a growth inhibitory signal that would come from the matrigel. Even under these conditions, shTL4 promoted the uptake of BrdU in liver parenchymal cells, that is, DNA synthesis of liver cells, and the promoting activity was synergistic with other growth factors. It was thus shown that shTL4 was a synergistic induction promoting factor of DNA synthesis in the growth process of liver parenchymal cells by the growth factors (FIG. 27).

### EXAMPLE 8

### Study of change in expression of each gene in model mouse with carbon tetrachloride-induced hepatic disorder

In order to study what change in expression of various genes, including mouse TL4, show in model mice with carbon tetrachloride (CCl₄)-induced hepatic disorders, specific PCR strands amplified by PCR were detected and quantified by ABI PRISM™ 7700 Sequence Detection System (SDS 7700) (PE Applied Biosystems).

First, model mice with CCl₄-induced hepatic disorder were prepared and hepatectomized. That is, C57BL/6 mice (male, 7 weeks old) (purchased from Japan SLC) were weighed. After disinfecting the abdomen with 70% ethanol, an 8 fold dilution of CCl₄ (Wako Pure Chemicals) with corn oil (Wako Pure Chemicals) was intraperitoneally injected to each mouse (N-3) in a dose of 0.5 ml/kg. The animal was again weighed 1, 4, 7, 12, 24, 48 and 72 hours after. After ethereal aesthesis, the liver was removed and weighed. The lateral left lobe of the removed liver was stored in formalin and the remaining liver in three mice was all frozen instantaneously in liquid nitrogen, which was kept at -80°C since then. For control, model mice intraperitoneally given with corn oil alone were prepared and treated by the same procedures as above.

The GPT activity in plasma was assayed as follows. Using a syringe previously heparinized prior to hepatectomy at each experimental point of time, blood was collected from the heart. The blood collected was centrifuged at 3,000 rpm for 10 minutes to prepare plasma. The GPT activity in the plasma was assayed. The assay of the GPT activity in plasma was performed using STA TEST WAKO (Wako Pure Chemicals). The procedure was performed by a modification of the protocol attached. As the result, the GPT activity in plasma increased in the CCl₄-administered mice, showing the peak on 24 hours after administration followed by decrease. In the control group administered with corn oil, no increase of the GPT activity was observed, indicating that CCl₄-dependent hepatic disorders could be induced. Also, there was a tendency that the liver weight decreased up to 24 hours and then increased in the CCl₄-administered group, whereas the liver weight was almost constant in the control group. This reveals that after the hepatic disorder was induced, liver regeneration occurred. On the other hand, PCNA (Proliferating Cell Nuclear Antigen) immunostaining was performed by the following procedures. That is, after formalin-fixed liver was dehydrated, penetrated, soaked with paraffin, the tissue was embedded in an embedding dish using paraffin having a melting point of 60°C, which was sliced into a thickness of 2 to 3 µ. The slice was put on a slide glass, immersed in hot water of 48-52°C to spread. After drying in an incubator at 37°C for at least 2 hours, xylene and ethanol were successively passed, deparaffinized, and then thoroughly washed with water. After treating in 0.01M citrate buffer (pH 6) in an autoclave of 121°C for 10 minutes, the slice was immersed for 20 minutes in a solution mixture of 0.03% hydrogen peroxide water and 0.1% NaN₃-PBS for endogenous treatment. Penetration in a 100-fold diluted anti-PCNA mouse monoclonal antibody clone PC10 (DAKO)-containing solution at room temperature for 60 minutes was followed by washing with PBS for 40 minutes. Then, penetration was effected in a 100-fold diluted biotinylated anti-rabbit anti-mouse Ig and F(ab')₂ (DAKO) solution at room temperature for 60 minutes, followed by washing with PBS for 40 minutes. After reacting for 30 minutes in an enzyme reagent of streptoavidin and biotin complex/peroxidase label (DAKO) as a ternary reagent, washing with PBS was performed for 40 minutes. Thereafter, a color was formed in 7030 mg of DAB (3,3'-diaminobenzidine tetrahydrochloride), 0.05 M Tris-HCl (pH 7.6) and 0.01% hydrogen peroxide water, while monitoring the degree of color formation. Nuclear staining was performed with hematoxylin solution followed by enclosing through the procedures of dehydration and penetration, and PCNA-positive cells were counted under a light microscope. As the result, markedly positive cells were confirmed since 4 hours after the CCl₄ administration, which lasted at least 72 hours after. It was thus shown that excellent liver regeneration was induced by CCl₄.

Next, the total RNA was prepared from the liver removed from model mouse with CCl₄-induced hepatic disorder, using the following procedures. That is, the liver sample stored at -80°C was again put in liquid nitrogen. After it was confirmed that no bubble was formed, the liver was put on a powdered medicine wrapping paper with a spatula, crushed with a mallet and poured in 30 ml of ISOGEN (K.K. Nippon Gene) previously prepared. After homogenizing with a polytron homogenizer (KINEMATICA). which had been previously treated with about 10% hydrogen peroxide and rinsed with RNase free sterile water (K.K. Nippon Gene), the homogenate was settled at room temperature for 15 minutes. The homogenate was transferred into a new tube for 50 ml volume. After 15 ml of ISOGEN was further added and thoroughly mixed, 6 ml of chloroform (Wako Pure Chemical Industries Co., Ltd.) was added, stirred with a vortex, and settled at room temperature for 5 minutes. After centrifugation at 10,000 rpm for 15 minutes (4°C), 10 ml of the aqueous phase was recovered and an equal volume of 2-propanol (Wako Pure Chemical Industries Co., Ltd.) was added there to. The mixture was gently mixed and settled at room temperature for 10 minutes. After centrifugation at 10,000 rpm for 10 minutes (4°C), RNA precipitates adhered to the inner wall of the tube were recovered as gel-like pellets. Rinsing with 70% ethanol was followed by air drying. The precipitates were dissolved in 10-20 ml of RNase free sterile water in the aqueous phase of a warm bath at 58°C, and the concentration was measured to obtain the total RNA (tRNA) solution. Next, the tRNA was treated with DNase I using Message Clean Kit (Gen Hunter Corporation). The reaction composition in the reaction was that 100 µg of tRNA, 11.4 µl of the attached buffer and 2 µl of DNase I were mixed to make the total volume 113.4 µl. After reacting at 37°C for 30 minutes, RNA was purified according to the protocol for RNA cleanup of RNeasy Mini Kit (QIAGEN). The purified RNA was subjected to reverse transcription (RT) reaction according to the protocol of TaqMan Gold RT-PCR Kit (PE Applied Biosystems) . The reaction composition in the reaction was as follows: 2 µg of tRNA, 10 µl of 10xTaqMan RT buffer, 5.5 mM MgCl₂, 0.5 mM dNTPs, 2.5 µM Random Hexamer, 0.4 U/µl of RNase Inhibitor and 1.25 U/µl of MultiScribe Reverse Transcriptase were mixed to make the total volume 100 µl. After PCR at 25°C for 10 minutes, 48°C for 30 minutes and 95°C for 5 minutes, it was stored at -20°C as the cDNA solution.

The change in expression of each gene in model mice with hepatic disorder induced by CCl₄ administration was quantified by the TaqMan method. That is, TaqMan probes and primers were designed using Primer Express (a software manufactured by PE Applied Biosystems). TaqMan probe sequence (SEQ ID NO:32) and TaqMan primer sequences(SEQ ID NO:33, SEQ ID NO:34) of mouse TL4, TaqMan probe sequence (SEQ ID NO:35) and TaqMan primer sequences(SEQ ID NO:36, SEQ ID NO:37) of mouse TNFα, TaqMan probe sequence (SEQ ID NO:38) and TaqMan primer sequences (SEQ ID NO:39, SEQ ID NO:40) of mouse c-myc, TaqMan probe sequence (SEQ ID NO:41) and TaqMan primer sequences (SEQ ID NO:42, SEQ ID NO:43) of mouse HB-EGF, TaqMan probe sequence (SEQ ID NO:44) and TaqMan primer sequences (SEQ ID NO:45, SEQ ID NO:46) of mouse TGFα were selected and synthesized. The reaction composition in the TaqMan PCR was as follows: cDNA already prepared was used as a template, 12.5 µl of 2 x TaqMan Universal PCR Master Mix (PE Applied Biosystems), 200 nM TaqMan probe and 100 nM each of TaqMan primers were added to make the total volume 25 µl. PCR was performed at 50°C for 2 minute and 95°C for 10 minutes and then repeated 40 cycles set to include 95°C for 15 seconds and 62°C for a minute. Simultaneously at completion of the reaction, quantitative automated analysis of PCR was conducted. Dispersion between cDNAs was corrected using TaqMan Rodent GAPDH Control Reagents (PE Applied Biosystems).

Hereinafter the base sequences of the probes and primers described above are listed below.

SEQ ID NO: 32; CCAACGCCAGCTTGATAGGTATTGGTGG
SEQ ID NO: 33; CCCAGCAGCACATCTTACAGGA
SEQ ID NO: 34; AGGCCAAGTCGTGTCTCCCATA
SEQ ID NO: 35; CTATGGCCCAGACCCTCACACTCAGATCAT
SEQ ID NO: 36; CAAATGGCCTCCCTCTCATCAG
SEQ ID NO: 37; GGCTACAGGCTTGTCACTCGAA
SEQ ID NO: 38; ACAACGAAAAGGCCCCCAAGGTAGTGA
SEQ ID NO: 39; GTGACCAGATCCCTGAATTGGAA
SEQ ID NO: 40; GTAGGCGGTGGCTTTTTTGAG
SEQ ID NO: 41; CCTCTTGCAAATGCCTCCCTGGTTACCA
SEQ ID NO: 42; ATACAAGGACTACTGCATCCACGG
SEQ ID NO: 43; GTAGAGTCAGCCCATGACACCTGT
SEQ ID NO: 44; TGTCCTCATTATCACCTGTGTGCTGATCCA
SEQ ID NO: 45; AAGAAGCAAGCCATCACTGCC
SEQ ID NO: 46; ACAGTGTTTGCGGAGCTGACAG

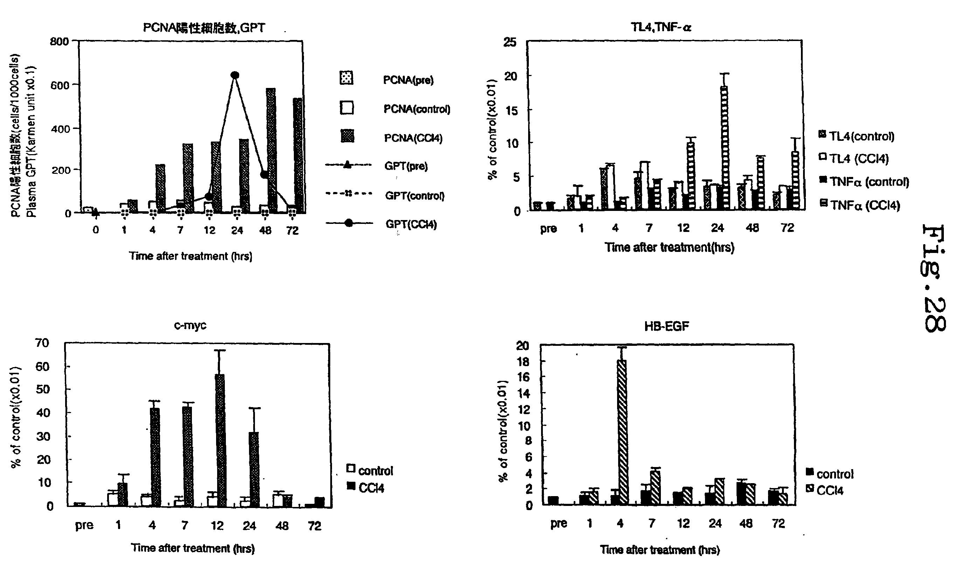

As shown in FIG. 28, the level of TL4 messenger RNA (mRNA) in the liver of the CCl₄-administered group increased with the peak at 7 hours and the expression then decreased. On the other hand, the expression showed a relatively high increase in the control group, but the level of TNFα mRNA in the liver showed increased expression after 24 hours, which revealed that the expression was considerably delayed than TL4. Turning to HB-EGF, the peak in expression was 4 hours after administration of CCl₄. In c-myc, a marked increase was noted 4 hours after administration and this increase lasted since then, showing the peak 12 hours after. Since the increase in expression of HB-EGF, which is an important hepatocyte proliferation factor in liver regeneration occurs at the relatively early stage like 4 hours after administration of CCl₄, and the increase of c-myc and PCNA-positive cells and the increase in expression of TL4 so as to be associated therewith, it was demonstrated that TL4 would be more likely to participate in regeneration of impaired liver more actively, rather than the role of TNFα in liver regeneration that has been hitherto reported. Taking into account that TL4 synergistically promotes DNA synthesis on a cell level by hepatocyte proliferation factors, including HB-EGF of normal liver parenchymal cell, these facts suggest that TL4 would play an important role in the course of repairing hepatic disorders.

### EXAMPLE 9

### Production of soluble mouse TL4 using the insect cell expression system

Using as a template plasmid pTB1958 inserted with the DNA encoding mouse TL4 protein (SEQ ID NO:2), PCR was carried out using, as primers, a synthetic oligonucleotide added with the cleavage site of restriction enzyme EcoRI at the 5' end (5' GTAGAATTCGGCCAACCCAGCAGCACATCTTAC 3'(SEQ ID NO:48)) and a synthetic oligonucleotide added with the cleavage site of restriction enzyme XbaI at the 3' end (5' AAATCTAGATATTGCTGGGTTTGAGGTGAGTCC 3'(SEQ ID NO:49)) to obtain the amplified DNA fragment of soluble TL4 encoding 90th alanine to 239th valine corresponding to the extracellular region of TL4. PCR was performed by treating at 94°C for a minute using DNA Thermal Cycler 9600, then repeating 25 cycles set to include 98°C for 10 seconds, 60°C for 5 seconds and 72°C for 1.5 minute using ExTaq DNA polymerase. The amplified fragment thus obtainedwas treated with restriction enzymes EcoRI and XbaI. Furthermore, pCMV-FLAG plasmid was treated similarly with restriction enzymes EcoRI and XbaI to acquire the DNA fragment encoding a signal sequence of preprotrypsin and FLAG protein added as a tag for facilitating purification and detection, respectively. The amplified DNA fragment of soluble TL4 treated with restriction enzyme was ligated to the preprotrypsin-FLAG protein-encoding DNA fragment at the 3' end. The obtained DNA fragment encoding the preprotrypsin-FLAG protein-soluble mouse TL4 protein was treated with restriction enzymes SacI and XbaI, which was inserted into vector pFAST Bacl (GIBCO BRL Lifetech, Inc.) for expression of inset cells, similarly treated with restriction enzymes SacI and XbaI. The obtained TL4-expressed plasmid pFAST Bac1/smTL4 was secreted in the cell culture supernatant in insect cells using preprotrypsin, and it was expected to be produced as a fused protein added wit the FLAG tag.

In the following procedures, Bac-to-Bac Baculovirus Expression System (GIBCO BRL Lifetech, Inc.) was used and the experimental procedures were conducted in accordance with the protocol attached. That is, recombinant plasmid pFAST Bac1/smTL4 inserted with DNA encoding the mouse-derived TL4 protein obtained was transfected to *Escherichia coli* DH10Bac attached. After a transformant was obtained, the recombinant bacmid??? was recovered from the transformant. The obtained recombinant bacmid??? was transfected to Sf9 insect cell using Celfectin reagent attached to obtain a recombinant baculovirus. After again infecting Sf9 insect cell with the recombinant baculovirus in a 1/20 amount of the total volume, incubation was continued for 2 days to recover FLAG-mouse TL4 fused protein secreted into the culture supernatant. The protein was concentrated on a UF membrane (MWCO 3, 000, 0.1 square meter) and replaced by TBS (50 mM Tris-HCl, 150 mM NaCl, pH 7.4). The protein was purified using an anti-FLAG M2 antibody column. The eluate was purified on Sephadex G-25 column and further purified again using the anti-FLAG M2 antibody column. After the respective fraction were confirmed by SDS-PAGE, fractions of high purity were collected and recovered. The fractions were concentrated by Centriplus 10K. The concentrate was purified through Sephadex G-25 column fol lowed by PBS replacement. The FLAG mouse TL4 fused protein was named smTL4 and provided for the following experiments.

### EXAMPLE 10 Study of change in expression of each gene in model mice with hepatic disorder induced by administration of concanavalin A

In order to study what change in expression various genes, including mouse TL4, exhibit in model mice with concanavalin A (ConA)-induced hepatic disorder, specific PCR chain amplified by PCR was detected and quantified by ABI PRISM™ 7700 Sequence Detection System (SDS 7700) (PE Applied Biosystems).

First, model mice with ConA-induced hepatic disorder werepreparedandhepatectomized. That is, C57BL/6mice (male, 6 weeks old) (purchased from Japan SLC) were weighed. After disinfecting the abdomen with 70% ethanol, a sample prepared to have a 5 mg/ml concentration of ConA (Wako Pure Chemicals) with PBS was intraperitoneally administered to each mouse (N=3) in a dose of 20 mg/kg. The animal was again weighed after 1, 4, 7, 12 and 24 hours passed. After ethereal aesthesis, the livers from the three mice were all frozen instantaneously in liquid nitrogen, which was kept at -80°C since then. For control, model mice intraperitoneally given with PBS alone were prepared and treated by the same procedures as above.

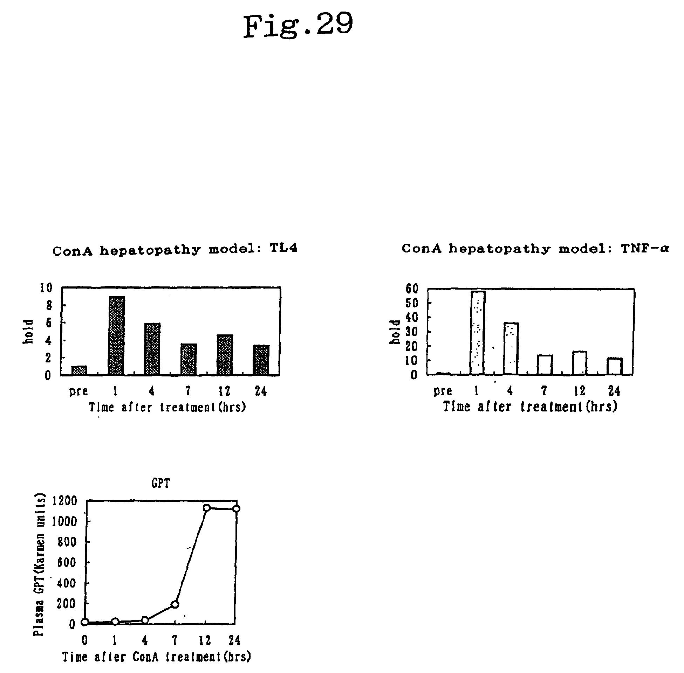

The GPT activity in plasma was assayed as follows. Using a syringe previously heparinized prior to hepatectomy at each experimental time, blood was collected from the heart. The blood collected was centrifuged at 3,000 rpm for 10 minutes to prepare plasma. The GPT activity in the plasma was assayed. The assay of the GPT activity in plasma was performed using STA TEST WAKO (Wako Pure Chemicals). The procedure was performed by a modification of the protocol attached. As the result, the GPT activity in plasma kept increasing in the ConA-administered mice on 7 hours after administration and continued the high level up to 24 hours. In the control group administered with PBS, no increase of the GPT activity was observed, indicating that ConA-dependent hepatic disorder could be induced (FIG. 29).

Next, the total RNA was prepared from the liver removed from model mouse with ConA-induced hepatic disorder, using the following procedures. That is, the liver sample stored at -80°C was again put in liquid nitrogen. After it was confirmed that no bubble was formed, the liver was put on a powdered medicine wrapping paper with a spatula, crushed with a mallet and poured in 30 ml of ISOGEN (K.K. Nippon Gene) previously prepared. After homogenizing with a polytron homogenizer (KINEMATICA), which had been previously treated with about 10% hydrogen peroxide and rinsed with RNase free sterile water (K.K. Nippon Gene), the homogenate was settled at room temperature for 15 minutes. The homogenate was transferred into a new tube for 50 ml volume. After 15 ml of ISOGEN was further added and thoroughly mixed, 6 ml of chloroform (Wako Pure Chemical Industries Co., Ltd.) was added, stirred with a vortex, and settled at room temperature for 5 minutes. After centrifugation at 10,000 rpm for 15 minutes (4°C), 10 ml of the aqueous phase was recovered and an equal volume of 2-propanol (Wako Pure Chemical Industries Co., Ltd.) was added thereto. The mixture was gently mixed and settled at room temperature for 10 minutes. After centrifugation at 10,000 rpm for 10 minutes (4°C), RNA precipitates adhered to the inner wall of the tube were recovered as gel-like pellets. Rinsing with 70% ethanol was followed by air drying. The precipitates were dissolved in 10-20 ml of RNase free sterile water in the aqueous phase of a warm bath at 58°C, and the concentration was measured to obtain the total RNA (tRNA) solution. Next, the tRNA was treated with DNase I using Message Clean Kit (Gen Hunter Corporation). The reaction composition in the reaction was that 100 µg of tRNA, 11.4 µl of the attached buffer and 2 µl of DNase I were mixed to make the total volume 113.4 µl. After reacting at 37°C for 30 minutes, RNA was purified according to the protocol for the RNA cleanup of RNeasy Mini Kit (QIAGEN). The purified RNA was subjected to reverse transcription (RT) reaction according to the protocol of TaqMan Gold RT-PCR Kit (PE Applied Biosystems) . The reaction composition in the reaction was as follows: 2 µg of tRNA, 10 µl of 10xTaqMan RT buffer, 5.5 mM MgCl₂, 0.5 mM dNTPs, 2.5 µM Random Hexamer, 0.4 U/µl of RNase Inhibitor and 1.25 U/µl of MultiScribe Reverse Transcriptase were mixed to make the total volume 100 µl. After PCR was carried out at 25°C for 10 minutes, 48°C for 30 minutes and 95°C for 5 minutes, it was stored at -20°C as the cDNA solution.

The change in expression of each gene in model mice with hepatic disorder induced by ConA administration was quantified by the TaqMan method. That is, TaqMan probes and primers were designed using Primer Express (a software manufactured by PE Applied Biosystems). TaqMan probe sequence (SEQ ID NO:50) and TaqMan primer sequences (SEQ ID NO:51, SEQ ID NO:52) of mouse TL4 and TaqMan probe sequence (SEQ ID NO:53) and TaqMan primer sequences(SEQ ID NO:54, SEQ ID NO:55) of mouse TNFα were selected and synthesis was performed. The reaction composition in the TaqMan PCR was as follows: cDNA already prepared was used as a template, 12.5 µl of 2x TaqMan Universal PCR Master Mix (PE Applied Biosystems), 200 nM TaqMan probe and 100 nM each of TaqMan primers were added to make the total volume 25 µl. PCR was performed at 50°C for 2 minute and 95°C for 10 minutes and then repeated 40 cycles in which one cycle was set to include (95°C for 15 seconds and 62°C for a minute) . Simultaneously at completion of the reaction, quantitative automated analysis of PCR was conducted. Dispersion between cDNAs was corrected using TaqMan Rodent GAPDH Control Reagents (PE Applied Biosystems).

Hereinafter the base sequences of the probes and primers described above are listed below.

SEQ ID NO: 50; CCAACGCCAGCTTGATAGGTATTGGTGG
SEQ ID NO: 51; CCCAGCAGCACATCTTACAGGA
SEQ ID NO: 52; AGGCCAAGTCGTGTCTCCCATA
SEQ ID NO: 53; CTATGGCCCAGACCCTCACACTCAGATCAT
SEQ ID NO: 54; CAAATGGCCTCCCTCTCATCAG
SEQ ID NO: 54; GGCTACAGGCTTGTCACTCGAA

As the results, the level of TL4 messenger RNA (mRNA) in the liver of the ConA-administered group increased to about 10 times that of the control group an hour after, and maintained the increased expression of 4 to 5 times up to 24 hours since then. On the other hand, the level of TNFα mRNA in the liver increased to about 60 times that of the control group an hour after, and maintained the increased expression of about 10 times up to 24 hours since then (FIG. 29). These results suggest that by administration of ConA, the expression of TL4 would give arise and increase at an early stage, and then the GPT value would increase to cause hepatic disorder.

### EXAMPLE 11

### Anti-apoptosis activity of soluble human TL4 against apoptosis of normal human liver parenchymal cells induced by actinomycin D and TNFα

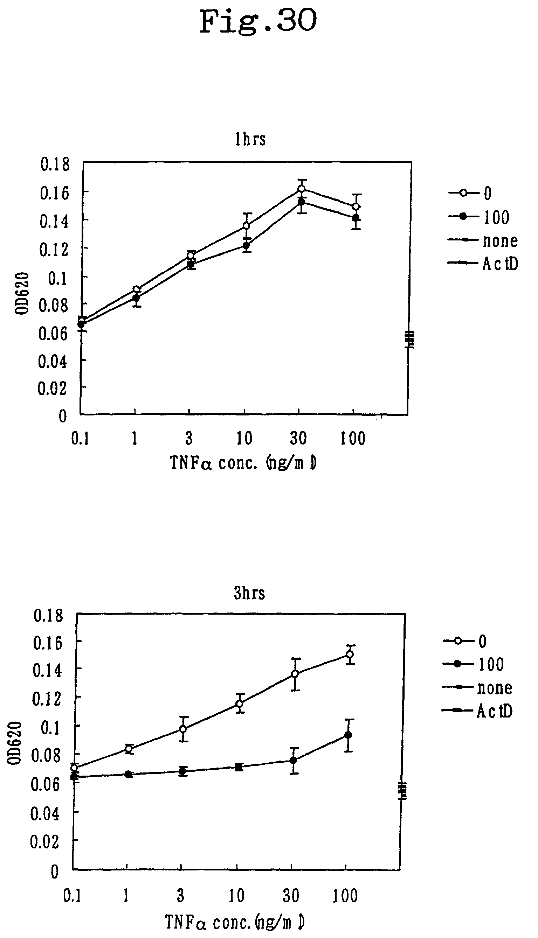

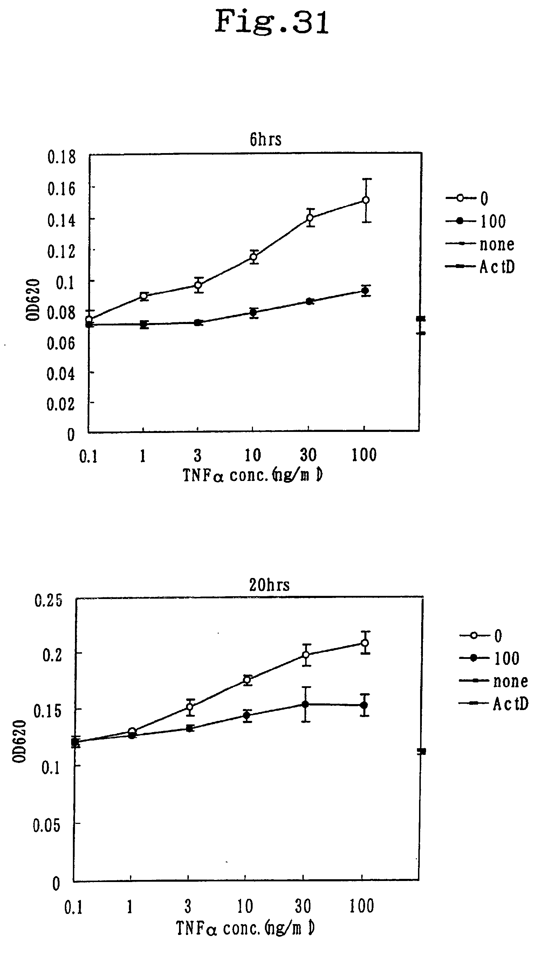

It was studied how TL4 would affect the apoptosis of normal human liver parenchymal cells induced by actinomycin D (ActD) and TNFα found in EXAMPLES 3 and 4. The culture conditions and experimental conditions were the same as in EXAMPLES 3 and 4, except those described below. The results reveal that by previously adding soluble human TL4 upon apoptosis induction in normal human liver parenchymal cells by simultaneous administration of ActD and TNFα, apoptosis by TNFα could be prevented. This anti-apoptosis activity of TL4 was found to be markedly induced by adding TL4 3 hours or more prior to the stimulation with ActD and TNFα (FIGS. 30 and 31, wherein -○- and -●- denotes a sample added with no soluble human TL4 and a sample added with 100 ng/ml of soluble human TL4, respectively). That is, the results reveal that TL4 itself does not show any cytotoxicity against liver parenchymal cells but has an activity of suppressing the apoptosis action by TNFα. It can be expected from the results that by administration of TL4 or administration of a low molecular compound or an antibody (agonist) having a similar activity to that of TL4, various hepatic disorders (diseases) associated with TNFα could be improved.

### INDUSTRIAL APPLICABILITY

The protein of the present invention, its partial peptide, or salts thereof have a liver function controlling activity (e.g., a liver cell maintenance activity, a liver cell death inhibiting activity, etc.), specifically, aliver regeneration activity (preferably, a liver parenchymal cell growth activity), etc., more specifically, an activity of promoting transfer from the G0 phase to the G1 phase in the cell cycle (preferably, the cell cycle of liver cells), etc., and are therefore useful also as medicaments as, e.g., a liver regeneration agent after partial hepatectomy (removal) in the patient with liver cancer.

## Claims

1. A liver function controlling agent comprising a protein containing an amino acid sequence, which is the same or substantially the same as the amino acid sequence represented by SEQ ID NO:1, SEQ ID NO:2, SEQ ID NO:3 or SEQ ID NO:31, or a salt thereof.

2. The agent according to claim 1. wherein substantially the same amino acid sequence as the amino acid sequence represented by SEQ ID NO:1, SEQ ID NO:2 or SEQ ID NO:3 is an amino acid sequence of 8-21, 54-59, 93-102, 109-116, 118-126, 128-134, 144-149, 162-170, 176-182, 184-189, 193-213, 215-219 and 228-240 in the amino acid sequence represented by SEQ ID NO:1.

3. The liver function controlling agent comprising a partial peptide of the protein according to claim 1, or a salt thereof.

4. The agent according to claim 3, wherein a partial peptide of the protein according to claim 1 is a peptide comprising an amino acid sequence having an amino acid sequence of 84-240 in the amino acid sequence represented by SEQ ID NO:1.

5. The liver function controlling agent comprising a DNA containing a DNA having a base sequence encoding the protein according to claim 1 or the partial peptide according to claim 3.

6. The agent according to claim 5, wherein the DNA is a DNA having a base sequence represented by any one of SEQ ID NO:4 to SEQ ID NO:10, or by SEQ ID NO:30.

7. The liver function controlling agent comprising an antibody to the protein according to claim 1 or the partial peptide according to claim 3, or to a salt thereof.

8. A method of screening a liver function controlling agent which comprises using (a) the protein according to claim 1 or the partial peptide according to claim 3, or a salt thereof, (b) the DNA according to claim 5, or (c) the antibody according to claim 7.

9. A liver function controlling agent comprising a compound obtained using the screening method according to claim 8, or a salt thereof.

10. The agent according to claim 1, 3, 5, 7 or 9, which is a liver function promoting agent.

11. The agent according to claim 1, 3, 5, 7 or 9, which is a liver regenerating agent.

12. The agent according to claim 1, 3, 5, 7 or 9, which has an action for promoting transfer from the G0 phase to the G1 phase of the cell cycle.

13. A protein comprising an amino acid sequence represented by SEQ ID NO:31, or a salt thereof.

14. A DNA bearing a DNA having a base sequence encoding the protein according to claim 13.

15. The DNA according to claim 14 having a base sequence represented by SEQ ID NO:30.

16. A recombinant vector containing the DNA according to claim 15.

17. A transformant transformed by the recombinant vector according to claim 16.

18. A process of producing the protein or its salt according to claim 13, which comprises culturing the transformant according to claim 17, producing, accumulating and collecting the protein according to claim 13.

19. An antibody to the protein or its salt according to claim 13.

20. A diagnostic agent comprising the DNA according to claim 14 or the antibody according to claim 19.

21. An antisense DNA containing a base sequence complementary or substantially complementary to the DNA according to claim 14 and having an action capable of suppressing expression of the DNA.

22. A method of screening a compound or its salt that accelerates or inhibits the activity of the protein or its salt according to claim 13, which comprises using the protein or its salt according to claim 13.

23. A kit for screening a compound or its salt that accelerates or inhibits the activity of the protein or its salt according to claim 13, comprising the protein or its salt according to claim 13.

24. A compound or its salt that accelerates or inhibits the activity of the protein or its salt according to claim 13, which is obtainable using the method of screening according to claim 22 or the kit for screening according to claim 23.

25. A pharmaceutical composition comprising the compound or its salt according to claim 24.

26. The pharmaceutical composition according to claim 25, which is a liver function controlling agent.


[Sequence Listing]
<110> Takeda Chemical Industries, Ltd.
<120> Liver Function Modulator
<130> 2610WOOP
<150> JP 11-141106
<151> 1999-05-21
<150> JP 2000-014044
<151> 2000-01-19
<160> 55
<210> 1
<211> 240
<212> PRT
<213> Human
<400> 1
Met Glu Glu Ser Val Val Arg Pro Ser Val Phe Val Val Asp Gly Gln
1               5                   10                  15
Thr Asp Ile Pro Phe Thr Arg Leu Gly Arg Ser His Arg Arg Gln Ser
            20                  25                  30
Cys Ser Val Ala Arg Val Gly Leu Gly Leu Leu Leu Leu Leu Met Gly
        35                  40                  45
Ala Gly Leu Ala Val Gln Gly Trp Phe Leu Leu Gln Leu His Trp Arg
    50                  55                  60
Leu Gly Glu Met Val Thr Arg Leu Pro Asp Gly Pro Ala Gly Ser Trp
65                  70                  75                  80
Glu Gln Leu Ile Gln Glu Arg Arg Ser His Glu Val Asn Pro Ala Ala
                85                  90                  95

His Leu Thr Gly Ala Asn Ser Ser Leu Thr Gly Ser Gly Gly Pro Leu
            100                 105                 110
Leu Trp Glu Thr Gln Leu Gly Leu Ala Phe Leu Arg Gly Leu Ser Tyr
        115                 120                 125
His Asp Gly Ala Leu Val Val Thr Lys Ala Gly Tyr Tyr Tyr Ile Tyr
    130                 135                 140
Ser Lys Val Gln Leu Gly Gly Val Gly Cys Pro Leu Gly Leu Ala Ser
145                 150                 155                 160
Thr Ile Thr His Gly Leu Tyr Lys Arg Thr Pro Arg Tyr Pro Glu Glu
                165                 170                 175
Leu Glu Leu Leu Val Ser Gln Gln Ser Pro Cys Gly Arg Ala Thr Ser
            180                 185                 190
Ser Ser Arg Val Trp Trp Asp Ser Ser Phe Leu Gly Gly Val Val His
        195                 200                 205
Leu Glu Ala Gly Glu Lys Val Val Val Arg Val Leu Asp Glu Arg Leu
    210                 215                 220
Val Arg Leu Arg Asp Gly Thr Arg Ser Tyr Phe Gly Ala Phe Met Val
225                 230                 235                 240
<210> 2
<211> 239
<212> PRT
<213> Murine
<400> 2
Met Glu Ser Val Val Gln Pro Ser Val Phe Val Val Asp Gly Gln Thr
1               5                   10                  15
Asp Ile Pro Phe Arg Arg Leu Glu Gln Asn His Arg Arg Arg Arg Cys

            20                  25                  30
Gly Thr Val Gln Val Ser Leu Ala Leu Val Leu Leu Leu Gly Ala Gly
        35                  40                  45
Leu Ala Thr Gln Gly Trp Phe Leu Leu Arg Leu His Gln Arg Leu Gly
    50                  55                  60
Asp Ile Val Ala His Leu Pro Asp Gly Gly Lys Gly Ser Trp Glu Lys
65                  70                  75                  80
Leu Ile Gln Asp Gln Arg Ser His Gln Ala Asn Pro Ala Ala His Leu
                85                  90                  95
Thr Gly Ala Asn Ala Ser Leu Ile Gly Ile Gly Gly Pro Leu Leu Trp
            100                 105                 110
Glu Thr Arg Leu Gly Leu Ala Phe Leu Arg Gly Leu Thr Tyr His Asp
        115                 120                 125
Gly Ala Leu Val Thr Met Glu Pro Gly Tyr Tyr Tyr Val Tyr Ser Lys
    130                 135                 140
Val Gln Leu Ser Gly Val Gly Cys Pro Gln Gly Leu Ala Asn Gly Leu
145                 150                 155                 160
Pro Ile Thr His Gly Leu Tyr Lys Arg Thr Ser Arg Tyr Pro Lys Glu
                165                 170                 175
Leu Glu Leu Leu Val Ser Arg Arg Ser Pro Cys Gly Arg Ala Asn Ser
            180                 185                 190
Ser Arg Val Trp Trp Asp Ser Ser Phe Leu Gly Gly Val Val His Leu
        195                 200                 205
Glu Ala Gly Glu Glu Val Val Val Arg Val Pro Gly Asn Arg Leu Val
    210                 215                 220
Arg Pro Arg Asp Gly Thr Arg Ser Tyr Phe Gly Ala Phe Met Val

225                    230                    235
<210> 3
<211> 239
<212> PRT
<213> Rat
<400> 3
Met Glu Ser Val Val Gln Pro Ser Val Phe Val Val Asp Gly Gln Thr
1               5                   10                  15
Asp Ile Pro Phe Arg Arg Leu Gly Gln Asn His Arg Arg Arg His Cys
            20                  25                  30
Gly Thr Val Gln Val Ser Leu Ala Leu Leu Leu Leu Leu Gly Ala Gly
        35                  40                  45
Leu Ala Thr Glu Gly Trp Phe Leu Leu Arg Leu His Gln Arg Leu Gly
    50                  55                  60
Asp Ile Val Ala His Leu Pro Asp Gly Gly Lys Gly Ser Trp Glu Lys
65                  70                  75                  80
Leu Ile Gln Asp Gln Arg Ser His Gln Pro Asn Pro Ala Ala His Leu
                85                  90                  95
Thr Gly Ala Asn Ala Ser Leu Ile Gly Ile Gly Gly Pro Leu Leu Trp
            100                 105                 110
Glu Thr Gln Leu Gly Leu Ala Phe Leu Arg Gly Leu Thr Tyr His Asp
        115                 120                 125
Gly Ala Leu Val Thr Thr Glu Ala Gly Tyr Tyr Tyr Val Tyr Ser Lys
    130                 135                 140
Val Gln Leu Ser Gly Val Gly Cys Pro Gln Gly Leu Ala Asn Gly Leu
145                 150                 155                 160

Pro Ile Thr His Gly Leu Tyr Lys Arg Thr Ser Arg Tyr Pro Lys Glu
                165                 170                 175
Leu Glu Leu Leu Val Ser Arg Arg Ser Pro Cys Gly Arg Ala Asn Ser
            180                 185                 190
Ser Arg Val Trp Trp Asp Ser Ser Phe Leu Gly Gly Val Val His Leu
        195                 200                 205
Glu Ala Gly Glu Glu Val Val Val Arg Val Pro Gly Asn Arg Leu Val
    210                 215                 220
Arg Pro Arg Asp Gly Thr Arg Ser Tyr Phe Gly Ala Phe Met Ile
225                 230                 235
<210> 4
<211> 720
<212> DNA
<213> Human
<400> 4
ATGGAGGAGA GTGTCGTACG GCCCTCAGTG TTTGTGGTGG ATGGACAGAC CGACATCCCA 60
TTCACGAGGC TGGGACGAAG CCACCGGAGA CAGTCGTGCA GTGTGGCCCG GGTGGGTCTG 120
GGTCTCTTGC TGTTGCTGAT GGGGGCTGGG CTGGCCGTCC AAGGCTGGTT CCTCCTGCAG 180
CTGCACTGGC GTCTAGGAGA GATGGTCACC CGCCTGCCTG ACGGACCTGC AGGCTCCTGG 240
GAGCAGCTGA TACAAGAGCG AAGGTCTCAC GAGGTCAACC CAGCAGCGCA TCTCACAGGG 300
GCCAACTCCA GCTTGACCGG CAGCGGGGGG CCGCTGTTAT GGGAGACTCA GCTGGGCCTG 360
GCCTTCCTGA GGGGCCTCAG CTACCACGAT GGGGCCCTTG TGGTCACCAA AGCTGGCTAC 420
TACTACATCT ACTCCAAGGT GCAGCTGGGC GGTGTGGGCT GCCCGCTGGG CCTGGCCAGC 480
ACCATCACCC ACGGCCTCTA CAAGCGCACA CCCCGCTACC CCGAGGAGCT GGAGCTGTTG 540
GTCAGCCAGC AGTCACCCTG CGGACGGGCC ACCAGCAGCT CCCGGGTCTG GTGGGACAGC 600
AGCTTCCTGG GTGGTGTGGT ACACCTGGAG GCTGGGGAGA AGGTGGTCGT CCGTGTGCTG 660

GATGAACGCC TGGTTCGACT GCGTGATGGT ACCCGGTCTT ACTTCGGGGC TTTCATGGTG 720
<210> 5
<211> 1491
<212> DNA
<213> Human
<400> 5
CGAGACTCCA TCTCAAAAAC AAAACAAATA AACGAACAAA AAAACCCACA ACGTATTATT 60
TTCTTGTTTA CGAGGTTTCT TGTCTCTCTG GCTCCACCAG AAGAGGAGCA GGGACCCTTC 120
TTGCTGTTGT TCATTGCTGC ATCCCCCACA CCGAGAGCAG AGCCTGGCAT GGGCAGAAAG 180
TCCTCAGTCG ATATTTGGTG GCCCCAAGCG AATGAAGCAT CCAAGAAGGG AAAGCTGGGG 240
GCTCCCCACT GCACTTGCCA CCTGAGTCAC ATTTTCAGAA GCCTCTGGAA AGTCGTGCAC 300
AGCCCAGGAG TGTTGAGCAA TTTCGGTTTC CTCTGAGGTT GAAGGACCCA GGCGTGTCAG 360
CCCTGCTCCA GACACCTTGG GCATGGAGGA GAGTGTCGTA CGGCCCTCAG TGTTTGTGGT 420
GGATGGACAG ACCGACATCC CATTCACGAG GCTGGGACGA AGCCACCGGA GACAGTCGTG 480
CAGTGTGGCC CGGGTGGGTC TGGGTCTCTT GCTGTTGCTG ATGGGGGCTG GGCTGGCCGT 540
CCAAGGCTGG TTCCTCCTGC AGCTGCACTG GCGTCTAGGA GAGATGGTCA CCCGCCTGCC 600
TGACGGACCT GCAGGCTCCT GGGAGCAGCT GATACAAGAG CGAAGGTCTC ACGAGGTCAA 660
CCCAGCAGCG CATCTCACAG GGGCCAACTC CAGCTTGACC GGCAGCGGGG GGCCGCTGTT 720
ATGGGAGACT CAGCTGGGCC TGGCCTTCCT GAGGGGCCTC AGCTACCACG ATGGGGCCCT 780
TGTGGTCACC AAAGCTGGCT ACTACTACAT CTACTCCAAG GTGCAGCTGG GCGGTGTGGG 840
CTGCCCGCTG GGCCTGGCCA GCACCATCAC CCACGGCCTC TACAAGCGCA CACCCCGCTA 900
CCCCGAGGAG CTGGAGCTGT TGGTCAGCCA GCAGTCACCC TGCGGACGGG CCACCAGCAG 960
CTCCCGGGTC TGGTGGGACA GCAGCTTCCT GGGTGGTGTG GTACACCTGG AGGCTGGGGA 1020
GAAGGTGGTC GTCCGTGTGC TGGATGAACG CCTGGTTCGA CTGCGTGATG GTACCCGGTC 1080
TTACTTCGGG GCTTTCATGG TGTGAAGGAA GGAGCGTGGT GCATTGGACA TGGGTCTGAC 1140
ACGTGGAGAA CTCAGAGGGT GCCTCAGGGG AAAGAAAACT CACGAAGCAG AGGCTGGGCG 1200

TGGTGGCTCT CGCCTGTAAT CCCAGCACTT TGGGAGGCCA AGGCAGGCGG ATCACCTGAG 1260
GTCAGGAGTT CGAGACCAGC CTGGCTAACA TGGCAAAACC CCATCTCTAC TAAAAATACA 1320
AAAATTAGCC GGACGTGGTG GTGCCTGCCT GTAATCCAGC TACTCAGGAG GCTGAGGCAG 1380
GATAATTTTG CTTAAACCCG GGAGGCGGAG GTTGCAGTGA GCCGAGATCA CACCACTGCA 1440
CTCCAACCTG GGAAACGCAG TGAGACTGTG CCTCAAAAAA AAAAAAAAAA A 1491
<210> 6
<211> 1353
<212> DNA
<213> Human
<400> 6
CCCACGCGTC CGCCCACGCG TCCGCTGAGG TTGAAGGACC CAGGCGTGTC AGCCCTGCTC 60
CAGACACCTT GGGCATGGAG GAGAGTGTCG TACGGCCCTC AGTGTTTGTG GTGGATGGAC 120
AGACCGACAT CCCATTCACG AGGCTGGGAC GAAGCCACCG GAGACAGTCG TGCAGTGTGG 180
CCCGGGTGGG TCTGGGTCTC TTGCTGTTGC TGATGGGGGC TGGGCTGGCC GTCCAAGGCT 240
GGTTCCTCCT GCAGCTGCAC TGGCGTCTAG GAGAGATGGT CACCCGCCTG CCTGACGGAC 300
CTGCAGGCTC CTGGGAGCAG CTGATACAAG AGCGAAGGTC TCACGAGGTC AACCCAGCAG 360
CGCATCTCAC AGGGGCCAAC TCCAGCTTGA CCGGCAGCGG GGGGCCGCTG TTATGGGAGA 420
CTCAGCTGGG CCTGGCCTTC CTGAGGGGCC TCAGCTACCA CGATGGGGCC CTTGTGGTCA 480
CCAAAGCTGG CTACTACTAC ATCTACTCCA AGGTGCAGCT GGGCGGTGTG GGCTGCCCGC 540
TGGGCCTGGC CAGCACCATC ACCCACGGCC TCTACAAGCG CACACCCCGC TACCCCGAGG 600
AGCTGGAGCT GTTGGTCAGC CAGCAGTCAC CCTGCGGACG GGCCACCAGC AGCTCCCGGG 660
TCTGGTGGGA CAGCAGCTTC CTGGGTGGTG TGGTACACCT GGAGGCTGGG GAGAAGGTGG 720
TCGTCCGTGT GCTGGATGAA CGCCTGGTTC GACTGCGTGA TGGTACCCGG TCTTACTTCG 780
GGGCTTTCAT GGTGTGAAGG AAGGAGCGTG GTGCATTGGA CATGGGTCTG ACACGTGGAG 840
AACTCAGAGG GTGCCTCAGG GGAAAGAAAA CTCACGAAGC AGAGGCTGGG ATTACAGGCG 900
TGAGCCACTG TTCCCAGCAG GAATTTCTTT TTTATAGTAT TGGATAAAGT TTGGTGTTTT 960

TACAGAGGAG AAGCAATGGG TCTTAGCTCT TTCTCTATTA TGTTATCATC CTCCCTTTTT 1020
TGTACAATAT GTTGTTTACC TGAAAGGAAG GTTTCTATTC GTTGGTTGTG GACCTGGACA 1080
AAGTCCAAGT CTGTGGAACT TAAAACCTTG AAGGTCTGTC ATAGGACTCT GGACAATCTC 1140
ACACCTTAGC TATTCCCAGG GAACCCCAGG GGGCAACTGA CATTGCTCCA AGATGTTCTC 1200
CTGATGTAGC TTGAGATATA AAGGAAAGGC CCTGCACAGG TGGCTGTTTC TTGTCTGTTA 1260
TGTCAGAGGA ACAGTCCTGT TCAGAAAGGG GCTCTTCTGA GCAGAAATGG CTAATAAACT 1320
TTGTGCTGAT CTGGAAAAAA AAAAAAAAAA AAA 1353
<210> 7
<211> 717
<212> DNA
<213> Murine
<400> 7
ATGGAGAGTG TGGTACAGCC TTCAGTGTTT GTGGTGGATG GACAGACGGA CATCCCATTC 60
AGGCGGCTGG AACAGAACCA CCGGAGACGG CGCTGTGGCA CTGTCCAGGT CAGCCTGGCC 120
CTGGTGCTGC TGCTAGGTGC TGGGCTGGCC ACTCAGGGCT GGTTTCTCCT GAGACTGCAT 180
CAACGTCTTG GAGACATAGT AGCTCATCTG CCAGATGGAG GCAAAGGCTC CTGGGAGAAG 240
CTGATACAAG ATCAACGATC TCACCAGGCC AACCCAGCAG CACATCTTAC AGGAGCCAAC 300
GCCAGCTTGA TAGGTATTGG TGGACCTCTG TTATGGGAGA CACGACTTGG CCTGGCCTTC 360
TTGAGGGGCT TGACGTATCA TGATGGGGCC CTGGTGACCA TGGAGCCCGG TTACTACTAT 420
GTGTACTCCA AAGTGCAGCT GAGCGGCGTG GGCTGCCCCC AGGGGCTGGC CAATGGCCTC 480
CCCATCACCC ATGGACTATA CAAGCGCACA TCCCGCTACC CGAAGGAGTT AGAACTGCTG 540
GTCAGTCGGC GGTCACCCTG TGGCCGGGCC AACAGCTCCC GAGTCTGGTG GGACAGCAGC 600
TTCCTGGGCG GCGTGGTACA TCTGGAGGCT GGGGAAGAGG TGGTGGTCCG CGTGCCTGGA 660
AACCGCCTGG TCAGACCACG TGACGGCACC AGGTCCTATT TCGGAGCTTT CATGGTC 717
<210> 8
<211> 795

<212> DNA
<213> Murine
<400> 8
TCTGCTCTGG CATGGAGAGT GTGGTACAGC CTTCAGTGTT TGTGGTGGAT GGACAGACGG 60
ACATCCCATT CAGGCGGCTG GAACAGAACC ACCGGAGACG GCGCTGTGGC ACTGTCCAGG 120
TCAGCCTGGC CCTGGTGCTG CTGCTAGGTG CTGGGCTGGC CACTCAGGGC TGGTTTCTCC 180
TGAGACTGCA TCAACGTCTT GGAGACATAG TAGCTCATCT GCCAGATGGA GGCAAAGGCT 240
CCTGGGAGAA GCTGATACAA GATCAACGAT CTCACCAGGC CAACCCAGCA GCACATCTTA 300
CAGGAGCCAA CGCCAGCTTG ATAGGTATTG GTGGACCTCT GTTATGGGAG ACACGACTTG 360
GCCTGGCCTT CTTGAGGGGC TTGACGTATC ATGATGGGGC CCTGGTGACC ATGGAGCCCG 420
GTTACTACTA TGTGTACTCC AAAGTGCAGC TGAGCGGCGT GGGCTGCCCC CAGGGGCTGG 480
CCAATGGCCT CCCCATCACC CATGGACTAT ACAAGCGCAC ATCCCGCTAC CCGAAGGAGT 540
TAGAACTGCT GGTCAGTCGG CGGTCACCCT GTGGCCGGGC CAACAGCTCC CGAGTCTGGT 600
GGGACAGCAG CTTCCTGGGC GGCGTGGTAC ATCTGGAGGC TGGGGAAGAG GTGGTGGTCC 660
GCGTGCCTGG AAACCGCCTG GTCAGACCAC GTGACGGCAC CAGGTCCTAT TTCGGAGCTT 720
TCATGGTCTG AAGGCTGCGG TGACAATGTA TTTTGTGGAG GGACCTCTCC AGGACTCACC 780
TCAAACCCAG CAATA 795
<210> 9
<211> 9058
<212> DNA
<213> Murine
<400> 9
TCTAGATTGC ATTAACAGAG AGAAGACCCT GAGTATGGGT GGAGTCATCT CAGGGCTGAG 60
GTCCTGGGCT GAATGAAAAT GACAGAGTGA GCAGAGCACC CGTGTTCTTT GGTTTCTGCT 120
ACCTCACTGT GGATTTGACA TGACCAACTG CCTCGTGCTA CCTCCCCTCT CCCCACTTTG 180
ACTTCTCCAC TGTGATAGAC ACTTCTCACT ATGAGCCAAG ATTTTTCTTC CTCCCAGTCT 240

TAGTTGGGGT TTCTACTGCT GTGCTAAAAC ACCATGACCA AAAGCAACTT GGAGAGAAGA 300
GGGTTTATTT CAGTTACATT TCCAGGTTAT AGTTCATCAC TGAATAAAAT CAGGACAGGA 360
ATTCTAATTG TGCAGGATCC TGGAGGCAGT AGGTGACGCA GAGGCCATGA GGATGCTGCT 420
TGCTAGTTTG CTCCCCATGG CTTGCTTGCT CAGCCTGCTT TCTTATAGAA CCCAGGATCA 480
TCAGCCCAGG GGATGGTGCC ATCCACAGTG GACTGGGCCC TTCCGCATCA ATCACTAATG 540
GAGAAAATGC CCCACGGGCT TAAATGCATC CTGATCTTAT GGAGGCATTT TTTTTTCAAC 600
TGAGGCTCCC TCCTCTCAGA TGATTTTTGC TCGTGTCAAG TTGACATAAA ACTATCCAGC 660
ACCCTGTACC CTTTGTCATC TTGACACACA AACACAACAC TAGTAAGCCA CAACCTTTCC 720
TTTCTTGTCA TCTCCAAGAT CAAACATTAA TATCAACGTC ACAACATAAA AACATTTTGT 780
GGGCTAGAGA GATGGCTTGG AGGCTAAGAG CACTGGCTGT CCTTCAAGAA AATGAGCAAT 840
TCCCAGCACC CACACGGCAG CTCACAACTG TCTTTAATTC ATTTTCCAGG GGATCCAAAT 900
CCCTCACACA TGCAAGCAAA ACACTAATGT ACAGAAAATA GAAATAAATA AATTTTTAAA 960
CATTTTGCAA ACTTAAAAAA AAAAAAAGTC CCACCACTTT ACAAATTCAA ACAGGTTAAA 1020
ATTTAGTGTC TTTAAAACTC CAAAGTTTAA AGTTGAAAAA CCTTTAAAAT CTAAAATATT 1080
TTAAAAGCTA GCCTCTCAGC TGTGGGATCC TATAACATTG AAAACAAGCT TAATACTTCC 1140
TTATTTCAAG AGGGAAGAAC CACGGCACAG TCAACAACCT GAACAAGCAA AACCAAATAC 1200
CAGCTGTGTA AATAATTCGA TTTCCAGTGT CTGGGATTCA AACATGATCT TCTGGGCTCC 1260
TCCAAAGGTC CTGAGTGCCT TCTCTGGTGC TGCCCTATGC AGCCCTCACA TCTTGTCTTC 1320
TAGGCTGGGG CTGGCTCCAC CCCAAGGCTG CTGCTGCTGC TGCTGCTGCT GGTGATCATC 1380
CCAAGACACC AGCATCTTCA AAATACTGTG GTCTTCTGCT GCCACGGAGG CTGCACTTTC 1440
ACCAACAGCC TCTCCTGGTC CCACACAGTG CCATGCCTCA GCTTCTCTCC ATGGCCTCCT 1500
TATCCTTCCA AACCCAGCAT CATCTGAGGA TAACCTTGTT CACCTTAACA GCACAGCTTC 1560
TGTGTACTGG TTCTCTCAGG AAACACTCCC CAGATTTCAC CTCAGTGATG CAGGTCTCTT 1620
CTAAATCACG GCCAGTTCTG CAGAACAAGC TAAGCAGAAC TAAGAATCTC ATTTCAAATA 1680
TCAAACATCC CCAATGCTCT CCTTGTTGTT GTTTTTGTTT TAAAATAGAA TCCCTTTATA 1740
GTTCTTGCTC TCTTGGAACT CACTATGTAG AATAGGCTGG TTTTGATCTC ACAGGAGATC 1800

CATCTGCCTC TGCCTCCCAA AGGTGTGTAC TACCCTACCC AGGAAATCGT GGTGGTCTTT 1860
AAGAAACTCT CAAACTTCCC TCTGTAATTT CACAAGGCAG GCCTCCATCA TCTGCCGTAT 1920
TCTCAGCATT TTGAACTTCC AAGCTCCCAC AGAACAGCCC ACTGAGCTCT GCATGGAACA 1980
GTTTCTCCAG TCCAAAGTTG AGAGGCCGTA CAATCTTCTC AATACCATAG TCAGGTCTTT 2040
CCTATTCCTA ACCCACACCT GATTCCAATT TGTGTCTTGG CTAGGATTTC TATTGCTGTC 2100
ATAAAACAGC ATGATCAAAA GCAACCTCAG CTTACACTTT CAGGTCACAC CCCATCCACT 2160
GAGGGAGGTC AGGACAGGAA CCTGGAGACA GGAGCTGATG CAGAGACTAT GGAAGAACAC 2220
TGCTTACTGG CTTGTGTCCT ATGACTTGTT CAGCCTGCTT TCTTATAGAA CTCAGGAATG 2280
CCAGTGCAGG GAAGGCCCAG TTTACAGTGA GCACGGCCTC CCACAAGCCA ATCTGTTGGG 2340
GGTGTTTTCT CCATTGAAGT TTCCTCTTCC AAAATGAATG TAGCTTGTGT CAAATTGACA 2400
CTGGCCAGCA CAGACCATGA GAGATTAGAG AACGCTTCCA GGCAGATCCT TTATGGAGTA 2460
TCAGATACTC CGGGCTGCTG AGTAATTTGC ACAGTTCCAG GAATGGACCT GAGTTGGGCA 2520
ACAGCAAAGA GATGAAGAAA CGACAAACAC AGACATCCAG GAATCCTGGG GTCATGTGAT 2580
CTGGGCCTTC TGCACACGAG TTCTCTTAGC ACGTTATTAT ATAGAGTCGA GTGGAGAAGT 2640
GGGGTTATTG CCTACCACTG AAAAAGGCAA CTTATTACAT ACAGTTAAGC AAGGAGGCAG 2700
GGTTTATATG CACAATGGGA TGGGGAAGGC AGGTTGAGTT AGTCTCCATA AGAACTGTCC 2760
CTGGAAGGGA GCTATGTTCA TGCGTCAGCA CTCAAGGGGG AGGATGGTGA ACACATTCTA 2820
CACACACTGG CGACATGCAC ATCCATGCGT GGACCAGAAG AGAAGGCTAT GTTTCCCTCT 2880
GGGTCTGAGG CTCTGGAGTT GTTGACAAGC TCATGTCAAC AGCACACATC CCTTCAGCAC 2940
CTCACAGACC TGGGCTTCCT CTGAAGGGCT CTGTTGCTGA GGCAGGTGGA TGGGTGGGTC 3000
CTGCAGGGGG ACAGGAGTCT CAGCAGTACT TTCCCCTGGA CCCTTGGTTA GAGAACCCAT 3060
GAATCAGCAG CGTGGCAGAG CACTGGGGTT CTGTGGTCAC AGCATCGCCA GCCCTGTGTC 3120
AAGACAAACA CTACAGCATG GGGGCCATTG ACATTGAGAA GGGGCGCTGG TGATTCTTAT 3180
AACTGAACCC CCAAGTCTTC AGTATCTGGG AGGGTGTCTG ATGGGGGTAA GCTCCAAGCT 3240
GAAGATTTGG GGACAAGGTG GTATCTGAAA ATCTACCCCA GCACCGTCTC CAGTGGACAC 3300
ACATATGAAC ACATCTGAGG TTCATGTGTG CATGTGCTAA TAGATGATAG ACACTGTAGT 3360

GTGTGTGTGT GTGTGTGTCC ATATATGGAC CTGCACACAC ATCAGGAAAT GCCTGTGTGC 3420
TTGTTCCAGC ACTTGCAGAC ACATATCAGA ACATCTACAC CTGTGTGCAC ACATCCACGA 3480
ACCGTGCATC TGTACAAGCA CCTGCACCCA TATGCACAGA CTAAAGCAAA CACAGAGGCC 3540
TCTGCTTGTC AGCGCGTGCA AATGCAGGTG CGGTGCAAGA TGCTTGCATG GAACCCACAG 3600
AAGTTCTTTT GAGGGAAACA AAAGCCACCA AGTACATCGG AGCAGGGGCT GCCCCATCCA 3660
TCCCACCTGA GTCACATTTT CTGGAAAGTG TGAGCTATGG TGGCCTCAGT GAGAGTGATC 3720
GACCGGGGGC TGGCCTTCTG GGGGCACAGG AAGAAGAGGA GGTACGTGAG GAAAGGGGAG 3780
GCACCAGACT TCAGCTTTAA AGTGAGTCCT AGGGGTGACA GGAACCTTTT GCAGTTTGCA 3840
CAGCCCGAGC GTGTTGGGCA ATTGTGGTTT CCTCCGGAGA GGAGGAACTC AGGCTTGCCA 3900
ACCCTTTCCC CTGGGCTTCG GAGCCTCAGC TGCTCTGGCA TGGAGAGTGT GGTACAGCCT 3960
TCAGTGTTTG TGGTGGATGG ACAGACGGAC ATCCCATTCA GGCGGCTGGA ACAGAACCAC 4020
CGGAGACGGC GCTGTGGCAC TGTCCAGGTC AGCCTGGCCC TGGTGCTGCT GCTAGGTGCT 4080
GGGCTGGCCA CTCAGGGCTG GTTTCTCCTG AGACTGCATC AACGTCTTGG AGACATAGTA 4140
GCTCATCTGC CAGTAAGTGG GGCTTGGGGA ACCAGCGAGT CTTCTCCAGT ACCTGTCCCT 4200
TTATGGTTGT GTATTGTGTG CTAGAACCCA GGGCTTCGGT TAGCCTGTCT CTCTGACCCT 4260
CAGTCTTCTC ATCTTTACAT GAGGATCTGA TACACGTGCA GTGCCCGGCA GTTTCCTTGG 4320
GATTCACCTA CTCGGTTGTT GTTTCCAGCC CAAACCTTTG TCCACCACTG GCTTCATGGT 4380
CACCCTGCTT CACTCGCAGC TCTCTCATGT GCTTGCTCTA CTGTTTCCTT TCAGACAAGC 4440
CATGGGTGTG TCACAGTATG CTCCCGAGTC TTTGCCTATA TTTTCTTTTT AAGTTTTTTT 4500
TTTTAATTAC ATTTACTTAT TTAGTGTGTA GACATGAAAA AGTATGCATG TGCCGCAGCA 4560
TGTATGTGGA ACACCTCTAT GGAATCACTT CTCTGTGCCT TCCTTTGTGG AGGGTCCTAG 4620
GGACTGAACT CAGGTAGCCA GGCTTGGCAG CAAGCACTTT TACCGCCTGA GCCCTCTCAC 4680
CGGCCCTCAA CTATCCAAAA ACCTCGAAAA CAGTTCCGAA ACAATTCTAG TCTCAAGCAT 4740
TTCAGACACT CGACTTGACT AGTTAATTCC TCATCCCAGC ACTTGGGCAG GGGAGGCAGA 4800
AGACTGAGGG AGTTCAAGGT CATCCTAGGC TATTTAGCAA GTCTGAGGCC AGACTGGGCT 4860
ACATGAGAGC CTATCTTGGA AATACAACAG ATCTAATTAG GTGTGTGCCC CTCCCCCTGC 4920

ATCTGTGTGT ATGTATGTGA TATGTGCATG TGTGGCTCTG CATACATGTC CATGGGTGGT 4980
GTCTGTGGCC ATGGCCAGAC AGTCTCATCA CCACATACCA GTGAGACTCG GAGCGGCATG 5040
TCCTTACCAA ACATCCCTGT CTGCATTTCA GGATGGAGGC AAAGGCTCCT GGGAGAAGCT 5100
GATACAAGGT GAGTTTGGGC CCCAGTCCCT TTGAAGCAGG TGGAATGGTT TCCGTTCAGC 5160
ACCTCCCCAC ACCAACTCTG GGTCATCACC CAGTGTCACA TTGTAGGCCT CGCAGGTGCC 5220
CCTCCCCCAG GGCCAGAGAA GCAGAAATCT GGGTCCAAAA GGGACAGAGC CTGACACAGG 5280
TAGCCTCAAT TGATGTGTGA GAAACTTCGG GTACACACAC ACTGAGACAT GCGTGCACAC 5340
ACACACATAC ACACACACAC ACACACACAC AATCCTCTTT TTGTCTCTAA CCTTTGACTC 5400
TCTTCCTCAG ATCAACGATC TCACCAGGCC AACCCAGCAG CACATCTTAC AGGTGAGAGG 5460
GACCCCAGAT CTTCACACGG AATGGGGTTT AGCATGTCCT GGGAGACTCA GATCTTTATA 5520
AGTTAGAGCA CTCGTTTATG CATTCATTTA GTGTGTCTTT AAATTTTTTT TGACATTTAC 5580
TTCTTTTTAT TCATTTATTA TTGCACATGT GCATGCCATA GTGTAGAGGT CAGAAAACTC 5640
TCAGGGGAAC GATTCTTTCC TTCCACCTTT TGGCCTCCGT GTACTGATCT CAGCCATCAG 5700
GCTTGGCTCT AAGTACCTTT ACTCTCTGCG CCATCTTGCC GGTCACTTTT TTTTTTCTCA 5760
GTGTTTACTA AAAAACAGCC CAGAGAAGAG CCACAGGGAG CCATGTAAAG TCAACTCTTC 5820
ACCCTGCTAG TCATGGGGAC ACATCCCAAG GTCCTTGGAG GCCTCAGAAG ATGTTGGGAC 5880
TCAGACCTAC CACAGTTTAT ATTTGTTTGT TTGTTTGGTT GGTTGGTTGG TTGGTTGGTT 5940
GGTTGGTTGA TTGGTTGGTT GGTTTTGGTT TTGGAGACAG GGTCTCTCTG TGTAGCTTGG 6000
GCTGCCTGGG TAGACCTGCC TCTGTCCTGA GTACTGGAAT TAACAGGAAT TAACGACGTG 6060
GGCCACTACA CCCAGCTGTA TATACTGGTT TTTCTTTTGG CATAGACAGT TTAATTTATA 6120
ATTTAACAAG TAAGAAACTA ACAAGAATAC ACATAAAGTA TGCTGTAATT AAAAACTATT 6180
TTTGAAACAT GATGTGGGAG ATAGGGAGGT GGCTCCATGT GCAAATTGCT AGCCACTTTT 6240
GACTCAACAT CTGAGCCAAA AGCCAAACAC ACACTCCCGT GACAGTTACA GAGGCAGATT 6300
CAGACAGACA CACACTCCTG TGACAGTCAT GGAGACAGAG ACAGGCAGAG AGGTCCCTAG 6360
GACCTGCTGT CCAGCAGCCT TGACAAACAG GGGACTTCCA GGGTCAATAA GAGACTCTGC 6420
CTCAAAACTA AGGTGGACAG AGGTTGAAAC TGAGGAAGTT GCCTGGTTTT GACTTCTGGG 6480

CACACACACA CACACACACA CACATATATA TAACACATAC ATCACACACA TATACACAAA 6540
CATACAGTCA CACACACTTT TTGAGATAGA GTCTCATGTA GCCTAAACCG GCCTTGAACT 6600
CTTGATCCTC CAGACTCGAC GTCCCAAGAT CTGGATGTCA CCACTACACC TGATTTATTT 6660
GGTACTGGGG CTGGAATGTA CAGCCTTCGG CATGCTGGGC GAGTGTTCCA CCTCCTAAAT 6720
CCTAACCCCA TTTAAAAAAA ATTTTTTTGA CATTTCACTA GGAAGCTTCA ATACCTTGTG 6780
CCTCAGTTTC TTCTTTGGTA TTCAAGTGAC TTTGCTCCTC AGTGCTTCAG TGTCTACCCT 6840
TGCAGAAGGA GATGGGAATG AACGGAGTTA AGACACAACC ACCAAATTAC TTTGCTTGCT 6900
TTCCTGTATC TCAGTTTCTT CCCCCTGCAA GATAGGAATG GGCTGAGTCA ACCCAGACAA 6960
GCAGCCTACT ATGGTGGTGA AACCAGAGGG AACTAGCATA AGGTCACACA GAAAGGGTGG 7020
GCACAATGCT AACCGTGCTG ACGGGTTATC TTTCTCTCTC CTTTCCTCCC AGGAGCCAAC 7080
GCCAGCTTGA TAGGTATTGG TGGACCTCTG TTATGGGAGA CACGACTTGG CCTGGCCTTC 7140
TTGAGGGGCT TGACGTATCA TGATGGGGCC CTGGTGACCA TGGAGCCCGG TTACTACTAT 7200
GTGTACTCCA AAGTGCAGCT GAGCGGCGTG GGCTGCCCCC AGGGGCTGGC CAATGGCCTC 7260
CCCATCACCC ATGGACTATA CAAGCGCACA TCCCGCTACC CGAAGGAGTT AGAACTGCTG 7320
GTCAGTCGGC GGTCACCCTG TGGCCGGGCC AACAGCTCCC GAGTCTGGTG GGACAGCAGC 7380
TTCCTGGGCG GCGTGGTACA TCTGGAGGCT GGGGAAGAGG TGGTGGTCCG CGTGCCTGGA 7440
AACCGCCTGG TCAGACCACG TGACGGCACC AGGTCCTATT TCGGAGCTTT CATGGTCTGA 7500
AGGCTGCGGT GACAATGTAT TTTGTGGAGG GACCTCTCCA GGACTCACCT CAAACCCAGC 7560
AATAGGGTTT GAAGTCCTCC CTTTAAGGAG CCCTGAACTC TGCAGTGCTC GGGGCGGTGT 7620
TCACTGCTGA CCTGCTTTGG GCAATCTTCA AATCAGAGAC CTGGAGACTT GGGGCGTGGA 7680
GCCCAGGAGC GAGGGGTCAG CTCATTTGCC TGATATTCAG GAAGAAAGAA TCAAGCTGGG 7740
GTATTTATGC TTCTGATGCA AACACTGAGA TTTCGGCTTT CTGGGTTTTG AGCTGGAGGC 7800
AAGAAACCTT CCCAGAGTGT CATCAGGACC ATGTTGGCAG GACTTGGGGC TCCAGACTTG 7860
CCACCACACT CTGGCCTCTC CCATCCATCC GCTGCATTGG TTTCCAGCCA CCAAAACAGC 7920
ACTGGCCCCC TGGCTGCAAC TGGCCAGGTA CGAGCTTCTG AGCACCTACA TTCCTCAGGG 7980
ACATCTTGAT GAGATCTCAG TACTCAGTCC AATGCGCAGC AGCGACAGAC ATGCCAGGAA 8040

TGGTTGGTCA GAAGGGAAGG GAGGAAAGGG AGGAAAGAAC GGAATGCACA AGAGAAGGGG 8100
GGAAAACAAG ACCAAAACAA AACAGCAACA ACAAAGCGGC AGGGAAGAAG TTGACACCCT 8160
TGGGGATACT TTAGTCAACA CACTTAGAAC AGATTGTGCC AGGCCTGTTG GATTCCTGGA 8220
GTTGATGGGA TCGTGGGAAG GCACAATGGG GAGCAAGTGG GCTTGGGTTA TGGCTCAGTG 8280
GGTAAAGTGC AATTATGGGG ATCTGAGTTT GAATCCCTGG TACCCATATA AAGACACAGA 8340
TGCGGTGATG GGCACTTGTG ACAATGAGAT CATCAATAGG GAATGGAGAC AGGAGGGACC 8400
TCTGGGGTTC ACTGGCCAGG CAGTCTAGCT GAATCAAAGA GCTCCAAGTT CAGTCGATAG 8460
CTCCTGAAGA TGACAACTGA GGCTATTCTC CAAACCCCAC ACGCAGGGAC ACATGCGTAA 8520
TAAATAAAAT TTTAAAAATA TTAAATAATA GTGTTTGAGA GGCACTTTAT ATGTTCTAAT 8580
CATTTGTGCT TTTGTTTATT TGTTCAGGTT TGTTTGTTTG TTTGTTGGAA GACAGGGGTC 8640
TCAAGTAGCC CAGGCTAGCT TTGAACTATA TATTTCATAT ATTTTGAGGC AGTCTTATGC 8700
CTTTTATCTA GGTTTTCTTG GCATCTAAAG CTATAGCTGT GTGTTACTCC AATGGAACAT 8760
CTGGGCAGTT ACCCAGCACC TTCAAATGCA GAGCCCACAC CTGATAGGGT CGGGCTCGCC 8820
CTGCTCAGGG GTCGCTGCTA GTCCAAGCCA GTCCAAGCGG ACTTCCCGCG TCCTGTCCTT 8880
GCAACCCTGG TGGGAGGTGG AAAAGCCCCC AAATACCCAG TCTCACCCTC CATCGGAGTT 8940
TCCTTTATGC TTATCACGGC CTGTTTCCGT GTCTTTGATG AGACCAAGGT GTGGGGACAG 9000
TATATTTATA AAAAGCCACC AGCAGTTTCC GCTGTAAGGA AAAAAAAATC ACTCTAGA 9058
<210> 10
<211> 717
<212> DNA
<213> Rat
<400> 10
ATGGAGAGTG TGGTACAGCC TTCAGTGTTT GTGGTGGATG GACAGACAGA CATCCCATTC 60
AGGCGGCTGG GACAGAACCA CAGGAGACGG CACTGCGGCA CTGTCCAGGT CAGCCTGGCC 120
CTGCTGCTGC TGCTGGGTGC TGGGCTGGCC ACTGAGGGCT GGTTTCTCCT GAGACTGCAT 180
CAGCGTCTTG GGGACATAGT AGCTCATCTG CCAGATGGAG GCAAAGGCTC CTGGGAGAAG 240

CTGATACAAG ATCAACGATC TCACCAGCCC AACCCAGCAG CACATCTCAC AGGAGCTAAC 300
GCCAGCTTGA TAGGCATTGG TGGACCTCTG TTATGGGAGA CACAACTTGG CCTGGCCTTC 360
CTGAGGGGCC TGACGTATCA TGATGGGGCC CTGGTGACCA CCGAGGCTGG CTACTACTAC 420
GTGTACTCCA AAGTGCAGTT GAGTGGTGTG GGCTGCCCCC AGGGGCTGGC CAATGGCCTC 480
CCCATCACCC ACGGGCTGTA CAAGCGCACA TCCCGATACC CCAAGGAGTT AGAACTGCTG 540
GTCAGCCGGC GGTCACCTTG TGGCCGGGCC AACAGCTCCC GAGTCTGGTG GGACAGTAGT 600
TTCCTCGGCG GAGTGGTACA TCTGGAGGCC GGAGAAGAGG TGGTGGTCCG CGTGCCTGGA 660
AACCGCCTGG TCAGACCACG TGATGGCACG AGGTCCTATT TCGGAGCTTT CATGATC 717
<210> 11
<211> 24
<212> DNA
<213> Artificial Sequence
<220>
<223>
<400> 11
AGGTCAACCC AGCAGCGCAT CTCA 24
<210> 12
<211> 28
<212> DNA
<213> Artificial Sequence
<220>
<223>
<400> 12
AGGTCAACCC AGCAGCGCAT CTCACAGG 28
<210> 13
<211> 30

<212> DNA
<213> Artificial Sequence
<220>
<223>
<400> 13
CAAATTAAAC CGGGTACCAT CACGCAGTCG     30
<210> 14
<211> 25
<212> DNA
<213> Artificial Sequence
<220>
<223>
<400> 14
TCTGCTCTGG CATGGAGAGT GTGGT     25
<210> 15
<211> 24
<212> DNA
<213> Artificial Sequence
<220>
<223>
<400> 15
CTATTGCTGG GTTTGAGGTG AGTC     24
<210> 16
<211> 22
<212> DNA
<213> Artificial Sequence

<220>

<223>

<400> 16

GTAATACGAC TCACTATAGG GC     22

<210> 17

<211> 19

<212> DNA

<213> Artificial Sequence

<220>

<223>

<400> 17

ACTATAGGGC ACGCGTGGT     19

<210> 18

<211> 48

<212> DNA

<213> Artificial Sequence

<220>

<223>

<400> 18

GTAATACGAC TCACTATAGG GCACGCGTGG TCGACGGCCC GGGCTGGT     48

<210> 19

<211> 27

<212> DNA

<213> Artificial Sequence

<220>

<223>

<400> 19
CAGCCCAGCA CCTAGCAGCA GCACCAG 27
<210> 20
<211> 27
<212> DNA
<213> Artificial Sequence
<220>
<223>
<400> 20
GCCGCCTGAA TGGGATGTCC GTCTGTC 27
<210> 21
<211> 32
<212> DNA
<213> Artificial Sequence
<220>
<223>
<400> 21
ACGAATTCCA AGAGCGAAGG TCTCACGAGG TC 32
<210> 22
<211> 35
<212> DNA
<213> Artificial Sequence
<220>
<223>
<400> 22
AGTCTAGACT CCTTCCTTCA CACCATGAAA GCCCC 35

<210> 23
<211> 24
<212> DNA
<213> Artificial Sequence
<220>
<223>
<400> 23
CCTGACCCTG GGCTTCTGAG CCTC          24
<210> 24
<211> 27
<212> DNA
<213> Artificial Sequence
<220>
<223>
<400> 24
TCCACAAAAT CCATTGTCGT CATAGCC          27
<210> 25
<211> 242
<212> PRT
<213> Human, Murine, Rat
<400> 25
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30
Cys Xaa Xaa Xaa Xaa Val Xaa Leu Xaa Leu Xaa Leu Leu Leu Xaa Gly

        35                  40                  45
Ala Gly Leu Ala Xaa Gln Gly Trp Phe Leu Leu Xaa Leu His Xaa Arg
    50                  55                  60
Leu Gly Xaa Xaa Vla Xaa Xaa Leu Pro Asp Gly Xaa Xaa Gly Ser Trp
65 70 75 80
Glu Xaa Leu Ile Gln Xaa Xaa Arg Ser His Xaa Xaa Asn Pro Ala Ala
85 90 95
His Leu Thr Gly Ala Asn Xaa Ser Leu Xaa Gly Xaa Gly Gly Pro Leu
100 105 110
Leu Trp Glu Thr Xaa Leu Gly Leu Ala Phe Leu Arg Gly Leu Xaa Tyr
115 120 125
His Asp Gly Ala Leu Val Xaa Xaa Xaa Xaa Gly Tyr Tyr Tyr Xaa Tyr
130 135 140
Ser Lys Val Gln Leu Xaa Gly Val Gly Cys Pro Xaa Gly Leu Ala Xaa
145 150 155 160
Xaa Xaa Xaa Ile Thr His Gly Leu Tyr Lys Arg Thr Xaa Arg Tyr Pro
165 170 175
Glu Xaa Leu Glu Leu Leu Val Ser Xaa Xaa Ser Pro Cys Gly Arg Ala
180 185 190
Xaa Xaa Ser Ser Arg Val Trp Trp Asp Ser Ser Phe Leu Gly Gly Val
195 200 205
Val His Leu Glu Ala Gly Glu Xaa Val Val Val Arg Val Xaa Xaa Xaa
210 215 220
Arg Leu Val Arg Xaa Arg Asp Gly Thr Arg Ser Tyr Phe Gly Ala Phe
225 230 235 240
Met Val

<210> 26
<211> 34
<212> DNA
<213> Artificial Sequence
<220>
<223>
<400> 26
GAATTCGATA CAAGAGCGAA GGTCTCACGA GGTC 34
<210> 27
<211> 35
<212> DNA
<213> Artificial Sequence
<220>
<223>
<400> 27
AAATCTAGAT CCTTCCTTCA CACCATGAAA GCCCC 35
<210> 28
<211> 36
<212> DNA
<213> Artificial Sequence
<220>
<223>
<400> 28
AGGAATTCAT GGAGGAGAGT GTCGTACGGC CCTCAG 36
<210> 29
<211> 35

<212> DNA
<213> Artificial Sequence
<220>
<223>
<400> 29
AGCTCGAGCT CCTTCCTTCA CACCATGAAA GCCCC 35
<210> 30
<211> 612
<212> DNA
<213> Human
<400> 30
ATGGAGGAGA GTGTCGTACG GCCCTCAGTG TTTGTGGTGG ATGGACAGAC CGACATCCCA 60
TTCACGAGGC TGGGACGAAG CCACCGGAGA CAGTCGTGCA GTGTGGCCCG GGACGGACCT 120
GCAGGCTCCT GGGAGCAGCT GATACAAGAG CGAAGGTCTC ACGAGGTCAA CCCAGCAGCG 180
CATCTCACAG GGGCCAACTC CAGCTTGACC GGCAGCGGGG GGCCGTTGTT ATGGGAGACT 240
CAGCTGGGCC TGGCCTTCCT GAGGGGCCTC AGCTACCATG ATGGGGCCCT TGTGGTCACC 300
AAAGCTGGCT ACTACTACAT CTACTCCAAG GTGCAGCTGG GCGGTGTGGG CTGCCCGCTG 360
GGCCTGGCCA GCACCATCAC CCACGGCCTC TACAAGCGCA CACCCCGCTA CCCCGAGGAG 420
CTGGAGCTGT TGGTCAGCCA GCAGTCACCC TGCGGACGGG CCACCAGCAG CTCCCGGGTC 480
TGGTGGGACA GCAGCTTCCT GGGTGGTGTG GTACACCTGG AGGCTGGGGA GAAGGTGGTC 540
GTCCGTGTGC TGGATGAACG CCTGGTTCGA CTGCGTGATG GTACCCGGTC TTACTTCGGG 600
GCTTTCATGG TG 612
<210> 31
<211> 204
<212> PRT
<213> Human

<400> 31
Met Glu Glu Ser Val Val Arg Pro Ser Val Phe Val Val Asp Gly Gln
1               5                   10                  15
Thr Asp Ile Pro Phe Thr Arg Leu Gly Arg Ser His Arg Arg Gln Ser
            20                  25                  30
Cys Ser Val Ala Arg Asp Gly Pro Ala Gly Ser Trp Glu Gln Leu Ile
        35                  40                  45
Gln Glu Arg Arg Ser His Glu Val Asn Pro Ala Ala His Leu Thr Gly
    50                  55                  60
Ala Asn Ser Ser Leu Thr Gly Ser Gly Gly Pro Leu Leu Trp Glu Thr
65                  70                  75                  80
Gln Leu Gly Leu Ala Phe Leu Arg Gly Leu Ser Tyr His Asp Gly Ala
                85                  90                  95
Leu Val Val Thr Lys Ala Gly Tyr Tyr Tyr Ile Tyr Ser Lys Val Gln
            100                 105                 110
Leu Gly Gly Val Gly Cys Pro Leu Gly Leu Ala Ser Thr Ile Thr His
        115                 120                 125
Gly Leu Tyr Lys Arg Thr Pro Arg Tyr Pro Glu Glu Leu Glu Leu Leu
    130                 135                 140
Val Ser Gln Gln Ser Pro Cys Gly Arg Ala Thr Ser Ser Ser Arg Val
145                 150                 155                 160
Trp Trp Asp Ser Ser Phe Leu Gly Gly Val Val His Leu Glu Ala Gly
                165                 170                 175
Glu Lys Val Val Val Arg Val Leu Asp Glu Arg Leu Val Arg Leu Arg
            180                 185                 190
Asp Gly Thr Arg Ser Tyr Phe Gly Ala Phe Met Val

        195                 200
<210> 32
<211> 28
<212> DNA
<213> Artificial Sequence
<220>
<223>
<400> 32
CCAACGCCAG CTTGATAGGT ATTGGTGG 28
<210> 33
<211> 22
<212> DNA
<213> Artificial Sequence
<220>
<223>
<400> 33
CCCAGCAGCA CATCTTACAG GA 22
<210> 34
<211> 22
<212> DNA
<213> Artificial Sequence
<220>
<223>
<400> 34
AGGCCAAGTC GTGTCTCCCA TA 22
<210> 35

<211> 30
<212> DNA
<213> Artificial Sequence
<220>
<223>
<400> 35
CTATGGCCCA GACCCTCACA CTCAGATCAT 30
<210> 36
<211> 22
<212> DNA
<213> Artificial Sequence
<220>
<223>
<400> 36
CAAATGGCCT CCCTCTCATC AG 22
<210> 37
<211> 22
<212> DNA
<213> Artificial Sequence
<220>
<223>
<400> 37
GGCTACAGGC TTGTCACTCG AA 22
<210> 38
<211> 27
<212> DNA

<213> Artificial Sequence
<220>
<223>
<400> 38
ACAACGAAAA GGCCCCCAAG GTAGTGA          27
<210> 39
<211> 23
<212> DNA
<213> Artificial Sequence
<220>
<223>
<400> 39
GTGACCAGAT CCCTGAATTG GAA          23
<210> 40
<211> 22
<212> DNA
<213> Artificial Sequence
<220>
<223>
<400> 40
GTAGGCGGTG GCTTTTTTG AG          22
<210> 41
<211> 28
<212> DNA
<213> Artificial Sequence
<220>

<223>
<400> 41
CCTCTTGCAA ATGCCTCCCT GGTTACCA 28
<210> 42
<211> 24
<212> DNA
<213> Artificial Sequence
<220>
<223>
<400> 42
ATACAAGGAC TACTGCATCC ACGG 24
<210> 43
<211> 24
<212> DNA
<213> Artificial Sequence
<220>
<223>
<400> 43
GTAGAGTCAG CCCATGACAC CTGT 24
<210> 44
<211> 30
<212> DNA
<213> Artificial Sequence
<220>
<223>
<400> 44

TGTCCTCATT ATCACCTGTG TGCTGATCCA 30
<210> 45
<211> 21
<212> DNA
<213> Artificial Sequence
<220>
<223>
<400> 45
AAGAAGCAAG CCATCACTGC C 21
<210> 46
<211> 22
<212> DNA
<213> Artificial Sequence
<220>
<223>
<400> 46
ACAGTGTTTG CGGAGCTGAC AG 22
<210> 47
<211> 204
<212> PRT
<213> Human
<400> 47
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30

Cys Xaa Xaa Xaa Xaa Asp Gly Xaa Xaa Gly Ser Trp Glu Xaa Leu Ile
        35                  40                  45
Gln Xaa Xaa Arg Ser His Xaa Xaa Asn Pro Ala Ala His Leu Thr Gly
    50                  55                  60
Ala Asn Xaa Ser Leu Xaa Gly Xaa Gly Gly Pro Leu Leu Trp Glu Thr
65                  70                  75                  80
Xaa Leu Gly Leu Ala Phe Leu Arg Gly Leu Xaa Tyr His Asp Gly Ala
                85                  90                  95
Leu Val Xaa Xaa Xaa Xaa Gly Tyr Tyr Tyr Xaa Tyr Ser Lys Val Gln
            100                 105                 110
Leu Xaa Gly Val Gly Cys Pro Xaa Gly Leu Ala Xaa Xaa Ile Thr His
        115                 120                 125
Gly Leu Tyr Lys Arg Thr Xaa Arg Tyr Pro Glu Xaa Leu Glu Leu Leu
    130                 135                 140
Val Ser Xaa Xaa Ser Pro Cys Gly Arg Ala Xaa Xaa Ser Ser Arg Val
145                 150                 155                 160
Trp Trp Asp Ser Ser Phe Leu Gly Gly Val Val His Leu Glu Ala Gly
                165                 170                 175
Glu Xaa Val Val Val Arg Val Xaa Xaa Xaa Arg Leu Val Arg Xaa Arg
            180                 185                 190
Asp Gly Thr Arg Ser Tyr Phe Gly Ala Phe Met Val
195 200 204
<210> 48
<211> 33
<212> DNA
<213> Artificial Sequence

<220>
<223> Primer
<400> 48
GTAGAATTCG GCCAACCCAG CAGCACATCT TAC          33
<210> 49
<211> 33
<212> DNA
<213> Artificial Sequence
<220>
<223> Primer
<400> 49
AAATCTAGAT ATTGCTGGGT TTGAGGTGAG TCC          33
<210> 50
<211> 28
<212> DNA
<213> Artificial Sequence
<220>
<223> Probe
<400> 50
CCAACGCCAG CTTGATAGGT ATTGGTGG          28
<210> 51
<211> 22
<212> DNA
<213> Artificial Sequence
<220>
<223> Primer

<400> 51
CCCAGCAGCA CATCTTACAG GA 22
<210> 52
<211> 22
<212> DNA
<213> Artificial Sequence
<220>
<223> Primer
<400> 52
AGGCCAAGTC GTGTCTCCCA TA 22
<210> 53
<211> 30
<212> DNA
<213> Artificial Sequence
<220>
<223> Primer
<400> 53
CTATGGCCCA GACCCTCACA CTCAGATCAT 30
<210> 54
<211> 22
<212> DNA
<213> Artificial Sequence
<220>
<223> Primer
<400> 54
CAAATGGCCT CCCTCTCATC AG 22

<210> 55
<211> 22
<212> DNA
<213> Artificial Sequence
<220>
<223> Primer
<400> 55
GGCTACAGGC TTGTCACTCG AA 22